# EUROPÄISCHE PATENTSCHRIFT

(11) **EP 3 424 907 B1**
(45) Veröffentlichungstag und Bekanntmachung des Hinweises auf die Patenterteilung: **12.03.2025**
(21) Anmeldenummer: 18183320.3
(22) Anmeldetag: 27.06.2013
(51) Int. Cl.: C07D 219/02, H05B 33/10, C07D 307/91, C07F 5/02, C07D 209/90, C07D 209/86, C07C 217/80, C07C 215/88, C07C 211/61, C07C 217/92, C09K 11/06, C07D 311/80, C07D 333/76, C07C 211/54, C07D 405/04, C07D 407/04, C07D 407/12

(54) **VERBINDUNGEN UND ORGANISCHE ELEKTRONISCHE VORRICHTUNGEN**
CONNECTIONS AND ORGANIC ELECTRONIC DEVICES
RACCORDEMENTS ET DISPOSITIFS ÉLECTRO-ORGANIQUES

(30) Priorität: 23.07.2012 EP 12005371
(43) Veröffentlichungstag der Anmeldung: 09.01.2019
(62) Teilanmeldung aus: 13734657.3
(73) Patentinhaber: Merck Patent GmbH, 64293 Darmstadt (DE)
(72) Erfinder: Mujica-Fernaud, Teresa, 64283 DARMSTADT (DE); Montenegro, Elvira, 69469 WEINHEIM (DE); Parham, Amir, 60486 FRANKFURT AM MAIN (DE); Buesing, Arne, 65929 Frankfurt am Main (DE); Voges, Frank, 67098 BAD DUERKHEIM (DE)
(74) Vertreter: Merck Patent Association

(56) Entgegenhaltungen:
- EP-A1- 2 468 725
- WO-A1-2012/026780
- WO-A2-2010/110553
- JP-A- H0 378 756
- DATABASE CAPLUS [online] CHEMICAL ABSTRACTS SERVICE, COLUMBUS, OHIO, US; YASHIRO, RYOJI ET AL: "Electrophotographic photoreceptor using arylamine charge-transporting agent", XP002715438, retrieved from STN Database accession no. 1991:570935

## Beschreibung

Die vorliegende Erfindung betrifft neue organische Verbindungen sowie eine elektronische Vorrichtung enthaltend wenigstens eine der Verbindungen. Weiterhin betrifft die vorliegende Erfindung ein Verfahren zur Herstellung der Verbindungen sowie Zusammensetzungen und Formulierungen enthaltend wenigstens eine der Verbindungen.

Die Entwicklung von funktionellen Verbindungen zur Verwendung in elektronischen Vorrichtungen ist aktuell Gegenstand intensiver Forschung. Ziel ist hierbei insbesondere die Entwicklung von Verbindungen, mit denen verbesserte Eigenschaften der elektrolumineszierenden Vorrichtungen in einem oder mehreren relevanten Punkten erzielt werden können, wie beispielsweise Leistungseffizienz, Lebensdauer oder Farbkoordinaten des emittierten Lichts.

Unter dem Begriff elektronische Vorrichtung werden gemäß der vorliegenden Erfindung unter anderem organische integrierte Schaltungen (OICs), organische Feld-Effekt-Transistoren (OFETs), organische Dünnfilmtransistoren (OTFTs), organische lichtemittierende Transistoren (OLETs), organische Solarzellen (OSCs), organische optische Detektoren, organische Photorezeptoren, organische Feld-Quench-Devices (OFQDs), organische lichtemittierende elektrochemische Zellen (OLECs), organische Laserdioden (O-Laser) und organische Elektrolumineszenzvorrichtungen (OLEDs) verstanden.

Von besonderem Interesse ist die Bereitstellung von Verbindungen zur Verwendung in den zuletzt genannten, als OLEDs bezeichneten elektronischen Vorrichtungen. Der allgemeine Aufbau sowie das Funktionsprinzip von OLEDs ist dem Fachmann gut bekannt und unter anderem in US 4539507, US 5151629, EP 0676461 und WO 1998/27136 beschrieben.

Betreffend die Leistungsdaten von OLEDs sind noch weitere Verbesserungen erforderlich, insbesondere in Hinblick auf eine breite kommerzielle Verwendung, beispielsweise in Anzeigevorrichtungen oder als Lichtquellen. Von besonderer Bedeutung sind in diesem Zusammenhang die Lebensdauer, die Effizienz und die Betriebsspannung der OLEDs sowie die realisierten Farbwerte. Zudem ist es wünschenswert, dass die Verbindungen zur Verwendung als Funktionsmaterialien in elektronischen Vorrichtungen eine hohe thermische Stabilität und eine hohe Glasübergangstemperatur aufweisen und sich unzersetzt sublimieren lassen.

In diesem Zusammenhang besteht insbesondere Bedarf an alternativen Lochtransportmaterialien. Bei Lochtransportmaterialien gemäß dem Stand der Technik steigt im Allgemeinen die Spannung mit zunehmender Schichtdicke der Lochtransportschicht an. In der Praxis wäre häufig eine höhere Schichtdicke der Lochtransportschicht wünschenswert, dies hat jedoch oftmals eine höhere Betriebsspannung und schlechtere Leistungsdaten zur Folge. In diesem Zusammenhang besteht Bedarf an neuen Lochtransportmaterialien, die eine hohe Ladungsträgerbeweglichkeit aufweisen, so dass dickere Lochtransportschichten mit lediglich geringem Anstieg der Betriebsspannung realisiert werden können.

Im Stand der Technik wird die Verwendung von verschiedenen Fluorenen als Ladungstransportmaterial in elektronischen und elektrolumineszierenden Vorrichtungen beschrieben.

In WO 2011/055493 werden sekundäre Amine offenbart, die mehrfach mit Fluorenen in Position 3 substituiert sind.

JP 2008-34701 und WO 2007/072952 offenbaren Fluorene, die in Position 4 mit einer Amingruppe substituiert sind, wobei die Amingruppe selbst wieder mehrere Fluorene enthält.

WO 2010/110553 offenbart mit Amingruppen in Position 2, 3 oder 4 substituierte Fluorene, wobei die Amingruppen Carbazolgruppen enthalten.

In JP 05303221 werden Fluorene offenbart, die in Position 2 oder 4 mit einer Amingruppe substituiert sein können. Die Verbindungen mit der Amingruppe in Position 4 des Fluorens enthalten Phenyl-Reste. Die Verbindungen werden als Photorezeptoren eingesetzt.

Trotz der bereits bekannten Verbindungen besteht unverändert Bedarf an neuen Lochtransport- und Lochinjektionsmaterialien zur Verwendung in OLEDs. Insbesondere besteht Bedarf an Materialien, mit denen die oben genannten, hoch erwünschten Verbesserungen der Leistungsdaten und Eigenschaften der OLEDs erreicht werden können.

Ebenfalls besteht Bedarf an neuen Matrixmaterialien zur Verwendung in OLEDs sowie in anderen elektronischen Vorrichtungen. Insbesondere besteht Bedarf an Matrixmaterialien für phosphoreszierende Dotanden sowie an Matrixmaterialien für Mixed-Matrix-Systeme, welche bevorzugt zu guter Effizienz, hoher Lebensdauer und geringer Betriebsspannung der elektronischen Vorrichtungen führen.

Der vorliegenden Erfindung liegt somit die Aufgabe zu Grunde, elektroluminesziernede Vorrichtungen und Verbindungen, welche sich zur Verwendung in elektrolumineszierenden Vorrichtungen wie beispielsweise in fluoreszierenden oder phosphoresziereneden OLEDs eignen, bereitzustellen und welche insbesondere als Lochtransportmaterialien und/oder als Lochinjektionsmaterialien in einer Lochtransport- bzw. Exzitonenblockierschicht oder als Matrixmaterial in eienr emittierenden Schicht eingesetzt werden können.

Im Rahmen der vorliegenden Erfindung wurde überraschend gefunden, dass sich Verbindungen der unten angegebenen Formel (1) ausgezeichnet für die oben genannten Verwendungen in elektronischen und insbesondere in elektrolumineszierenden Vorrichtungen eignen.

Gegenstand der Erfindung ist somit eine Verbindung der allgemeinen Formel (1) 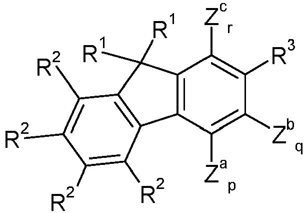 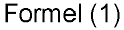 wobei für die verwendeten Symbole und Indices gilt:
- R¹: ist bei jedem Auftreten gleich oder verschieden, bevorzugt gleich, H, D, F, Cl, Br, I, C(=O)R⁴, CN, Si(R⁴)₃, NO₂, P(=O)(R⁴)₂, S(=O)R⁴, S(=O)₂R⁴, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁴ substituiert sein können und wobei eine oder mehrere CH₂-Gruppen in den oben genannten Gruppen durch -R⁴C=CR⁴-, -C≡C-, Si(R⁴)₂, C=O, C=S, C=NR⁴, -C(=O)O-, -C(=O)NR⁴-, P(=O)(R⁴), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 6 bis 30 aromatischen Ringatomen, das jeweils durch einen oder mehrere Reste R⁴ substituiert sein kann, oder eine Aryloxygruppe mit 5 bis 60 aromatischen Ringatomen, die mit einem oder mehreren Resten R⁴ substituiert sein kann, oder einer Aralkylgruppe mit 5 bis 60 aromatischen Ringatomen, die jeweils mit einem oder mehreren Resten R⁴ substituiert sein kann, wobei die beiden Reste R¹ miteinander verknüpft sein können und einen Ring bilden können, so dass eine Spiroverbindung in Position 9 des Fluorens entsteht, wobei Spirobifluorene ausgeschlossen sind;
- R², R³: sind bei jedem Auftreten gleich oder verschieden, bevorzugt gleich, H, D, F, Cl, Br, I, C(=O)R⁴, CN, Si(R⁴)₃, NO₂, P(=O)(R⁴)₂, S(=O)R⁴, S(=O)₂R⁴, N(R⁴)₂, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁴ substituiert sein können und wobei eine oder mehrere CH₂-Gruppen in den oben genannten Gruppen durch -R⁴C=CR⁴-, -C=C-, Si(R⁴)₂, C=O, C=S, C=NR⁴, -C(=O)O-, -C(=O)NR⁴-, P(=O)(R⁴), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches Ringsystem mit 6 bis 30 aromatischen Ringatomen, das jeweils durch einen oder mehrere Reste R⁴ substituiert sein kann;
- R⁴: ist bei jedem Auftreten gleich oder verschieden H, D, F, Cl, Br, I, C(=O)R⁵, CN, Si(R⁵)₃, NO₂, P(=O)(R⁵)₂, S(=O)R⁵, S(=O)₂R⁵, , N(R⁵)₂, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁵ substituiert sein können und wobei eine oder mehrere CH₂-Gruppen in den oben genannten Gruppen durch -R⁵C=CR⁵-, -C≡C-, Si(R⁵)₂, C=O, C=S, C=NR⁵, -C(=O)O-, -C(=O)NR⁵-, P(=O)(R⁵), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches Ringsystem mit 5 bis 30 aromatischen Ringatomen, das jeweils durch einen oder mehrere Reste R⁵ substituiert sein kann;
- R⁵: ist ausgewählt aus der Gruppe bestehend aus H, D, F, einem aliphatischem Kohlenwasserstoffrest mit 1 bis 20 C-Atomen, in dem ein oder mehrere H-Atome durch D oder F ersetzt sein können, wobei zwei oder mehr benachbarte Substituenten R⁵ miteinander ein mono- oder polycyclisches, aliphatisches, Ringsystem bilden können;
- r: ist 1;
- p, q: sind 0;
- Z^{a}₀, Z^{b}₀: sind gleich oder verschieden bei jedem Auftreten gleich R³
- Z^{c}₁: ist gleich 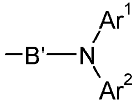
- B': ist eine Einfachbindung, eine Arylgruppe mit 6 bis 30 Ringatomen oder eine mono- oder bicyclische Heteroarylgruppe mit 5 bis 30 Ringatomen, die jeweils mit einem oder mehreren Resten R⁴ substituiert sein können, bevorzugt eine Einfachbindung oder eine Phenylen-, Biphenylen-, Terphenylen-, Naphthylen-, Pyridinylen-, Pyrimidinylen-, Pyrazinylen-, Pyridazinylen-, Triazinylen-, Dibenzofuranylen- oder Dibenzothiophenylengruppe, die mit einem oder mehreren Resten R⁴ substituiert sein können, ganz bevorzugt eine Einfachbindung oder eine Phenylen-, Biphenylen-, Terphenylen-, Naphthylen-, Dibenzofuranylen- oder Dibenzothiophenylengruppe, die mit einem oder mehreren Resten R⁴ substituiert sein können, ganz besonders bevorzugt ist B' eine Einfachbindung oder eine Phenylengruppe, die mit einem oder mehreren Resten R⁴ substituiert sein kann, insbesondere bevorzugt ist B' eine Einfachbindung, wobei, wenn B' eine Einfachbindung ist, das Stickstoffatom direkt an das Fluoren gebunden ist.
- Ar¹, Ar²: sind bei jedem Auftreten gleich oder verschieden ausgewählt aus den folgenden Gruppen der Formeln (37) bis (66), (69) bis (72) und (76) bis (96), die mit einem oder mehreren Resten R⁶ substituiert sein können 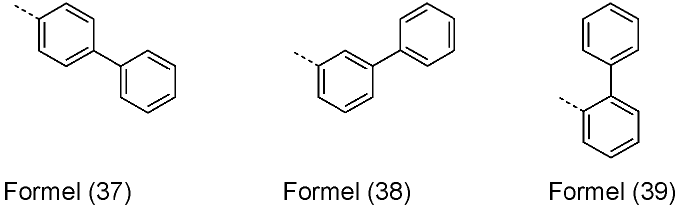 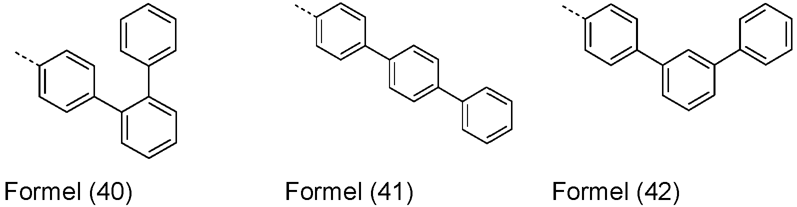 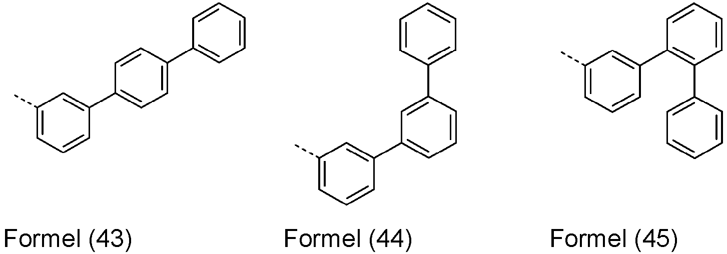 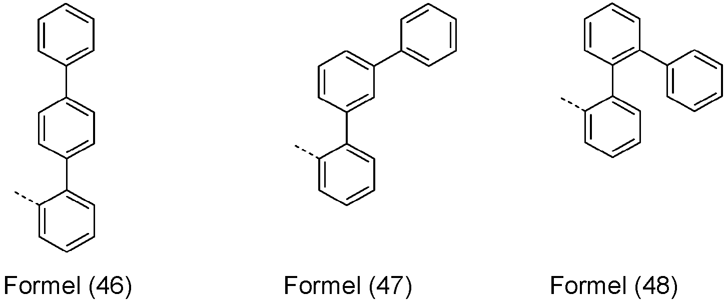 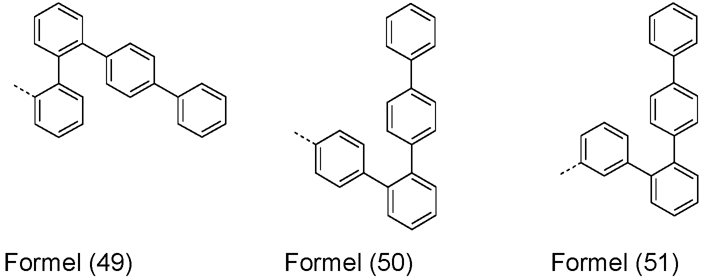 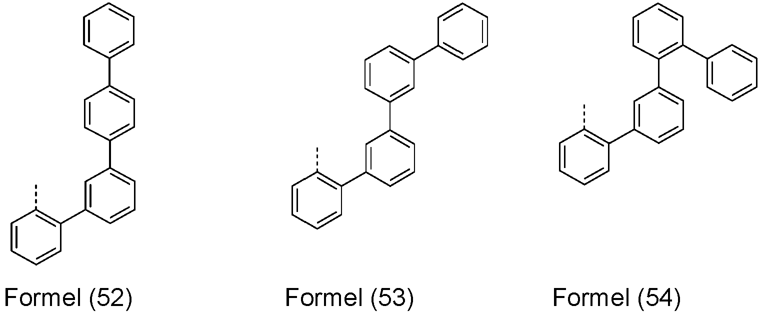 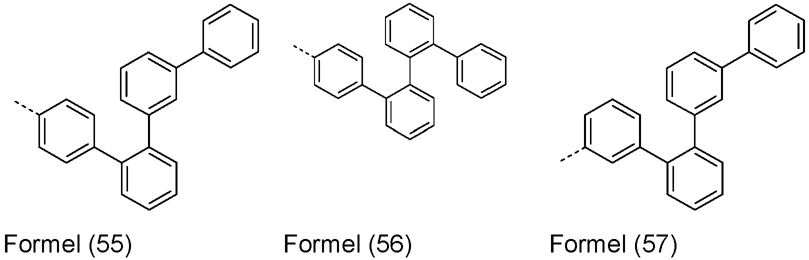 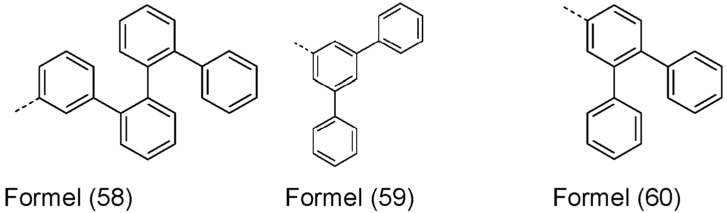 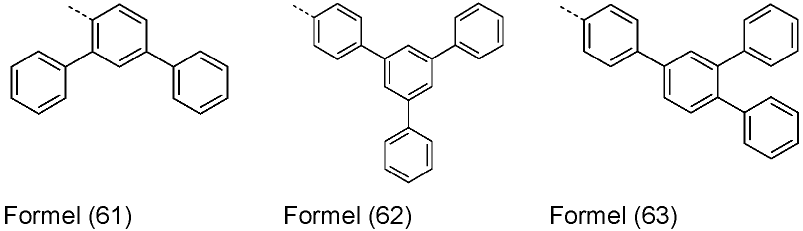 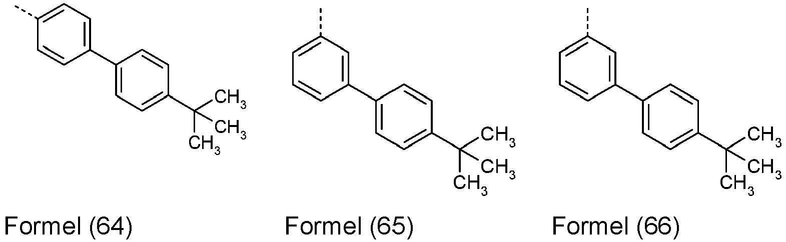 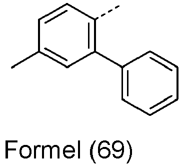 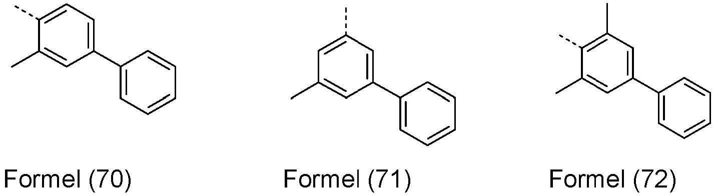 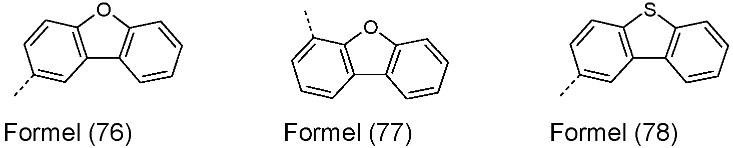 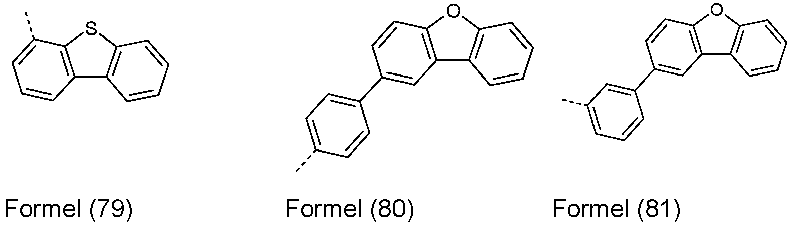 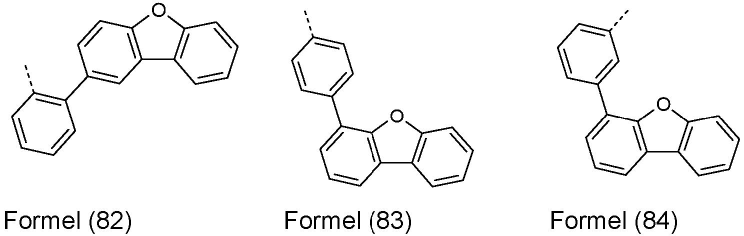 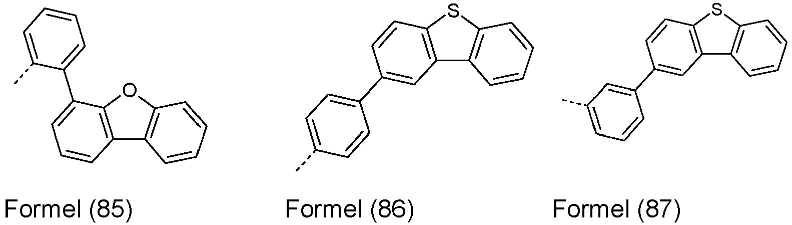 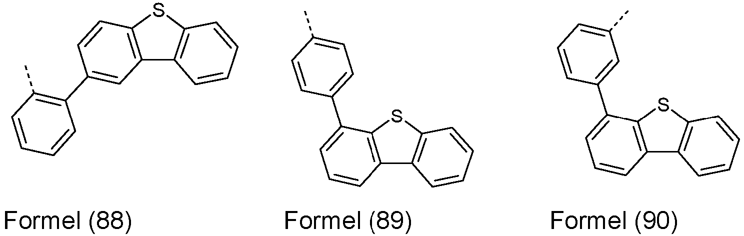 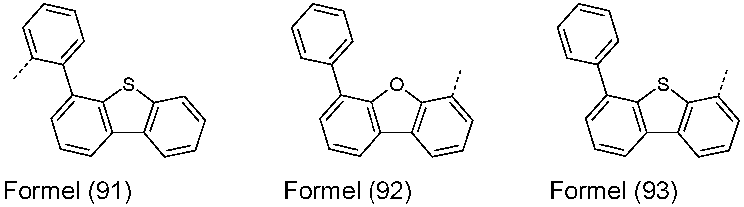 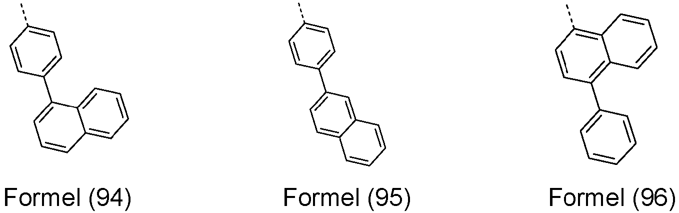 wobei die gestrichelte Linie die Verknüpfungsposition zum Stickstoffatom kennzeichnet;
- R⁶: ist bei jedem Auftreten gleich oder verschieden H, D, F, Cl, Br, I, C(=O)R⁵, CN, Si(R⁵)₃, NO₂, P(=O)(R⁵)₂, S(=O)R⁵, S(=O)₂R⁵, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁵ substituiert sein können und wobei eine oder mehrere CH₂-Gruppen in den oben genannten Gruppen durch -R⁵C=CR⁵-, -C=C-, Si(R⁵)₂, C=O, C=S, C=NR⁵, -C(=O)O-, -C(=O)NR⁵-, P(=O)(R⁵), -O-, -S-, SO oder SO₂ ersetzt sein können und wobei ein oder mehrere H-Atome in den oben genannten Gruppen durch D, F, Cl, Br, I, CN oder NO₂ ersetzt sein können.

Weiterhin bevorzugt im Sinne der vorliegenden Erfindung ist, wenn die Verbindung nach Formel (1) keine weiteren Fluorene oder Carbazole enthält.

Die Zählweise am Fluoren ist dabei wie folgt festgelegt.

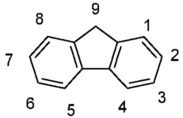

Eine Arylgruppe im Sinne dieser Erfindung enthält 6 bis 60 aromatische Ringatome; eine Heteroarylgruppe im Sinne dieser Erfindung enthält 5 bis 60 aromatische Ringatome, von denen mindestens eines ein Heteroatom darstellt. Die Heteroatome sind bevorzugt ausgewählt aus N, O und S. Dies stellt die grundlegende Definition dar. Werden in der Beschreibung der vorliegenden Erfindung andere Bevorzugungen angegeben, beispielsweise bezüglich der Zahl der aromatischen Ringatome oder der enthaltenen Heteroatome, so gelten diese.

Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin oder Thiophen, oder ein kondensierter (annellierter) aromatischer bzw. heteroaromatischer Polycyclus, beispielsweise Naphthalin, Phenanthren, Chinolin oder Carbazol verstanden. Ein kondensierter (annellierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Unter einer Aryloxygruppe gemäß der Definition der vorliegenden Erfindung wird eine Arylgruppe, wie oben definiert, verstanden, welche über ein Sauerstoffatom gebunden ist. Eine analoge Definition gilt für Heteroaryloxygruppen.

Ein aromatisches Ringsystem im Sinne dieser Erfindung enthält 6 bis 60 C-Atome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein sp³-hybridisiertes C-, Si-, N- oder O-Atom, ein sp²-hybridisiertes C- oder N-Atom oder ein sp-hybridisiertes C-Atom, verbunden sein können. So sollen beispielsweise auch Systeme wie 9,9'-Spirobifluoren, 9,9'-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Aryl- oder Heteroarylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin.

Unter einem aromatischen oder heteroaromatischen Ringsystem mit 5 - 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Kombinationen dieser Gruppen.

Im Rahmen der vorliegenden Erfindung werden unter einer geradkettigen Alkylgruppe mit 1 bis 40 C-Atomen bzw. einer verzweigten oder cyclischen Alkylgruppe mit 3 bis 40 C-Atomen bzw. einer Alkenyl- oder Alkinylgruppe mit 2 bis 40 C-Atomen, in der auch einzelne H-Atome oder CH₂-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer Alkoxy- oder Thioalkylgruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Es ist bevorzugt, wenn B' in der Verbindung nach Formel (1) eine Einfachbindung ist.

Es ist weiterhin bevorzugt, wenn B' in der Verbindung nach Formel (1) eine o-Phenylen-, m-Phenylen- oder p-Phenylengruppe, eine 1,4-Naphthylen-, 2,4-Naphthylen-, 1,5-Naphthylen- oder 2,5-Naphthylengruppe, eine 3,7-Dibenzofuranylengruppe oder eine 3,7-Dibenzothiophenylengruppe ist, wobei ganz bevorzugt ist, wenn B' eine o-Phenylen-, m-Phenylen- oder p-Phenylengruppe und ganz besonders bevorzugt ist, wenn B' eine p-Phenylengruppe ist, wobei die Gruppen mit einem oder mehreren Resten R⁴ substituiert sein können, die bei jedem Auftreten gleich oder verschieden sein können, wobei bevorzugt ist, wenn die Gruppen unsubstituiert sind.

Weiterhin bevorzugt ist eine Verbindung der allgemeinen Formel (1), dadurch charakterisiert, dass R¹ bei jedem Auftreten gleich oder verschieden, bevorzugt gleich, eine geradkettige Alkyl- oder Alkoxygruppe mit 1 bis 20 C-Atomen oder eine verzweigte oder cyclische Alkyl- oder Alkoxygruppe mit 3 bis 20 C-Atomen ist, wobei die oben genannten Gruppen jeweils mit einem oder mehreren Resten R⁴ substituiert sein können oder ein aromatisches oder heteroaromatisches Ringsystem mit 6 bis 30 aromatischen Ringatomen, das jeweils durch einen oder mehrere Reste R⁴ substituiert sein kann, oder eine Aryloxygruppe mit 5 bis 60 aromatischen Ringatomen, die mit einem oder mehreren Resten R⁴ substituiert sein kann, oder einer Aralkylgruppe mit 5 bis 60 aromatischen Ringatomen, die jeweils mit einem oder mehreren Resten R⁴ substituiert sein kann, wobei die beiden Reste R¹ miteinander verknüpft sein können und einen Ring bilden können, so dass eine Spiroverbindung in Position 9 des Fluorens entsteht, wobei Spirobifluorene ausgeschlossen sind.

Weiterhin ganz bevorzugt ist eine Verbindung der allgemeinen Formel (1), dadurch charakterisiert, dass R¹ bei jedem Auftreten gleich oder verschieden, bevorzugt gleich, eine geradkettige Alkylgruppe mit 1 bis 20 C-Atomen oder eine verzweigte oder cyclische Alkylgruppe mit 3 bis 20 C-Atomen ist, wobei die oben genannten Gruppen jeweils mit einem oder mehreren Resten R⁴ substituiert sein können oder ein aromatisches oder heteroaromatisches Ringsystem mit 6 bis 30 aromatischen Ringatomen, das jeweils durch einen oder mehrere Reste R⁴ substituiert sein kann, wobei die beiden Reste R¹ miteinander verknüpft sein können und einen Ring bilden können, so dass eine Spiroverbindung in Position 9 des Fluorens entsteht, wobei Spirobifluorene ausgeschlossen sind.

Weiterhin ganz besonders bevorzugt ist eine Verbindung der allgemeinen Formel (1), dadurch charakterisiert, dass R¹ bei jedem Auftreten gleich eine geradkettige Alkylgruppe mit 1 bis 20 C-Atomen oder eine verzweigte oder cyclische Alkylgruppe mit 3 bis 20 C-Atomen ist, wobei die oben genannten Gruppen jeweils mit einem oder mehreren Resten R⁴ substituiert sein können, wobei insbesondere bevorzugt ist, wenn R¹ eine Methyl-, Ethyl-, n-/i-Propyl- oder n-/i-/t-Butylgruppe ist.

Schließlich ist weiterhin ganz besonders bevorzugt eine Verbindung der allgemeinen Formel (1), dadurch charakterisiert, dass R¹ bei jedem Auftreten ein aromatisches oder heteroaromatisches Ringsystem mit 6 bis 30 aromatischen Ringatomen ist, das jeweils durch einen oder mehrere Reste R⁴ substituiert sein kann, wobei das Ringsystem insbesondere bevorzugt ausgewählt ist aus der Gruppe bestehend aus einer Phenyl-, Biphenyl-, Terphenyl- oder Pyridylgruppe.

Weiterhin bevorzugt ist eine Verbindung der allgemeinen Formel (1), dadurch charakterisiert, dass R² bei jedem Auftreten gleich oder verschieden, bevorzugt gleich, ausgewählt ist aus H, D, F, Cl, Br, I, N(R⁵)₂, einer geradkettigen Alkyl-, Alkoxylgruppe mit 1 bis 20 C-Atomen oder einer verzweigten oder cyclischen Alkyl-, Alkoxygruppe mit 3 bis 20 C-Atomen oder einer Alkenyl- oder Alkinylgruppe mit 2 bis 20 C-Atomen, wobei die oben genannten Gruppen jeweils mit einem oder mehreren Resten R⁴ substituiert sein können und wobei ein oder mehrere H-Atome in den oben genannten Gruppen durch D, F, Cl, Br, I, CN oder NO₂ ersetzt sein können, oder ein aromatisches Ringsystem mit 6 bis 30 aromatischen Ringatomen, das jeweils durch einen oder mehrere Reste R⁴ substituiert sein kann.

Weiterhin ganz bevorzugt ist eine Verbindung der allgemeinen Formel (1), dadurch charakterisiert, dass R² bei jedem Auftreten gleich oder verschieden, bevorzugt gleich, ausgewählt ist aus H, D, F, Cl, Br, I, N(R⁵)₂, einer geradkettigen Alkylgruppe mit 1 bis 20 C-Atomen oder einer verzweigten oder cyclischen Alkylgruppe mit 3 bis 20 C-Atomen, wobei die oben genannten Gruppen jeweils mit einem oder mehreren Resten R⁴ substituiert sein können und wobei ein oder mehrere H-Atome in den oben genannten Gruppen durch D, F, Cl, Br, I, CN oder NO₂ ersetzt sein können, oder einem aromatischen Ringsystem mit 6 bis 30 aromatischen Ringatomen, das jeweils durch einen oder mehrere Reste R⁴ substituiert sein kann.

In einer ganz besonders bevorzugten Ausführungsform betrifft die vorliegende Erfindung eine Verbindung der allgemeinen Formel (1), die dadurch charakterisiert ist, dass R² gleich H ist.

In einer weiteren ganz besonders bevorzugten Ausführungsform betrifft die vorliegende Erfindung eine Verbindung der allgemeinen Formel (1), die dadurch charakterisiert ist, dass R² eine geradkettigen Alkylgruppe mit 1 bis 20 C-Atomen oder einer verzweigten oder cyclischen Alkylgruppe mit 3 bis 20 C-Atomen ist.

In noch einer weiteren ganz besonders bevorzugten Ausführungsform betrifft die vorliegende Erfindung eine Verbindung der allgemeinen Formel (1), die dadurch charakterisiert ist, dass R² ein aromatisches Ringsystem mit 6 bis 30 aromatischen Ringatomen darstellt.

Weiterhin bevorzugt ist eine Verbindung der allgemeinen Formel (1), dadurch charakterisiert, dass R³ bei jedem Auftreten gleich oder verschieden ausgewählt ist aus H, D, F, Cl, Br, I, N(R⁵)₂, eine geradkettige Alkyl- oder Alkoxygruppe mit 1 bis 20 C-Atomen oder eine verzweigte oder cyclische Alkyl- oder Alkoxygruppe mit 3 bis 20 C-Atomen, wobei die oben genannten Gruppen jeweils mit einem oder mehreren Resten R⁴ substituiert sein können und wobei ein oder mehrere H-Atome in den oben genannten Gruppen durch D, F, Cl, Br, I, CN oder NO₂ ersetzt sein können, oder ein aromatisches Ringsystem mit 6 bis 30 aromatischen Ringatomen, das jeweils durch einen oder mehrere Reste R⁴ substituiert sein kann.

In einer bevorzugten Ausführungsform betrifft die vorliegende Erfindung eine Verbindung der allgemeinen Formel (5) 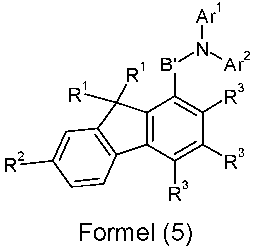 wobei für die verwendeten Symbole und Indices obige Definitionen gelten.

In einer weiterhin bevorzugten Ausführungsform betrifft die vorliegende Erfindung eine Verbindung der allgemeinen Formel (8) 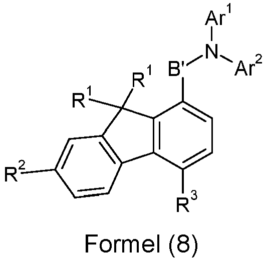 wobei für die verwendeten Symbole und Indices obige Definitionen gelten.

In einer weiterhin ganz besonders bevorzugten Ausführungsform betrifft die vorliegende Erfindung eine Verbindung der allgemeinen Formel (11) 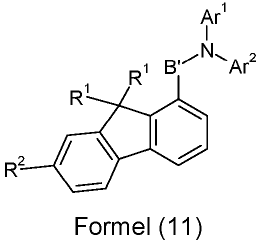 wobei für die verwendeten Symbole und Indices obige Definitionen gelten.

In einer weiterhin insbesondere bevorzugten Ausführungsform betrifft die vorliegende Erfindung eine Verbindung der allgemeinen Formel (14) 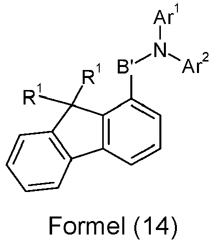 wobei für die verwendeten Symbole und Indices obige Definitionen gelten.

Weiterhin bevorzugt ist eine Verbindung der oben angegebenen Formeln in denen B' ausgewählt ist aus den Gruppen der Formeln (15) bis (36), wobei diese Gruppen noch durch ein oder mehrere voneinander unabhängige Reste R⁴ substituiert sein können und wobei R⁴ wie oben angegeben definiert ist. 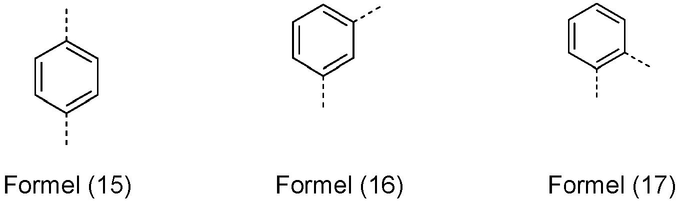 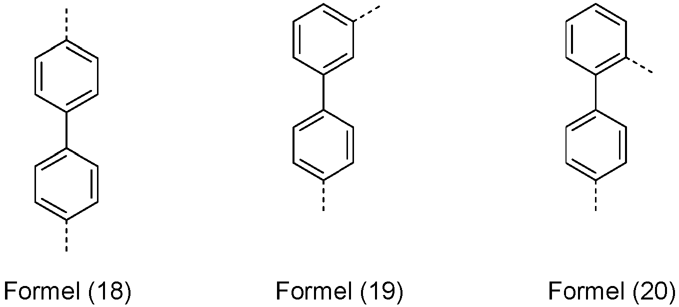 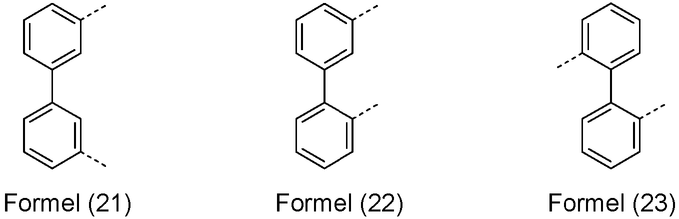 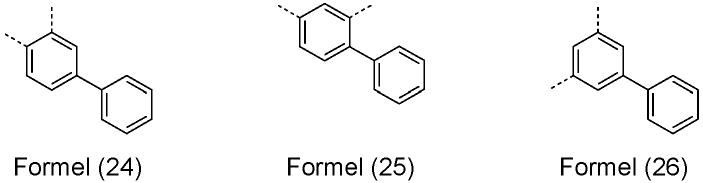 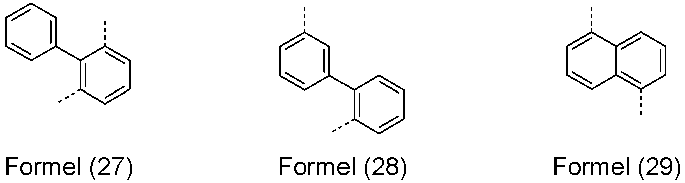 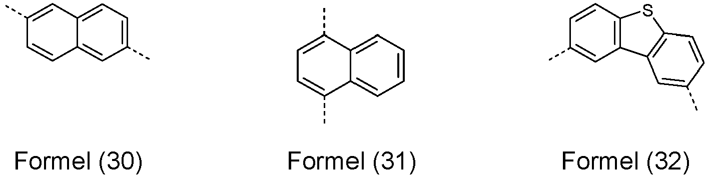 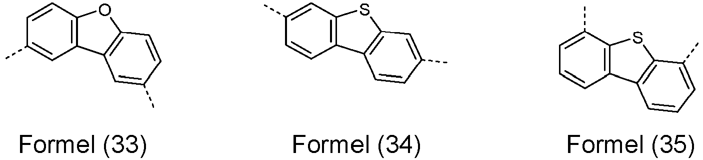 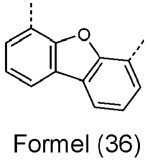 wobei die gestrichelten Linien die Verknüpfungspositionen kennzeichnen.

Ganz bevorzugt ist eine Verbindung der oben angegebenen Formeln (1) bis (14) in denen B' ausgewählt ist aus den Gruppen der Formeln (15) bis (36), wobei diese Gruppen unsubstituiert sind.

Ganz besonders bevorzugt ist eine Verbindung der oben angegebenen Formeln (1) bis (14) in denen B' der Formel (15) entspricht, wobei diese Gruppe unsubstituiert ist.

Insbesonders bevorzugt ist eine Verbindung der Formeln (1) bis (14), dadurch gekennzeichnet, dass B' eine Einfachbindung ist, wobei dann gilt, dass das Stickstoffatom direkt über eine Einfachbindung an das Fluoren gebunden ist.

Die vorliegende Erfindung betrifft in einer weiteren bevorzugten Ausführungsform eine Verbindung der allgemeinen Formel (8), wobei für die verwendeten Symbole gilt, dass
- R¹: bei jedem Auftreten gleich oder verschieden, bevorzugt gleich, eine geradkettige Alkylgruppe mit 1 bis 20 C-Atomen oder eine verzweigte oder cyclische Alkylgruppe mit 3 bis 20 C-Atomen ist, wobei die oben genannten Gruppen jeweils mit einem oder mehreren Resten R⁴ substituiert sein können oder ein aromatisches oder heteroaromatisches Ringsystem mit 6 bis 30 aromatischen Ringatomen, das jeweils durch einen oder mehrere Reste R⁴ substituiert sein kann, wobei die beiden Reste R¹ miteinander verknüpft sein können und einen Ring bilden können, so dass eine Spiroverbindung in Position 9 des Fluorens entsteht, wobei Spirobifluorene ausgeschlossen sind;
- R²: gleich H, eine geradkettigen Alkylgruppe mit 1 bis 20 C-Atomen oder einer verzweigten oder cyclischen Alkylgruppe mit 3 bis 20 C-Atomen ist, wobei die Gruppen durch einen oder mehrere Reste R⁴ substituiert sein können, ein aromatisches Ringsystem mit 6 bis 30 aromatischen Ringatomen, das durch ein oder mehrere Reste R⁴ substituiert sein kann;
- R³: gleich H, D, F, Cl, Br, I, eine geradkettige Alkyl- oder Alkoxygruppe mit 1 bis 20 C-Atomen oder eine verzweigte oder cyclische Alkyl-oder Alkoxygruppe mit 3 bis 20 C-Atomen ist, wobei die oben genannten Gruppen jeweils mit einem oder mehreren Resten R⁴ substituiert sein können und wobei ein oder mehrere H-Atome in den oben genannten Gruppen durch D, F, Cl, Br, I, CN oder NO₂ ersetzt sein können, oder ein aromatisches Ringsystem mit 6 bis 30 aromatischen Ringatomen, das jeweils durch einen oder mehrere Reste R⁴ substituiert sein kann;
- B': ausgewählt ist aus H oder den Gruppen der Formeln (15) bis (36), wobei diese Gruppen noch durch ein oder mehrere voneinander unabhängige Reste R⁴ substituiert sein können und wobei R⁴ wie oben angegeben definiert ist;
- Ar¹, Ar²: gleich oder verschieden bei jedem Auftreten, eine Phenyl-Naphthyl-, Biphenyl-, Terphenyl- oder Quarterphenylgruppe sind, die mit einem oder mehreren Resten R⁶, die gleich oder verschieden voneinander sein können, substituiert sein können, wobei die Ringe in Ar¹ bzw. in Ar² unverbrückte Ringe sind.

Die vorliegende Erfindung betrifft in einer ganz bevorzugten Ausführungsform eine Verbindung der allgemeinen Formel (8), wobei für die verwendeten Symbole gilt, dass
- R¹: bei jedem Auftreten gleich oder verschieden, bevorzugt gleich, eine geradkettige Alkylgruppe mit 1 bis 20 C-Atomen ist, wobei die genannte Gruppe mit einem oder mehreren Resten R⁴ substituiert sein kann oder ein aromatisches oder heteroaromatisches Ringsystem mit 6 bis 30 aromatischen Ringatomen, das jeweils durch einen oder mehrere Reste R⁴ substituiert sein kann, wobei die beiden Reste R¹ miteinander verknüpft sein können und einen Ring bilden können, so dass eine Spiroverbindung in Position 9 des Fluorens entsteht, wobei Spirobifluorene ausgeschlossen sind;
- R²: gleich H, eine geradkettigen Alkylgruppe mit 1 bis 20 C-Atomen oder einer verzweigten oder cyclischen Alkylgruppe mit 3 bis 20 C-Atomen ist, wobei die Gruppen durch einen oder mehrere Reste R⁴ substituiert sein können, ein aromatisches Ringsystem mit 6 bis 30 aromatischen Ringatomen, das durch ein oder mehrere Reste R⁴ substituiert sein kann;
- R³: gleich H oder ein aromatisches Ringsystem mit 6 bis 30 aromatischen Ringatomen, das jeweils durch einen oder mehrere Reste R⁴ substituiert sein kann, wobei R³ gleich H insbesondere bevorzugt ist;
- B': eine Einfachbindung ist;
- Ar¹, Ar²: gleich oder verschieden bei jedem Auftreten, eine Biphenyl-, Terphenyl- oder Quarterphenylgruppe sind, die mit einem oder mehreren Resten R⁶, die gleich oder verschieden voneinander sein können, substituiert sein können, wobei die Ringe in Ar¹ und Ar² unverbrückt sind, insbesondere bevorzugt sind Ar¹ und Ar² eine Biphenylgruppe, die mit einem oder mehreren Resten R⁶, die gleich oder verschieden voneinander sein können, substituiert sein kann.

Die vorliegende Erfindung betrifft in einer ganz besonders bevorzugten Ausführungsform eine Verbindung der allgemeinen Formel (8), wobei für die verwendeten Symbole gilt, dass
- R¹: bei jedem Auftreten gleich ist und eine geradkettige Alkylgruppe mit 1 bis 5 C-Atomen, bevorzugt eine Methylgruppe oder Ethylgruppe, ist, wobei die genannte Gruppe mit einem oder mehreren Resten R⁴ substituiert sein kann oder eine Phenyl-, Biphenyl, Pyridylgruppe, die jeweils durch einen oder mehrere Reste R⁴ substituiert sein kann, darstellt, wobei die beiden Alkylgruppen nach R¹ miteinander verknüpft sein können und einen Ring bilden können, so dass eine Spiroverbindung in Position 9 des Fluorens entsteht, wobei Spirobifluorene ausgeschlossen sind;
- R²: gleich H, eine geradkettigen Alkylgruppe mit 1 bis 20 C-Atomen oder einer verzweigten oder cyclischen Alkylgruppe mit 3 bis 20 C-Atomen ist, wobei die Gruppen durch einen oder mehrere Reste R⁴ substituiert sein können, ein aromatisches Ringsystem mit 6 bis 30 aromatischen Ringatomen, das durch ein oder mehrere Reste R⁴ substituiert sein kann;
- R³: gleich H oder ein aromatisches Ringsystem mit 6 bis 30 aromatischen Ringatomen, das jeweils durch einen oder mehrere Reste R⁴ substituiert sein kann, wobei R³ gleich H insbesondere bevorzugt ist;
- B': eine Einfachbindung ist;
- Ar¹, Ar²: gleich oder verschieden bei jedem Auftreten, eine Biphenyl-, Terphenyl- oder Quarterphenylgruppe sind, die mit einem oder mehreren Resten R⁶, die gleich oder verschieden voneinander sein können, substituiert sein können, wobei die Ringe in Ar¹ und Ar² unverbrückt sind, insbesondere bevorzugt sind Ar¹ und Ar² eine Biphenylgruppe, die mit einem oder mehreren Resten R⁶, die gleich oder verschieden voneinander sein können, substituiert sein kann.

In einer insbesonders bevorzugten Ausführungsform betrifft die vorliegende Erfindung Verbindungen der allgemeinen Formel (1) dadurch charakterisiert, dass es sich um Monoaminverbindungen handelt.

Die Synthese der erfindungsgemäßen Verbindungen kann nach Verfahren hergestellt werden, die dem Fachmann aus dem Stand der Technik bekannt sind. Die Herstellung kann, beispielsweise, mittels Halogenierung, Buchwald-Kupplung und Suzuki-Kupplung erfolgen.

Das folgende Reaktionsschema zeigt einen bevorzugten Syntheseweg zur Herstellung der erfindungsgemäßen Verbindungen der Formel (1). Zur Synthese der erfindungsgemäßen Verbindungen wird an das Fluoren-Verbindung A in einer Buchwald-Kupplung mit einem Amin B der Formel Ar¹-NH-Ar² umgesetzt 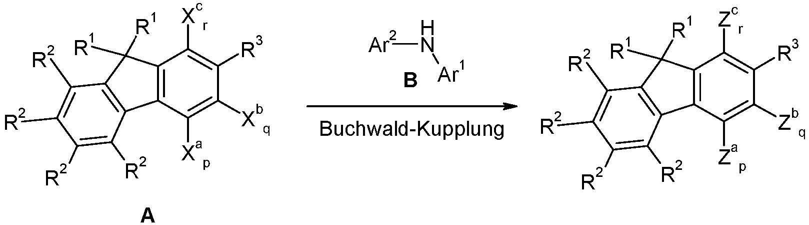 wobei für die verwendeten Symbole und Indices obige Definitionen gelten und wobei
X^{a}₀, X^{b}₀ gleich oder verschieden bei jedem Auftreten gleich R³ und
X^{c}₁ ist gleich -B'-Y, wobei Y eine Abgangsgruppe, beispielsweise Halogen, sind.

Ein anderer bevorzugter Syntheseweg zur Herstellung der erfindungsgemäßen Verbindungen wird im folgenden Reaktionsschema dargestellt. Der Syntheseweg umfasst zwei Kupplungsreaktionen: zunächst wird das das Fluoren-Verbindung **A** in einer ersten Buchwald-Kupplung mit einem Amin **B** der Formel Ar¹-NH₂ umgesetzt. Schließlich erfolgt eine zweite Buchwald-Kupplung mit einer Verbindung D, beispielsweise mit einer Bromaryl-Verbindung. 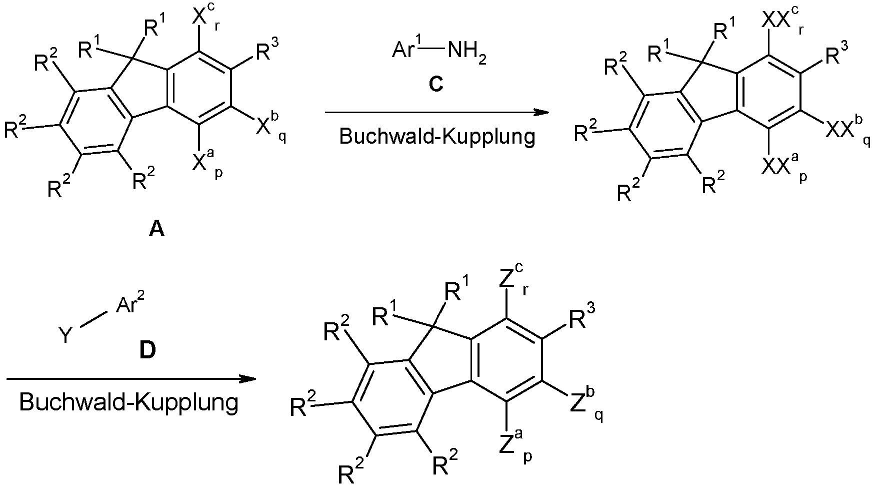 wobei Y wieder eine Abgangsgruppe, beispielsweise Halogen, ist;
und wobei
XX^{a}₀, XX^{b}₀ gleich oder verschieden bei jedem Auftreten gleich R³ und
XX^{c}₁ gleich -B'-NH-Ar¹ ist.

Das folgende Schema zeigt einen weiteren bevorzugten Syntheseweg zur Herstellung erfindungsgemäßer Verbindungen, wobei erfindungsgemäße Fluorene, die das Amin in Position 1 des Fluorens haben, analog hierzu dargestellt werden. Hierzu werden Benzochromenone **E** als Ausgangspunkt dienen. Die Addition von einem Organometallo-Reagenz beispielweise Grignard- oder Organolithium-Reagenz und anschließende säurekatalysierte Cyclisierung des intermediären Alkoholats führt zu den entsprechenden 4-hydroxy-Fluorenen **F.** Anschließend wird die Hydroxygruppe in eine Abgangsgruppe Y bzw. -B'-Y(=X^{a}₁), beispielweise in ein Triflat (TfO) oder ein Halogenid (bevorzugt Br oder Cl), umgewandelt und nach einer dem Fachmann geläufigen Methode (C-C-Kuplung wie Zusuki-, Negishi-, Yamamoto-, Grignard-Cross-, Stille-, Heck-Kupplung, etc.; C-N-Kupplung wie Buchwald- Kupplung) weiter zu den erfindungsgemäßen Verbindungen umgesetzt, wobei eine Buchwald Kupplung oder eine Suzuki Kupplung bevorzugt sind. Im Fall von X^{a}₁ ist selbstverständlich lediglich Y die Abgangsgruppe. 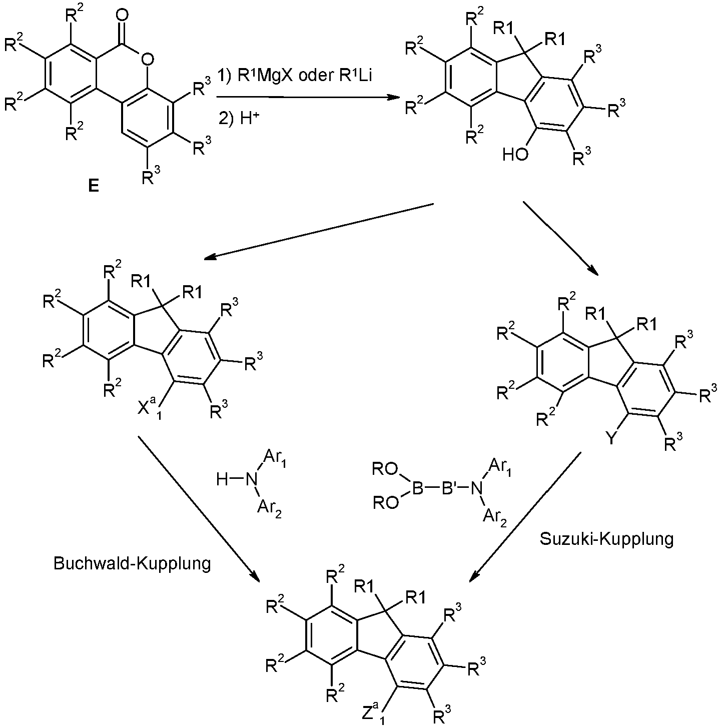 wobei die verwendeten Indizes wie oben angegeben definiert sind und B für ein Boratom steht.

Hiedurch lassen sich Fluorene darstellen, die das Amin in der bevorzugten Position 4 haben.

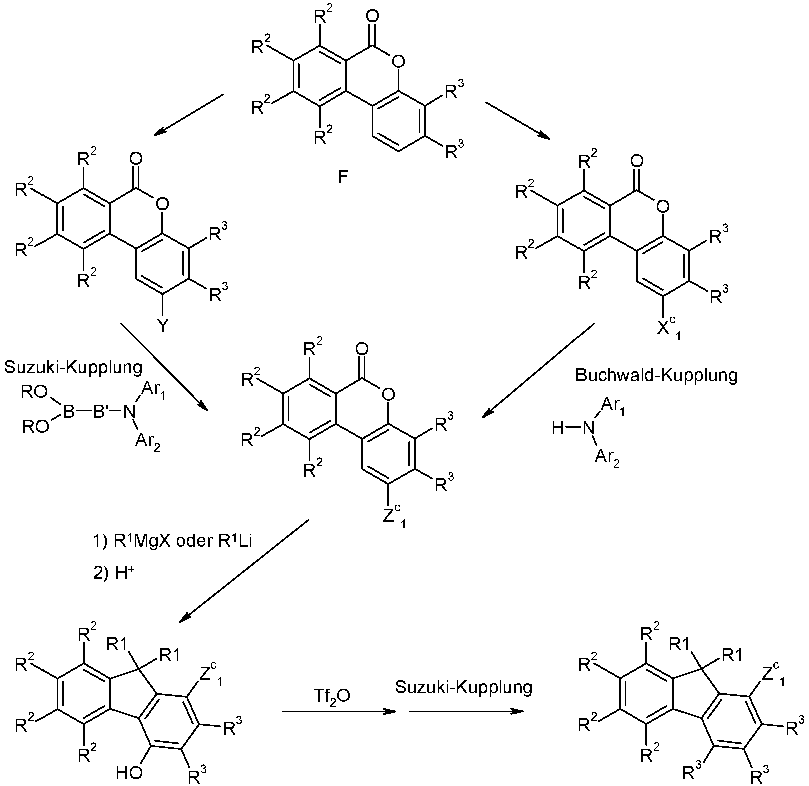

Für die Suzuki-Kupplng eingesetzte Boronsäureester mit den Gruppen R sind dem Fachmann gute bekannt.

Synthesewege für die Ausgangsverbindungen **A,** B, **C,** D, **E** und F. welche in der Synthese der erfindungsgemäßen Verbindungen eingesetzt werden, sind dem Fachmann geläufig. Weiterhin werden in den Ausführungsbeispielen einige explizite Syntheseverfahren im Detail dargestellt.

Bevorzugte Kupplungsreaktionen zur Herstellung der Verbindung der allgemeinen Formel (1) sind Buchwald-Kupplungen.

Bevorzugte erfindungsgemäße Verbindungen sind in der nachfolgenden Tabelle exemplarisch aufgeführt.

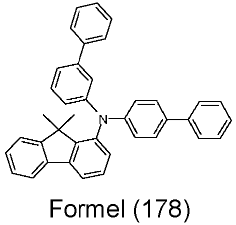

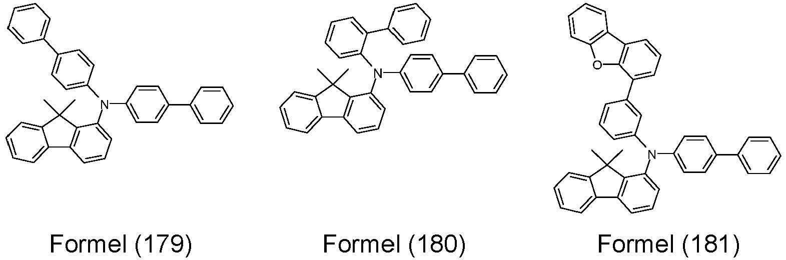

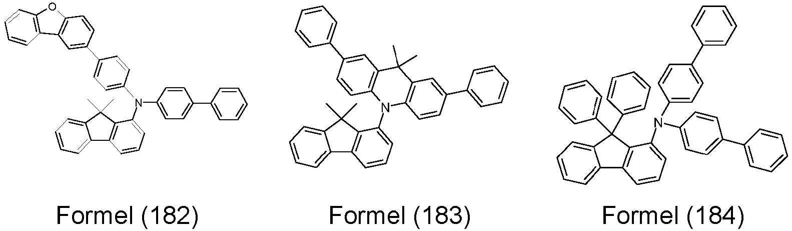

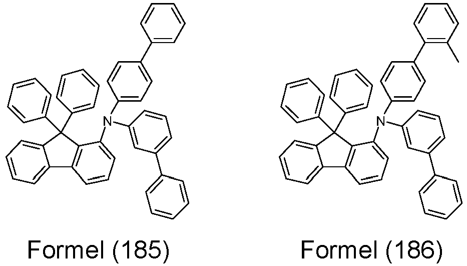

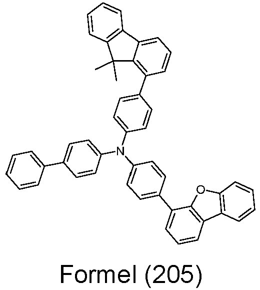

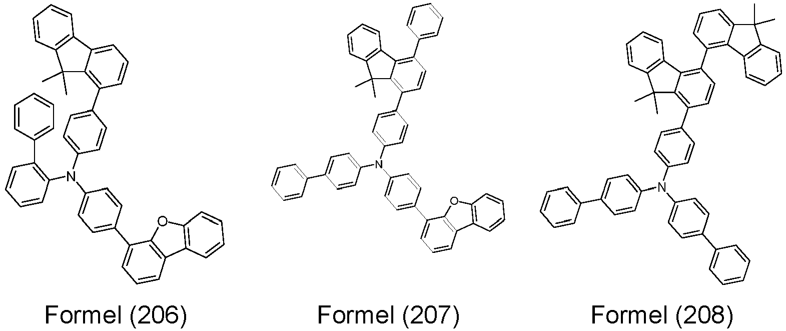

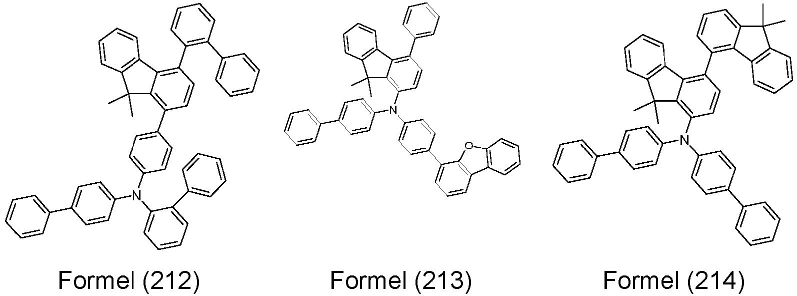

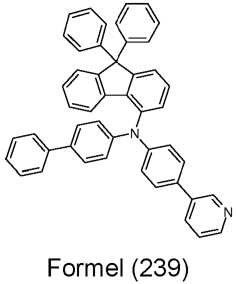

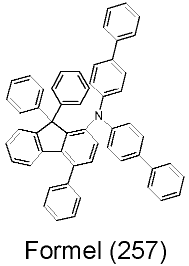

Die oben beschriebenen Verbindungen der Formel (1) können mit reaktiven Abgangsgruppen, wie Brom, Iod, Chlor, Boronsäure oder Boronsäureester substituiert werden. Diese können als Monomere zur Erzeugung entsprechender Oligomere, Dendrimere oder Polymere Verwendung finden. Geeignete reaktive Abgangsgruppen sind beispielsweise Brom, Iod, Chlor, Boronsäuren, Boronsäureester, Amine, Alkenyl- oder Alkinylgruppen mit endständiger C-C-Doppelbindung bzw. C-C-Dreifachbindung, Oxirane, Oxetane, Gruppen, die eine Cycloaddition, beispielsweise eine 1,3-dipolare Cycloaddition, eingehen, wie beispielsweise Diene oder Azide, Carbonsäurederivate, Alkohole und Silane.

Weiterer Gegenstand der Erfindung sind daher Oligomere, Polymere oder Dendrimere enthaltend eine oder mehrere Verbindungen gemäß Formel (1), wobei die Bindung(en) zum Polymer, Oligomer oder Dendrimer an beliebigen in Formel (1) möglichen Positionen lokalisiert sein können. Je nach Verknüpfung der Verbindung gemäß Formel (1) ist die Verbindung Bestandteil einer Seitenkette des Oligomers oder Polymers oder Bestandteil der Hauptkette. Unter einem Oligomer im Sinne dieser Erfindung wird eine Verbindung verstanden, welche aus mindestens drei Monomereinheiten aufgebaut ist. Unter einem Polymer im Sinne der Erfindung wird eine Verbindung verstanden, die aus mindestens zehn Monomereinheiten aufgebaut ist. Die erfindungsgemäßen Polymere, Oligomere oder Dendrimere können konjugiert, teilkonjugiert oder nichtkonjugiert sein. Die erfindungsgemäßen Oligomere oder Polymere können linear, verzweigt oder dendritisch sein. In den linear verknüpften Strukturen können die Einheiten gemäß Formel (1) ddirekt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe miteinander verknüpft sein. In verzweigten und dendritischen Strukturen können beispielsweise drei oder mehrere Einheiten gemäß Formeln (1) über eine trivalente oder höhervalente Gruppe, beispielsweise über eine trivalente oder höhervalente aromatische oder heteroaromatische Gruppe, zu einem verzweigten bzw. dendritischen Oligomer oder Polymer verknüpft sein.

Für die Wiederholeinheiten gemäß Formel (1) in Oligomeren, Dendrimeren und Polymeren gelten dieselben Bevorzugungen wie oben für Verbindungen gemäß Formel (1) beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 2000/22026), Spirobifluorenen (z. B. gemäß EP 707020, EP 894107 oder WO 2006/061181), Paraphenylenen (z. B. gemäß WO 1992/18552), Carbazolen (z. B. gemäß WO 2004/070772 oder WO 2004/113468), Thiophenen (z. B. gemäß EP 1028136), Dihydrophenanthrenen (z. B. gemäß WO 2005/014689 oder WO 2007/006383), cis- und trans-Indenofluorenen (z. B. gemäß WO 2004/041901 oder WO 2004/113412), Ketonen (z. B. gemäß WO 2005/040302), Phenanthrenen (z. B. gemäß WO 2005/104264 oder WO 2007/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 2007/068325) oder phosphoreszierende Metallkomplexe (z. B. gemäß WO 2006/003000), und/oder Ladungstransporteinheiten, insbesondere solche basierend auf Triarylaminen.

Die erfindungsgemäßen Polymere, Oligomere und Dendrimere weisen vorteilhafte Eigenschaften, insbesondere hohe Lebensdauern, hohe Effizienzen und gute Farbkoordinaten auf.

Die erfindungsgemäßen Polymere und Oligomere werden in der Regel durch Polymerisation von einer oder mehreren Monomersorten hergestellt, von denen mindestens ein Monomer im Polymer zu Wiederholungseinheiten der Formel (1) führt. Geeignete Polymerisationsreaktionen sind dem Fachmann bekannt und in der Literatur beschrieben. Besonders geeignete und bevorzugte Polymerisationsreaktionen, die zu C-C- bzw. C-N-Verknüpfungen führen, sind folgende:
(A) SUZUKI-Polymerisation;
(B) YAMAMOTO-Polymerisation;
(C) STILLE-Polymerisation; und
(D) HARTWIG-BUCHWALD-Polymerisation.

Wie die Polymerisation nach diesen Methoden durchgeführt werden kann und wie die Polymere dann vom Reaktionsmedium abgetrennt und aufgereinigt werden können, ist dem Fachmann bekannt und in der Literatur, beispielsweise in WO 2003/048225, WO 2004/037887 und WO 2004/037887, im Detail beschrieben.

Gegenstand der vorliegenden Erfindung ist somit auch ein Verfahren zur Herstellung der erfindungsgemäßen Polymere, Oligomere und Dendrimere, das dadurch gekennzeichnet ist, dass sie durch Polymerisation gemäß SUZUKI, Polymerisation gemäß YAMAMOTO, Polymerisation gemäß STILLE oder Polymerisation gemäß HARTWIG-BUCHWALD hergestellt werden. Die erfindungsgemäßen Dendrimere können gemäß dem Fachmann bekannten Verfahren oder in Analogie dazu hergestellt werden. Geeignete Verfahren sind in der Literatur beschrieben, wie z. B. in Frechet, Jean M. J.; Hawker, Craig J., "Hyperbranched polyphenylene and hyperbranched polyesters: new soluble, three-dimensional, reactive polymers", Reactive & Functional Polymers (1995), 26(1-3), 127-36; Janssen, H. M.; Meijer, E. W., "The synthesis and characterization of dendritic molecules", Materials Science and Technology (1999), 20 (Synthesis of Polymers), 403-458; Tomalia, Donald A., "Dendrimer molecules", Scientific American (1995), 272(5), 62-6; WO 2002/067343 A1 und WO 2005/026144 A1.

Die erfindungsgemäßen Verbindungen, Polymere, Oligomere und Dendrimerekönnen mit anderen organisch funktionellen Materialien, die in elektronischen Vorrichtungen verwendet werden, als Zusammensetzungen eingesetzt werden. Dem Fachmann ist hierbei eine Vielzahl möglicher organisch funktioneller Materialien aus dem Stand der Technik bekannt. Die vorliegende Erfindung betrifft daher auch eine Zusammensetzung enthaltend eine oder mehrere erfindungsgemäße Verbindungen der Formel (1) oder wenigstes ein erfindungsgemäßes Polymer, Oligomer oder Dendrimer und wenigstens ein weiteres organisch funktionelles Material ausgewählt aus der Gruppe bestehend aus fluoreszierenden Emitter, phosphoreszierenden Emitter, Host Materialien, Matrix Materialien, Elektronentransportmaterialien, Elektroneninjektionsmaterialien, Lochleitermaterialien, Lochinjektionsmaterialien, Elektronenblockiermaterialien und Lochblockiermaterialien.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Miniemulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Dimethylanisol, Mesitylen, Tetralin, Veratrol, THF, Methyl-THF, THP, Chlorbenzol, Dioxan oder Mischungen dieser Lösemittel.

Gegenstand der Erfindung ist daher weiterhin eine Formulierung, insbesondere eine Lösung, Dispersion oder Miniemulsion, enthaltend mindestens eine Verbindung gemäß Formel (1) oder mindestens ein Polymer, Oligomer oder Dendrimer enthaltend mindestens eine Einheit gemäß Formel (1) sowie mindestens ein Lösungsmittel, bevorzugt ein organisches Lösungsmittel. Wie solche Lösungen hergestellt werden können, ist dem Fachmann bekannt und beispielsweise in WO 2002/072714, WO 2003/019694 und der darin zitierten Literatur beschrieben.

Die erfindungsgemäßen Verbindungen eignen sich für den Einsatz in elektronischen Vorrichtungen, insbesondere in organischen Elektrolumineszenzvorrichtungen (z.B. OLEDs oder OLECs ). Abhängig von der Substitution werden die Verbindungen in unterschiedlichen Funktionen und Schichten eingesetzt.

Ein weiterer Gegenstand der vorliegenden Erfindung ist daher die Verwendung der Verbindung gemäß Formel (1) in elektronischen Vorrichtungen sowie elektronische Vorrichtungen selbst, welche eine oder mehrere Verbindungen gemäß Formel (1) enthalten. Dabei sind die elektronischen Vorrichtungen bevorzugt ausgewählt aus der Gruppe bestehend aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und besonders bevorzugt organischen Elektrolumineszenzvorrichtungen (OLEDs und OLECs).

Gegenstand der Erfindung sind, wie bereits oben ausgeführt, elektronische Vorrichtungen, enthaltend mindestens eine Verbindung gemäß Formel (1). Dabei sind die elektronischen Vorrichtungen bevorzugt ausgewählt aus den oben genannten Vorrichtungen. Besonders bevorzugt sind organische Elektrolumineszenzvorrichtungen (OLEDs), enthaltend Anode, Kathode und mindestens eine emittierende Schicht, dadurch gekennzeichnet, dass mindestens eine organische Schicht, die eine emittierende Schicht, eine Lochtransportschicht oder eine andere Schicht sein kann, mindestens eine Verbindung gemäß Formel (1) enthält.

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Excitonenblockierschichten, Zwischenschichten (Interlayers), Ladungserzeugungsschichten (Charge-Generation Layers) (IDMC 2003, Taiwan; Session 21 OLED (5), T. Matsumoto, T. Nakada, J. Endo, K. Mori, N. Kawamura, A. Yokoi, J. Kido, Multiphoton Organic EL Device Having Charge Generation Layer) und/oder organischen oder anorganischen p/n-Übergängen. Es sei aber darauf hingewiesen, dass nicht notwendigerweise jede dieser Schichten vorhanden sein muss und die Wahl der Schichten immer von den verwendeten Verbindungen abhängt und insbesondere auch von der Tatsache, ob es sich um eine fluoreszierende oder phosphoreszierende Elektrolumineszenzvorrichtung handelt.

Die erfindungsgemäße organische Elektrolumineszenzvorrichtung kann mehrere emittierende Schichten enthalten. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues oder gelbes oder orangefarbenes oder rotes Licht emittieren. Insbesondere bevorzugt sind Dreischichtsysteme, also Systeme mit drei emittierenden Schichten, wobei die drei Schichten blaue, grüne und orange oder rote Emission zeigen (für den prinzipiellen Aufbau siehe z. B. WO 2005/011013). Die erfindungsgemäßen Verbindungen können in solchen Vorrichtungen in einer Lochtransportschicht, einer emittierenden Schicht und/oder in einer anderen Schicht vorhanden sein. Es soll angemerkt werden, dass sich für die Erzeugung von weißem Licht anstelle mehrerer farbig emittierender Emitterverbindungen auch eine einzeln verwendete Emitterverbindung eignen kann, welche in einem breiten Wellenlängenbereich emittiert.

Es ist erfindungsgemäß bevorzugt, wenn die Verbindung gemäß Formel (1) in einer elektrolumineszierneden Vorrichtung enthaltend einen oder mehrere phosphoreszierende Dotanden eingesetzt wird. Dabei kann die Verbindung in unterschiedlichen Schichten, bevorzugt in einer Lochtransportschicht, einer Lochinjektionsschicht oder in einer emittierenden Schicht, verwendet werden. Die Verbindung gemäß Formel (1) kann aber auch erfindungsgemäß in einer elektronischen Vorrichtung enthaltend einen oder mehrere fluoreszierende Dotanden eingesetzt werden.

Vom Begriff phosphoreszierende Dotanden sind typischerweise Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Dotanden (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Erfindung alle lumineszierenden Iridium-, Platin- oder Kupferkomplexe als phosphoreszierende Verbindungen angesehen.

Beispiele der oben beschriebenen Emitter können den Anmeldungen WO 00/70655, WO 01/41512, WO 02/02714, WO 02/15645, EP 1191613, EP 1191612, EP 1191614, WO 2005/033244, WO 2005/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind. Auch kann der Fachmann ohne erfinderisches Zutun weitere phosphoreszierende Komplexe in Kombination mit den Verbindungen gemäß Formel (1) in organischen Elektrolumineszenzvorrichtungen einsetzen.

Explizite Beispiele für geeignete phosphoreszierende Emitterverbindungen können weiterhin der folgenden Tabelle entnommen werden.

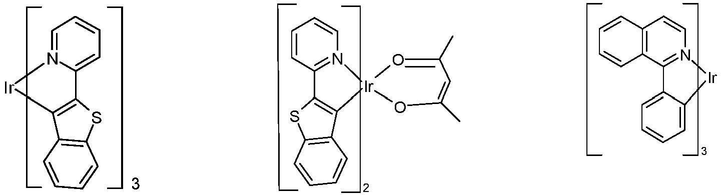

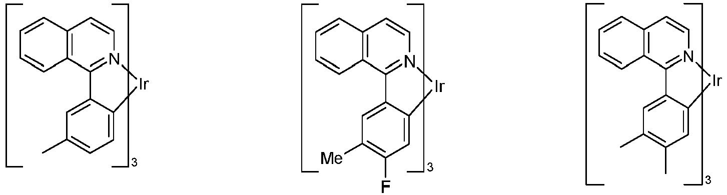

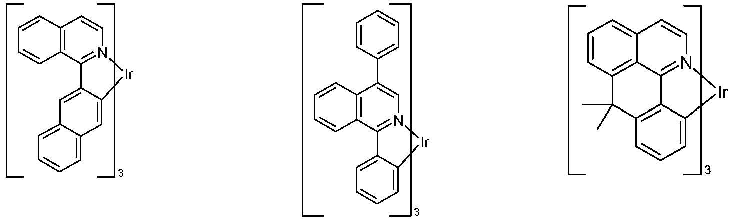

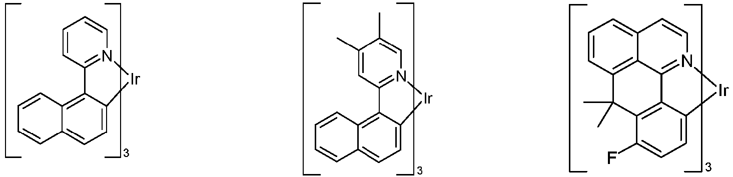

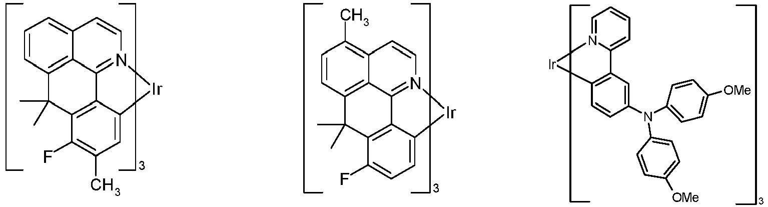

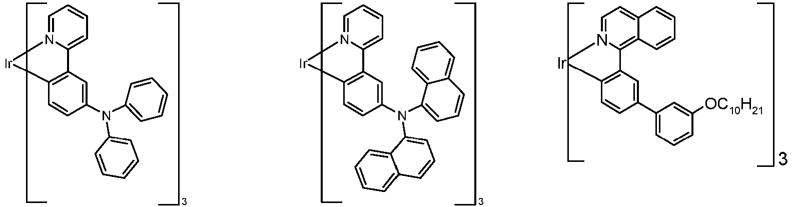

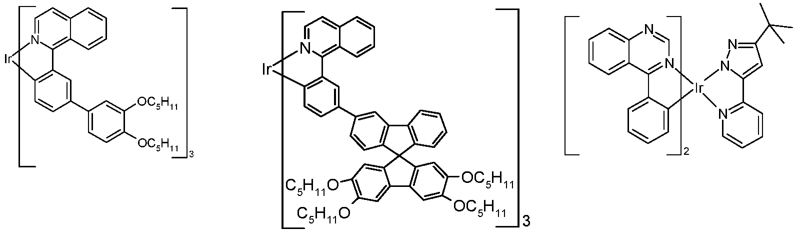

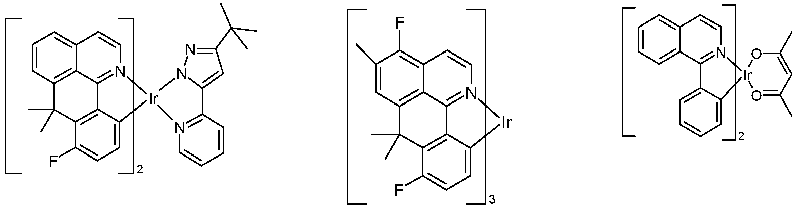

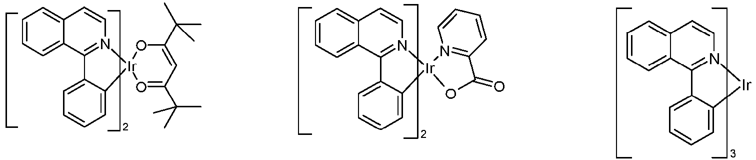

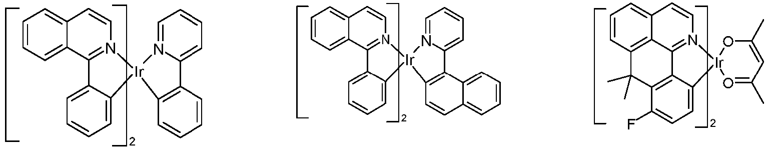

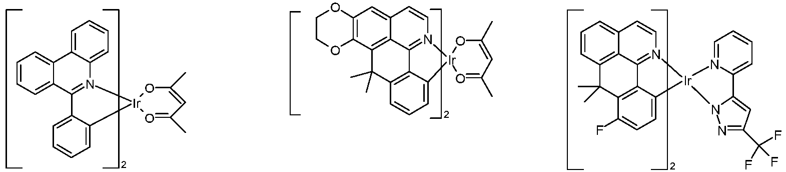

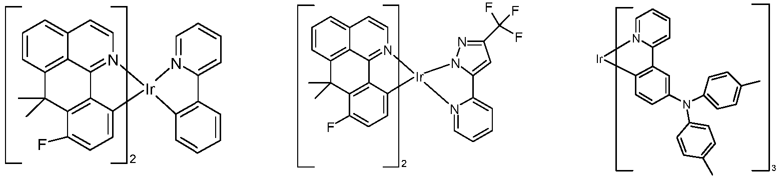

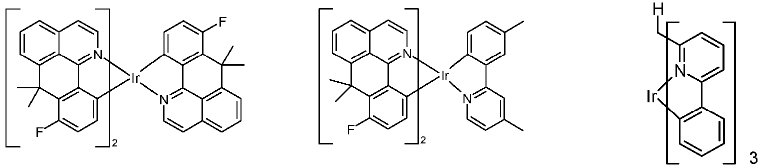

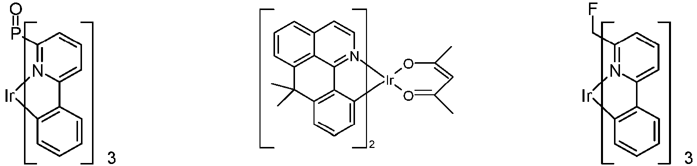

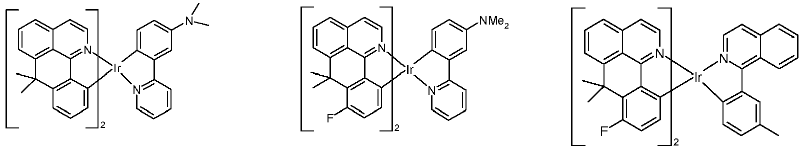

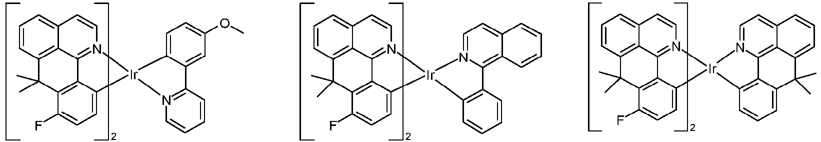

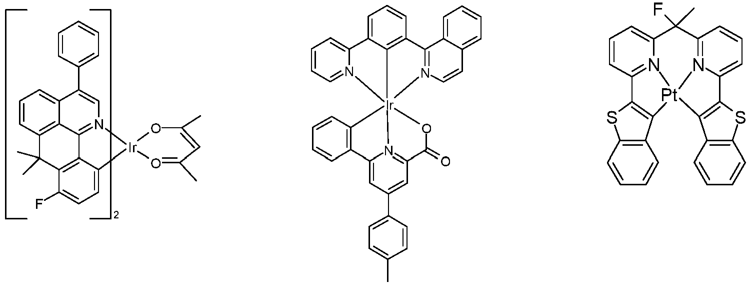

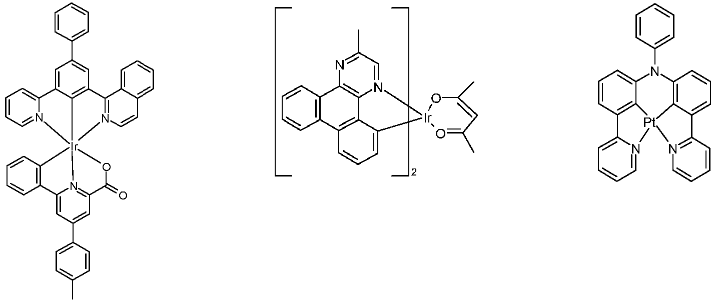

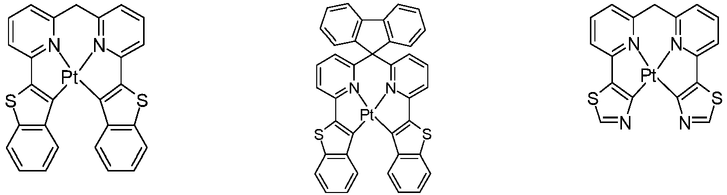

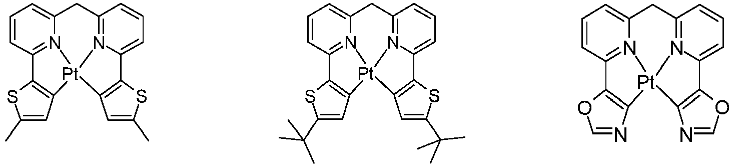

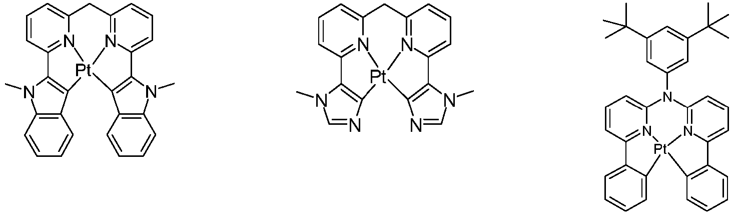

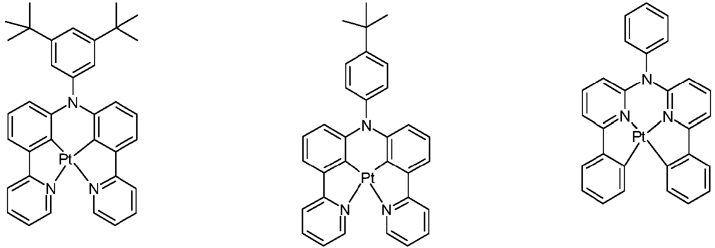

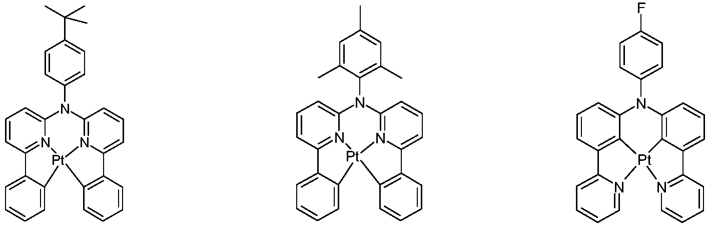

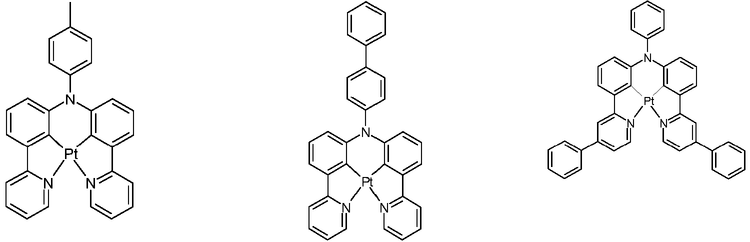

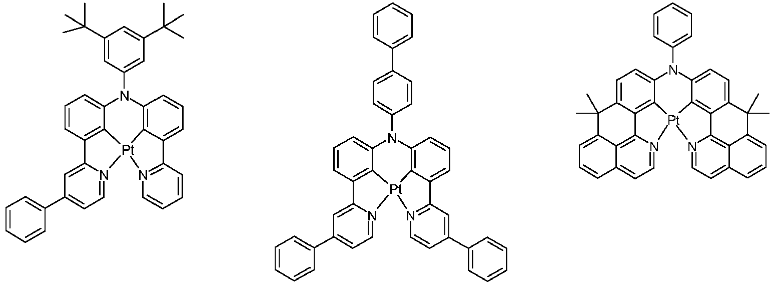

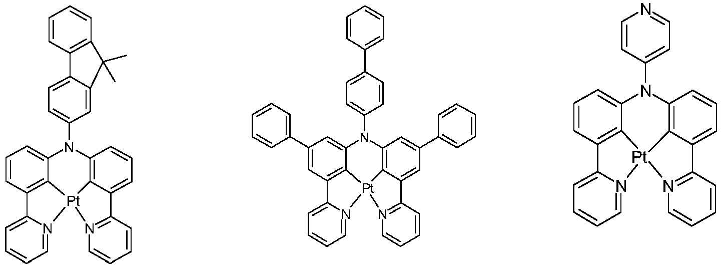

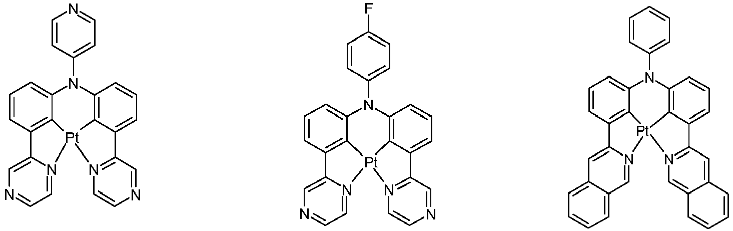

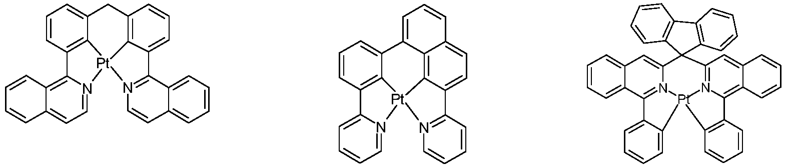

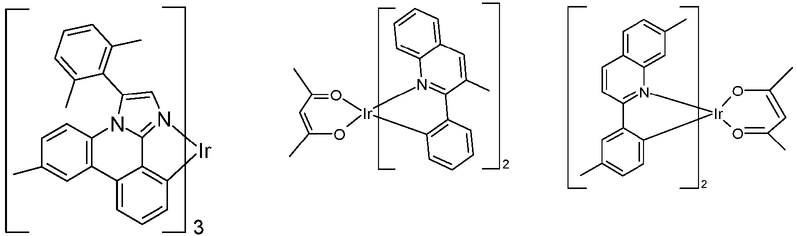

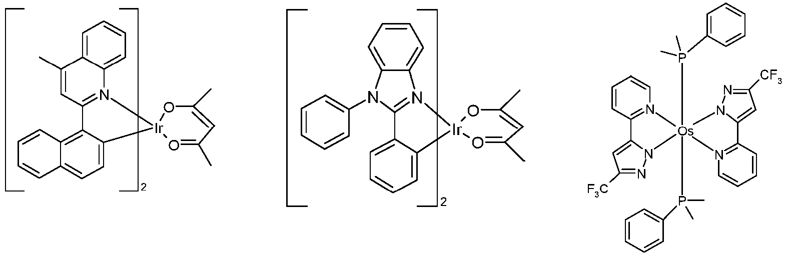

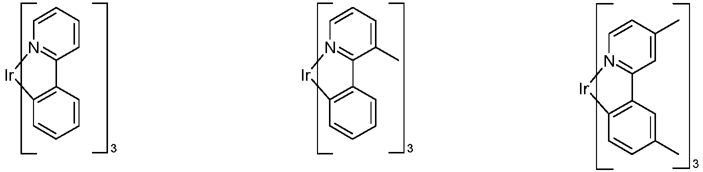

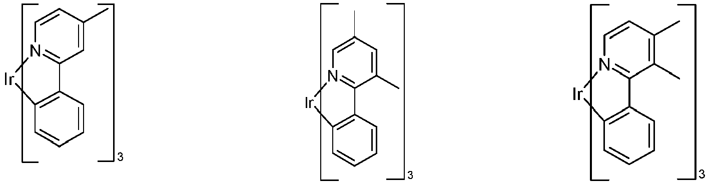

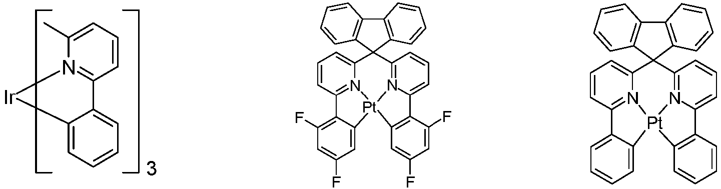

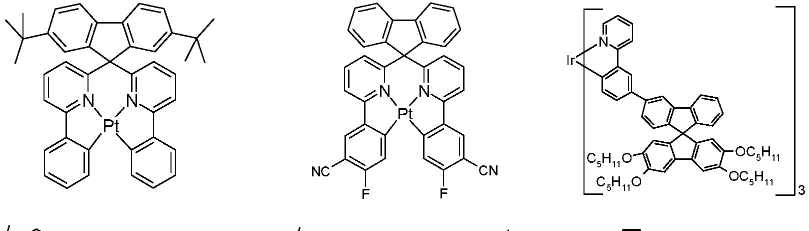

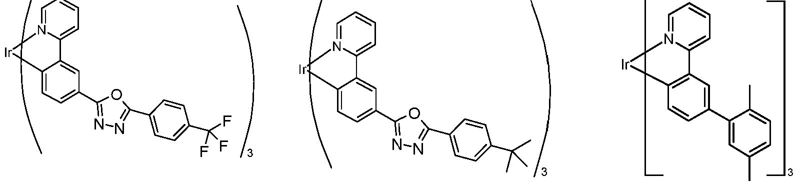

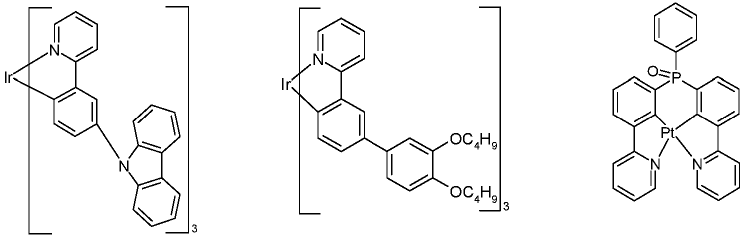

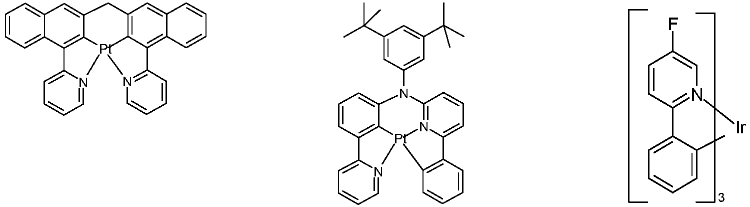

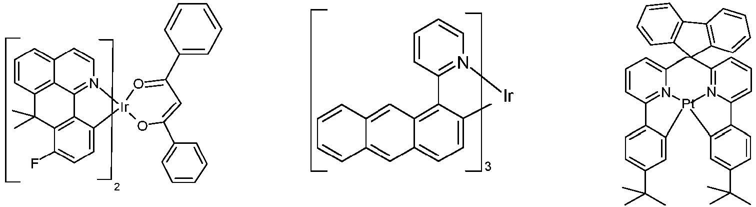

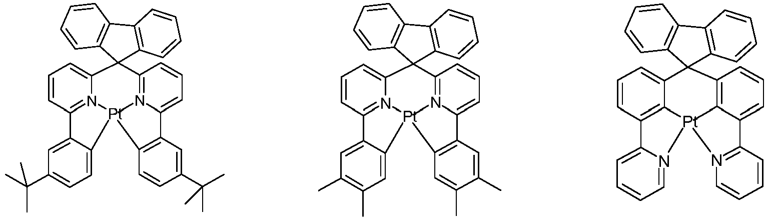

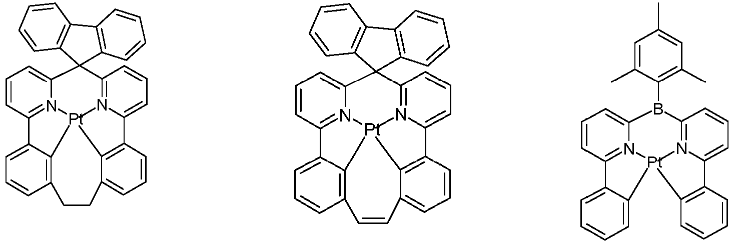

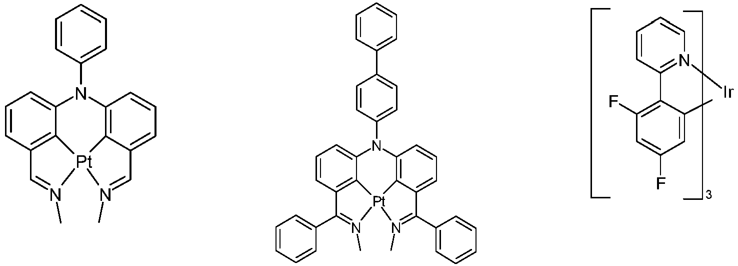

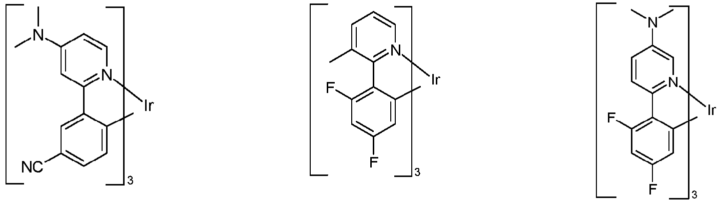

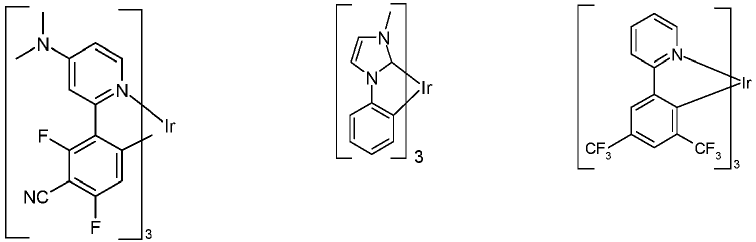

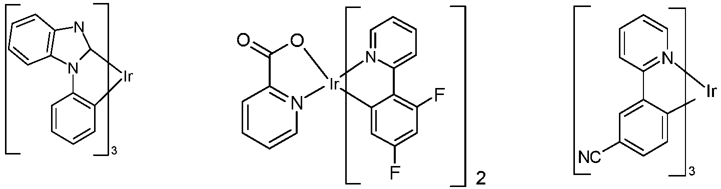

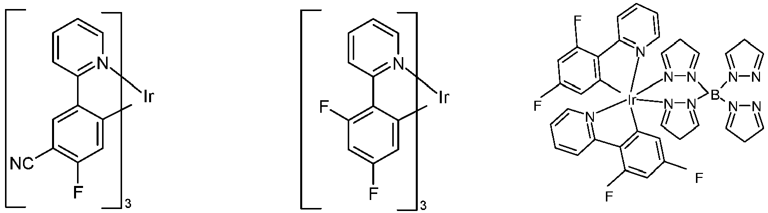

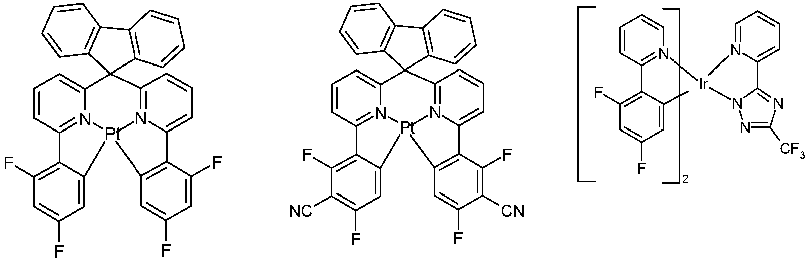

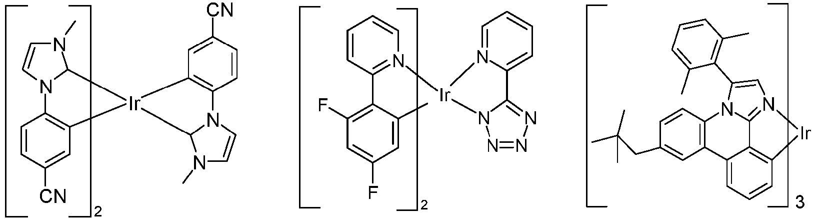

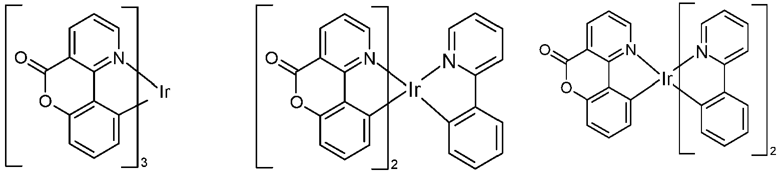

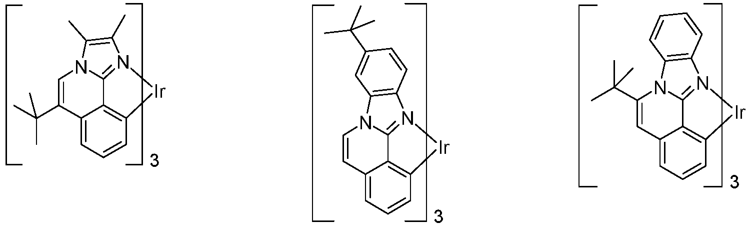

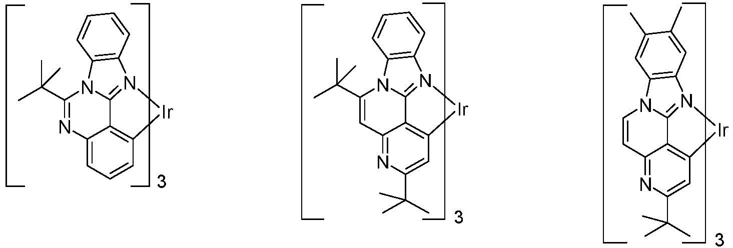

In einer bevorzugten Ausführungsform der Erfindung werden die Verbindungen gemäß der allgemeinen Formel (1) als Lochtransportmaterial eingesetzt. Die Verbindungen werden dann bevorzugt in einer Lochtransportschicht und/oder in einer Lochinjektionsschicht eingesetzt. Eine Lochinjektionsschicht im Sinne dieser Erfindung ist eine Schicht, die direkt an die Anode angrenzt. Eine Lochtransportschicht im Sinne dieser Erfindung ist eine Schicht, die zwischen der Lochinjektionsschicht und der Emissionsschicht liegt. Die Lochtransportschicht kann direkt an die Emissionschicht angrenzen. Wenn die Verbindungen gemäß Formel (1) als Lochtransportmaterial oder als Lochinjektionsmaterial verwendet werden, kann es bevorzugt sein, wenn sie mit Elektronenakzeptor-Verbindungen dotiert sind (p-Doping), beispielsweise mit F₄-TCNQ, F₆-TNAP oder mit Verbindungen, wie sie in EP 1476881 oder EP 1596445 beschrieben werden. In einer weiteren bevorzugten Ausführungsform der Erfindung wird eine Verbindung gemäß Formel (I) als Lochtransportmaterial in Kombination mit einem Hexaazatriphenylenderivat, wie in US 2007/0092755 beschrieben, verwendet. Besonders bevorzugt wird das Hexaazatriphenylenderivat dabei in einer separaten Schicht eingesetzt.

Werden die Verbindungen gemäß der allgemeinen Formel (1) als Lochtransportmaterial in einer Lochtransportschicht eingesetzt, so kann die Verbindung als Reinmaterial, d.h. in einem Anteil von 100 %, in der Lochtransportschicht eingesetzt werden, oder sie kann in Kombination mit einer oder mehreren weiteren Verbindungen in der Lochtransportschicht eingesetzt werden.

In einer weiteren Ausführungsform der vorliegenden Erfindung werden die Verbindungen der allgemeinen Formel (1) als emittierende Materialien eingesetzt. Die Verbindungen werden hierzu bevorzugt in einer Emissionsschicht eingesetzt. Die Emissionsschicht enthält neben wenigstens einer der Verbindungen nach der allgemeinen Formel (1) weiterhin wenigstens ein Host Material. Dabei kann der Fachmann aus den bekannten Hostmaterialien ohne Schwierigkeiten und ohne erfinderisch zu sein auswählen.

In einer weiteren Ausführungsform der vorliegenden Erfindung werden die Verbindungen der allgemeinen Formel (1) als Matrixmaterial in Kombination mit einem oder mehreren Dotanden, vorzugsweise phosphoreszierenden Dotanden, eingesetzt.

Unter einem Dotanden wird in einem System enthaltend ein Matrixmaterial und einen Dotanden diejenige Komponente verstanden, deren Anteil in der Mischung der kleinere ist. Entsprechend wird unter einem Matrixmaterial in einem System enthaltend ein Matrixmaterial und einen Dotanden diejenige Komponente verstanden, deren Anteil in der Mischung der größere ist.

Der Anteil des Matrixmaterials in der emittierenden Schicht beträgt in diesem Fall zwischen 50.0 und 99.9 Vol.-%, bevorzugt zwischen 80.0 und 99.5 Vol.-% und besonders bevorzugt für fluoreszierende emittierende Schichten zwischen 92.0 und 99.5 Vol.-% sowie für phosphoreszierende emittierende Schichten zwischen 85.0 und 97.0 Vol.-%.

Entsprechend beträgt der Anteil des Dotanden zwischen 0.1 und 50.0 Vol.- %, bevorzugt zwischen 0.5 und 20.0 Vol.-% und besonders bevorzugt für fluoreszierende emittierende Schichten zwischen 0.5 und 8.0 Vol.-% sowie für phosphoreszierende emittierende Schichten zwischen 3.0 und 15.0 Vol.-%.

Eine emittierende Schicht einer organischen Elektrolumineszenzvorrichtung kann auch Systeme umfassend mehrere Matrixmaterialien (Mixed-Matrix-Systeme) und/oder mehrere Dotanden enthalten. Auch in diesem Fall sind die Dotanden im Allgemeinen diejenigen Materialien, deren Anteil im System der kleinere ist und die Matrixmaterialien sind diejenigen Materialien, deren Anteil im System der größere ist. In Einzelfällen kann jedoch der Anteil eines einzelnen Matrixmaterials im System kleiner sein als der Anteil eines einzelnen Dotanden.

In einer weiteren bevorzugten Ausführungsform der Erfindung werden die Verbindungen gemäß der allgemeinen Formel (1) als eine Komponente von Mixed-Matrix-Systemen verwendet. Die Mixed-Matrix-Systeme umfassen bevorzugt zwei oder drei verschiedene Matrixmaterialien, besonders bevorzugt zwei verschiedene Matrixmaterialien. Bevorzugt stellt dabei eines der beiden Materialien ein Material mit lochtransportierenden Eigenschaften und das andere Material ein Material mit elektronentransportierenden Eigenschaften dar. Die gewünschten elektronentransportierenden und lochtransportierenden Eigenschaften der Mixed-Matrix-Komponenten können jedoch auch hauptsächlich oder vollständig in einer einzigen Mixed-Matrix-Komponente vereinigt sein, wobei die weitere bzw. die weiteren Mixed-Matrix-Komponenten andere Funktionen erfüllen. Die beiden unterschiedlichen Matrixmaterialien können dabei in einem Verhältnis von 1:50 bis 1:1, bevorzugt 1:20 bis 1:1, besonders bevorzugt 1:10 bis 1:1 und ganz besonders bevorzugt 1:4 bis 1:1 vorliegen. Bevorzugt werden Mixed-Matrix-Systeme in phosphoreszierenden organischen Elektrolumineszenzvorrichtungen eingesetzt. Genauere Angaben zu Mixed-Matrix-Systemen sind unter anderem in der Anmeldung WO 2010/108579 enthalten.

Die Mixed-Matrix-Systeme können einen oder mehrere Dotanden umfassen, bevorzugt einen oder mehrere phosphoreszierende Dotanden. Allgemein werden Mixed-Matrix-Systeme bevorzugt in phosphoreszierenden organischen Elektrolumineszenzvorrichtungen eingesetzt.

Besonders geeignete Matrixmaterialien, welche in Kombination mit den erfindungsgemäßen Verbindungen als Matrixkomponenten eines Mixed-Matrix-Systems verwendet werden können, sind ausgewählt aus den unten angegebenen bevorzugten Matrixmaterialien für phosphoreszierende Dotanden oder den bevorzugten Matrixmaterialien für fluoreszierende Dotanden, je nachdem welche Art von Dotand im mixed-Matrix-System eingesetzt wird.

Bevorzugte phosphoreszierende Dotanden zur Verwendung in Mixed-Matrix-Systemen sind die in der obenstehenden Tabelle aufgeführten phosphoreszierenden Dotanden.

Im Folgenden werden die in den erfindungsgemäßen Vorrichtungen in den betreffenden Funktionen bevorzugt eingesetzten Materialien aufgeführt.

Bevorzugte fluoreszierende Dotanden sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind.

Als Matrixmaterialien, bevorzugt für fluoreszierende Dotanden, kommen neben den erfindungsgemäßen Verbindungen Materialien verschiedener Stoffklassen in Frage. Bevorzugte Matrixmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

Bevorzugte Matrixmaterialien für phosphoreszierende Dotanden sind neben den erfindungsgemäßen Verbindungen aromatische Amine, insbesondere Triarylamine, z. B. gemäß US 2005/0069729, Carbazolderivate (z. B. CBP, N,N-Biscarbazolylbiphenyl) oder Verbindungen gemäß WO 2005/039246, US 2005/0069729, JP 2004/288381, EP 1205527 oder WO 2008/086851, verbrückte Carbazolderivate, z. B. gemäß WO 2011/088877 und WO 2011/128017, Indenocarbazolderivate, z. B. gemäß WO 2010/136109 und WO 2011/000455, Azacarbazolderivate, z. B. gemäß EP 1617710, EP 1617711, EP 1731584, JP 2005/347160, Indolocarbazolderivate, z. B. gemäß WO 2007/063754 oder WO 2008/056746, Ketone, z. B. gemäß WO 2004/093207 oder WO 2010/006680, Phosphinoxide, Sulfoxide und Sulfone, z. B. gemäß WO 2005/003253, Oligophenylene, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Aluminiumkomplexe, z. B. BAlq, Diazasilol- und Tetraazasilol-Derivate, z. B. gemäß WO 2010/054729, Diazaphosphol-Derivate, z. B. gemäß WO 2010/054730 und Aluminiumkomplexe, z. B. BAIQ.

Geeignete Ladungstransportmaterialien, wie sie in der Lochinjektions- bzw. Lochtransportschicht oder in der Elektronentransportschicht der erfindungsgemäßen organischen Elektrolumineszenzvorrichtung verwendet werden können, sind neben den erfindungsgemäßen Verbindungen beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Als Kathode der organischen Elektrolumineszenzvorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/NiOₓ, Al/PtOₓ) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere.

Die Vorrichtung wird entsprechend (je nach Anwendung) strukturiert, kontaktiert und schließlich versiegelt, da sich die Lebensdauer der erfindungsgemäßen Vorrichtungen bei Anwesenheit von Wasser und/oder Luft verkürzt.

In einer bevorzugten Ausführungsform ist die erfindungsgemäße organische Elektrolumineszenzvorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Dabei ist es jedoch auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (1) nötig. Hohe Löslichkeit lässt sich durch geeignete Substitution der Verbindungen erreichen.

Weiterhin bevorzugt ist es, dass zur Herstellung einer erfindungsgemäßen organischen Elektrolumineszenzvorrichtung eine oder mehrere Schichten aus Lösung und eine oder mehrere Schichten durch ein Sublimationsverfahren aufgetragen werden.

Erfindungsgemäß können die elektronischen Vorrichtungen enthaltend eine oder mehrere Verbindungen gemäß der allgemeinen Formel (1) in Displays, als Lichtquellen in Beleuchtungsanwendungen sowie als Lichtquellen in medizinischen und/oder kosmetischen Anwendungen (z.B. Lichttherapie) eingesetzt werden.

Vorrichtungen enthaltend die Verbindungen der allgemeinen Formel (1) können sehr vielseitig eingesetzt werden. So können, beispielsweise, elektrolumineszierende Vorrichtuungen enthaltend eine oder mehrere Verbindungen nach der allgemeinen Formel (1) in Displays für Fernseher, Mobiltelefone, Computer und Kameras eingesetzt werden. Die Vorrichtungen können aber auch in Beleuchtungsanwendungen verwendet werden. Weiterhin können elektrolumineszierende Vorrichtungen, z. B. in OLEDs oder OLECs, enthaltend wenigstens eine der Verbindung nach der allgemeinen Formel (1) in der Medizin oder Kosmetik zu Phototherapie genutzt werden. Somit kann eine Vielzahl von Erkrankungen (Psoriasis, atopische Dermatitis, Inflammation, Akne, Hautkrebs etc.) oder die Vermeidung sowie Reduktion von Hautfaltenbildung, Hautrötung und Hautalterung behandelt werden. Weiterhin können die lichtemittierenden Vorrichtungen dazu genutzt werden, um Getränke, Speisen oder Lebensmittel frisch zu halten oder um Geräte (bspw. medizinische Geräte) zu sterilisieren.

Die erfindungsgemäßen Verbindungen bzw. die erfindungsgemäßen organischen Elektrolumineszenzvorrichtungen zeichnen sich durch folgende überraschende Vorteile gegenüber dem Stand der Technik aus:
1. Die erfindungsgemäßen Verbindungen eignen sich sehr gut für den Einsatz in einer Lochtransportschicht oder einer Lochinjektionsschicht in elektronischen Vorrichtungen, wie beispielsweise in organischen Elektrolumineszenzvorrichtungen, insbesondere aufgrund ihrer hohen Lochbeweglichkeit.
2. Die erfindungsgemäßen Verbindungen weisen eine relativ niedrige Sublimationstemperatur, eine hohe Temperaturstabilität sowie eine hohe Oxidationsstabilität und eine hohe Glasübergangtemperatur auf, was sowohl für die Prozessierbarkeit, beispielweise aus Lösung oder aus der Gasphase, als auch für die Verbendung in elektronischen Vorrichtungen vorteilhaft ist.
3. Die Verwendung der erfindungsgemäßen Verbindungen in elektronischen Vorrichtungen, insbesondere eingesetzt als Lochtransport- oder Lochinjektionsmaterial führt zu hohen Effizienzen, geringen Betriebspannungen sowie zu langen Lebensdauern.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Es sei ferner darauf hingewiesen, dass viele der Merkmale, und insbesondere die der bevorzugten Ausführungsformen der vorliegenden Erfindung selbst erfinderisch und nicht lediglich als Teil der Ausführungsformen der vorliegenden Erfindung zu betrachten sind. Für diese Merkmale kann ein unabhängiger Schutz zusätzlich oder alternativ zu jeder gegenwärtig beanspruchten Erfindung begehrt werden.

Die mit der vorliegenden Erfindung offengelegte Lehre zum technischen Handeln kann abstrahiert und mit anderen Beispielen kombiniert werden.

Die Erfindung wird durch die nachfolgenden Beispiele näher erläutert, ohne sie dadurch einschränken zu wollen.

### Beispiele

### Materialien

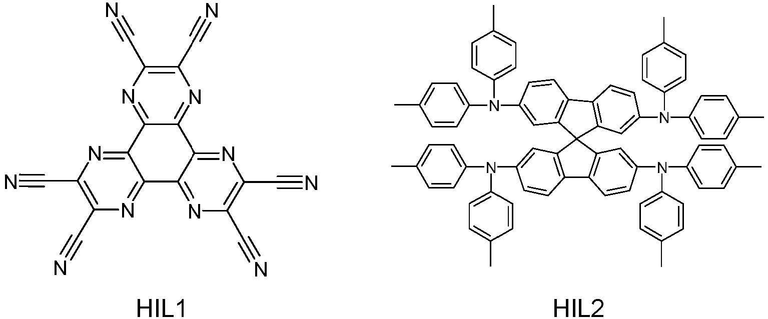

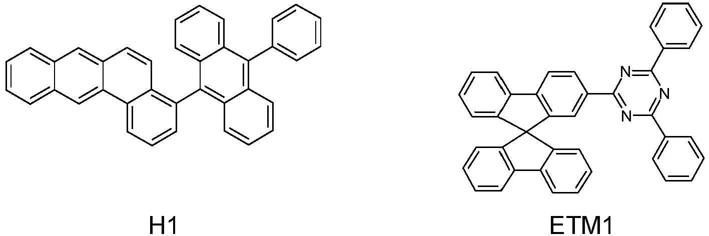

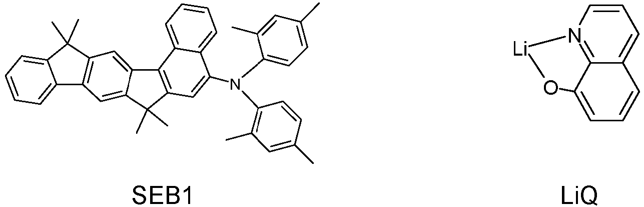

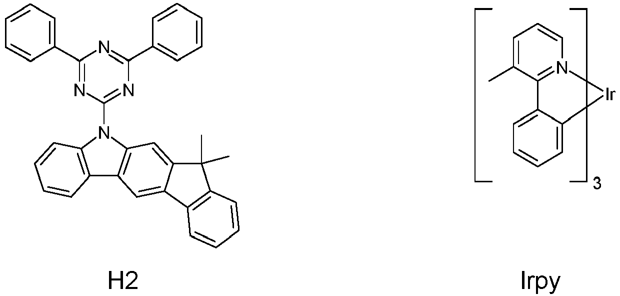

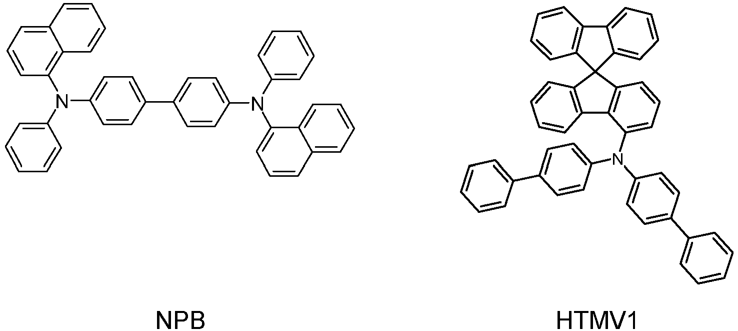

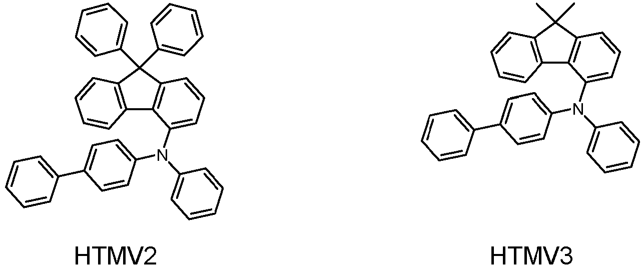

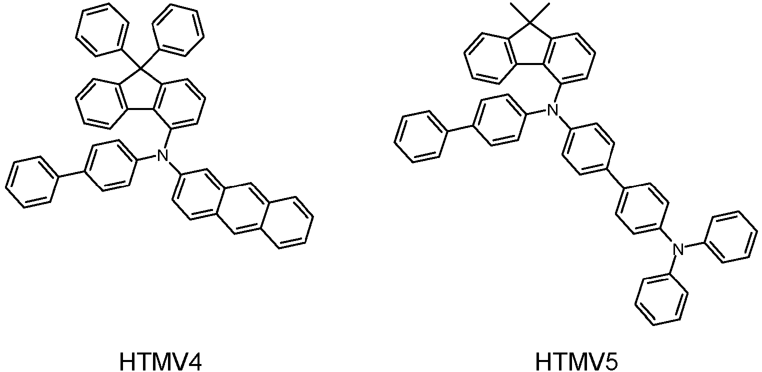

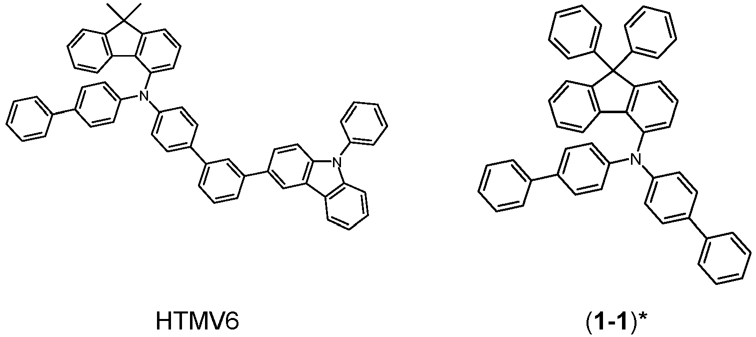

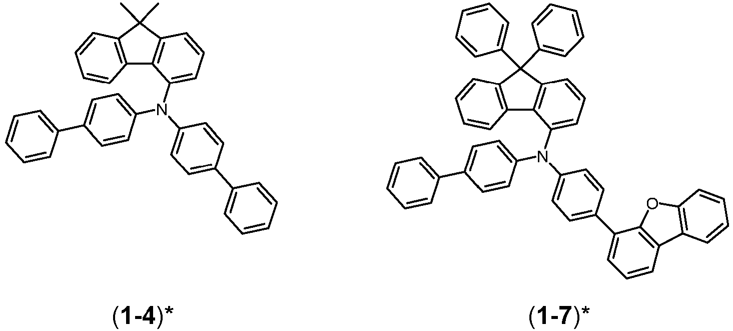

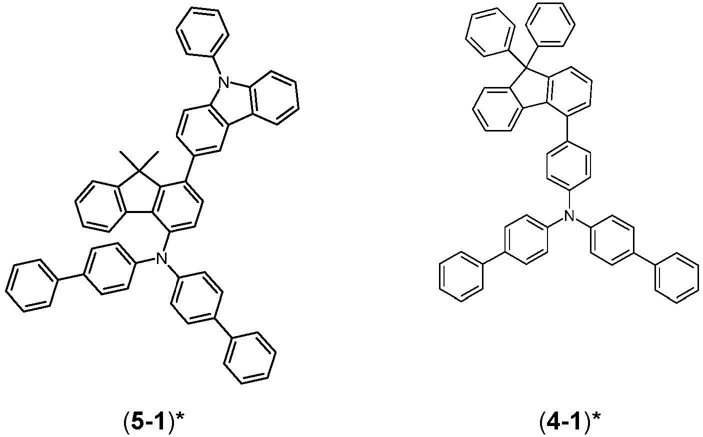

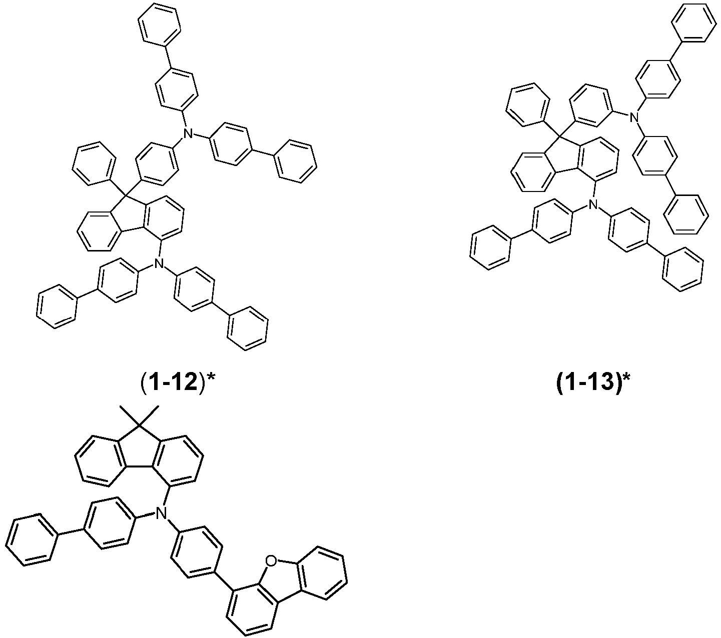

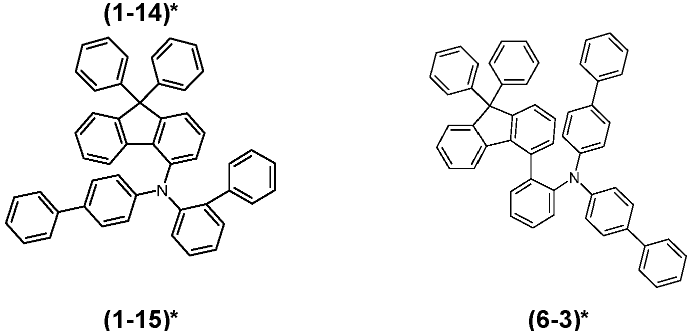

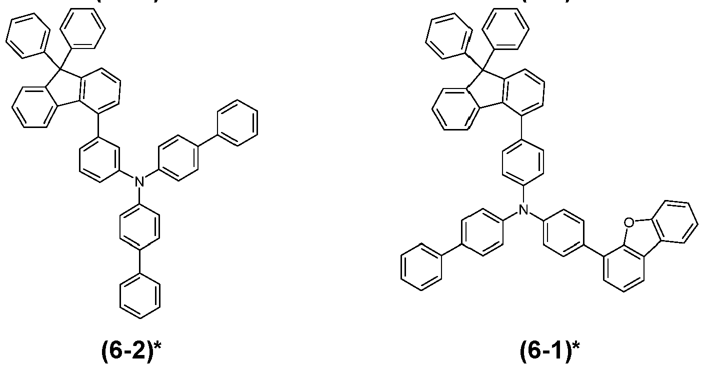

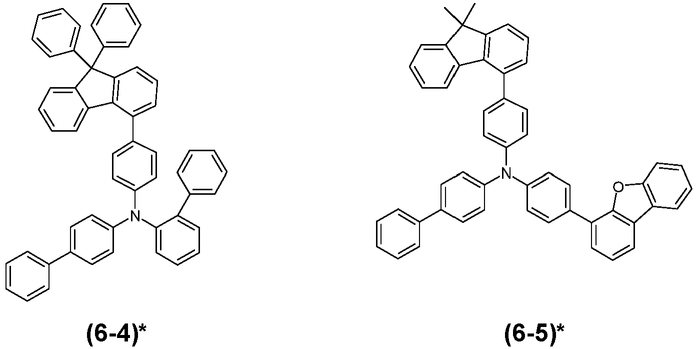

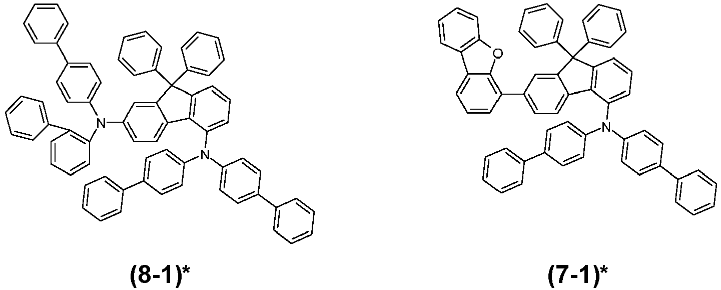

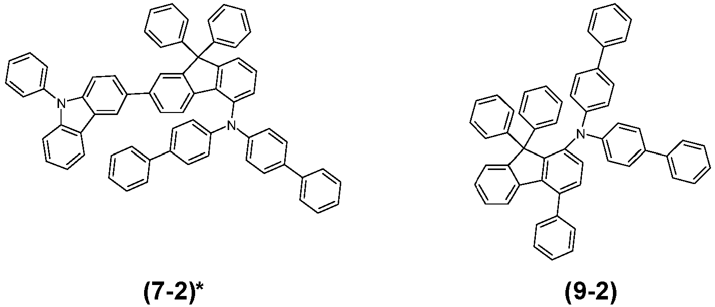

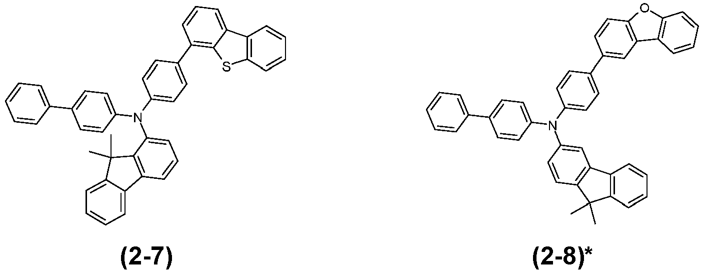

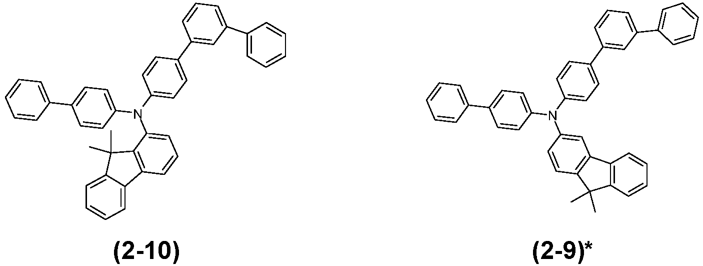

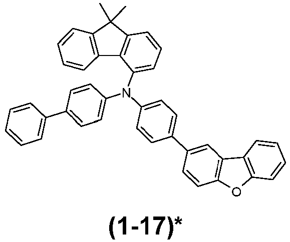

* nicht erfindungsgemäß

Die Materialien HIL1, HIL2 (EP 0676461), H1 (WO 2008/145239), H2 (WO 2010/136109), ETM1 (WO 2005/053055), SEB1 (WO 2008/006449), LiQ, Irpy und NPB sind dem Fachmann aus dem Stand der Technik gut bekannt. Die Verbindungen HTMV1 bis HTMV6 sind Vergleichsverbindungen, die analog zu dem in Beispiel 1 beschriebenen Verfahren hergestellt werden können. Die Verbindungen **(9-2), (2-7)** und **(2-10)** sind erfindungsgemäß.

### Beispiel 1

### Synthese der Verbindung Bis-biphenyl-4-yl-(9,9-diphenyl-9H-fluoren-4-yl)-amin (1-1) sowie der Verbindungen (1-2) bis (1-12)

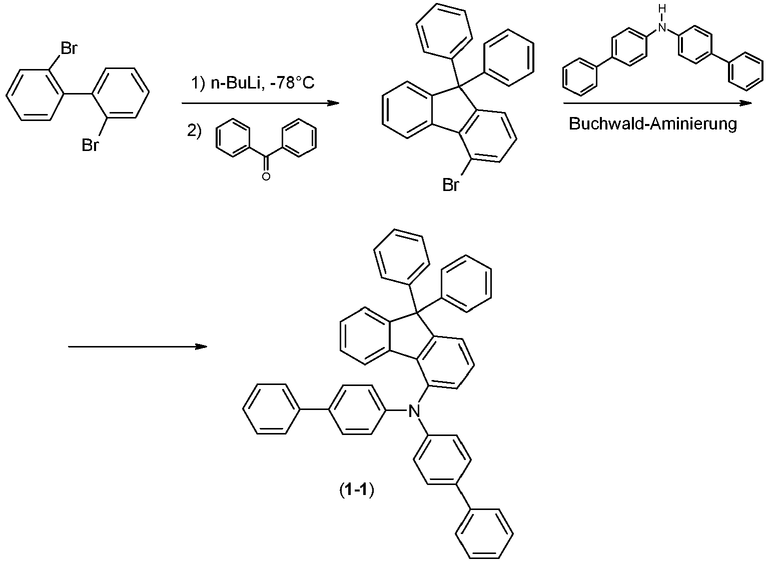

### 4-Bromo-9,9-diphenyl-9H-fluoren

37 g (152 mmol) 2,2'-Dibromo-biphenyl werden in einem ausgeheizten Kolben in 300 mL getrocknetem THF gelöst. Die Reaktionsmischung wird auf -78°C gekühlt. Bei dieser Temperatur werden 75 mL einer 15%-igen Lösung n-BuLi in Hexan (119 mmol) langsam zugetropft (Dauer: ca 1 Stunde). Der Ansatz wird 1 h bei -70°C nachgerührt. Anschließend werden 21,8 g Benzophenon (119 mmol) in 100 ml THF gelöst und bei -70°C zugetropft. Nach beendeter Zugabe wird die Reaktionsmischung langsam auf Raumtemperatur erwärmt, mit NH₄Cl gequencht und anschließend am Rotationsverdampfer eingeengt. Die einrotierte Lösung wird vorsichtig mit 510 ml Essigsäure versetzt und anschließen werden 100 ml rauchende HCl zugegeben. Der Ansatz wird auf 75°C erhitzt und 4 h auf dieser Temperatur gehalten. Dabei fällt ein weißer Feststoff aus. Der Ansatz wird dann auf Raumtemperatur abgekühlt, der ausgefallene Feststoff wird abgesaugt und mit Methanol nachgewaschen. Der Rückstand wird im Vakuum bei 40°C getrocknet. Ausbeute beträgt 33,2 g (83 mmol) (70% der Theorie)

Analog dazu werden die folgenden bromierten Verbindungen hergestellt:

| **Edukt 1** | **Edukt 2** | **Produkt** | **Ausbeute** |
|---|---|---|---|
| 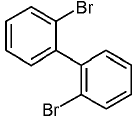 | 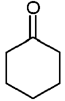 | 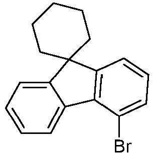 | 78% |
| 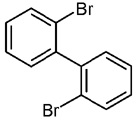 | 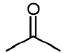 | 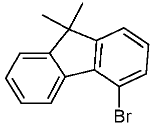 | 70% |
| 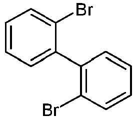 | 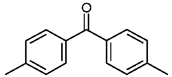 | 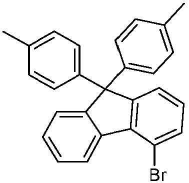 | 82% |
| 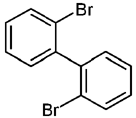 | 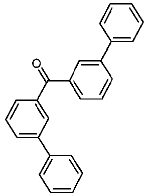 | 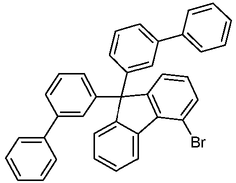 | 85% |
| 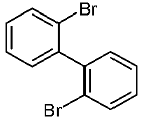 | 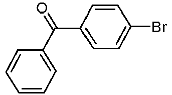 | 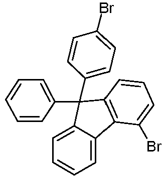 | 80% |
| 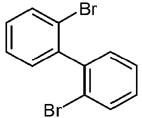 | 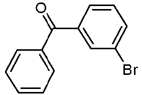 | 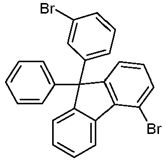 | 85% |
| 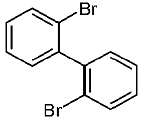 | 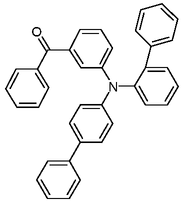 | 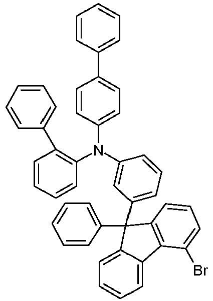 | 77% |

### Bis-biphenyl-4-yl-(9,9-diphenyl-9H-fluoren-4-yl)-amin (1-1)

17 g Bis-biphenyl-4-yl-amin (53 mmol) und 23,1 g 4-Bromo-9,9-diphenyl-9H-fluoren (58 mmol) werden in 500 mL Toluol gelöst: Die Lösung wird entgast und mit N₂ gesättigt. Danach wird sie mit 5,3 mL (5,3 mmol) einer 1 M Tri-tert-Butylphosphin-Lösung und 0,6 g (2,65 mmol) Palladium(II)-acetat versetzt. Anschließend werden 12,7 g Natrium-tert-butylat (132,23 mmol) zugegeben. Die Reaktionsmischung wird 3 h unter Schutzatmosphäre zum Sieden erhitzt. Das Gemisch wird im Anschluss zwischen Toluol und Wasser verteilt, die organische Phase dreimal mit Wasser gewaschen, über Na₂SO₄ getrocknet und einrotiert. Nach Filtration des Rohproduktes über Kieselgel mit Toluol wird der verbleibende Rückstand aus Heptan/Toluol umkristallisiert und abschließend im Hochvakuum sublimiert. Die Reinheit beträgt 99.9%. Die Ausbeute beträgt 29 g (87% der Theorie).

Analog hierzu werden die folgenden Verbindungen **(1-2)** bis **(1-17)** hergestellt.

| **Edukt 1** | **Edukt 2** | **Produkt** | **Ausbeute** |
|---|---|---|---|
| 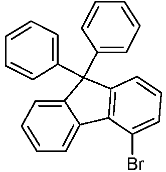 | 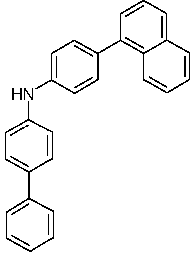 | 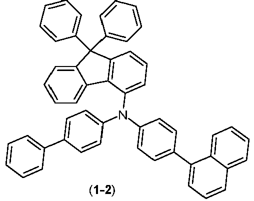 | 78 % |
| 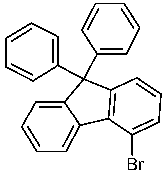 | 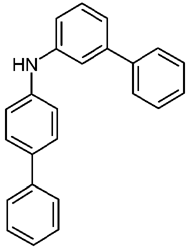 | 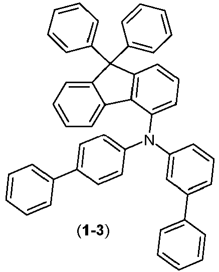 | 83% |
| 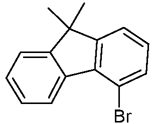 | 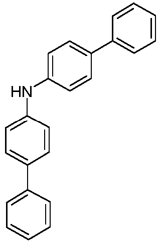 | 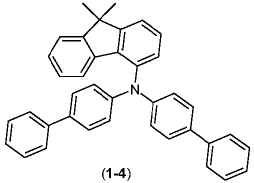 | 92% |
| 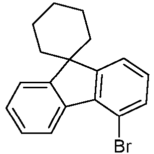 | 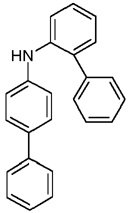 | 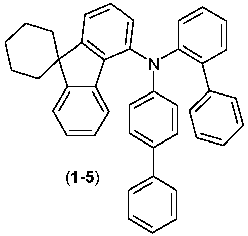 | 88% |
| 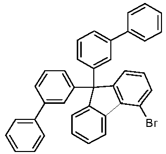 | 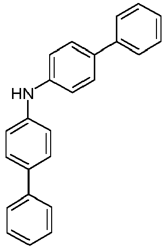 | 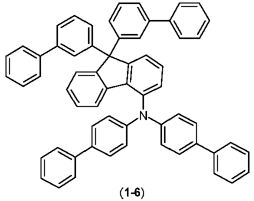 | 77% |
| 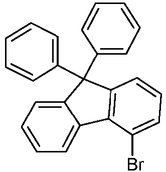 | 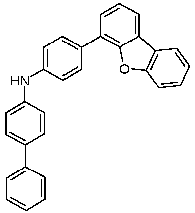 | 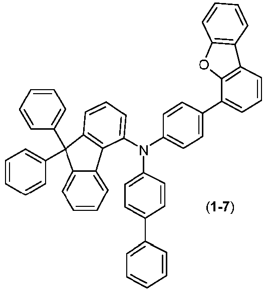 | 75% |
| 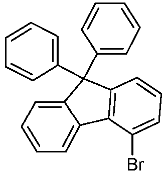 | 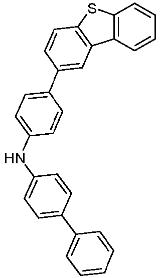 | 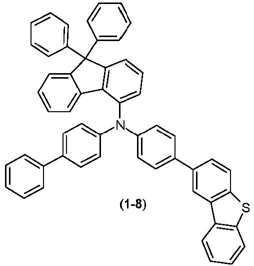 | 80% |
| 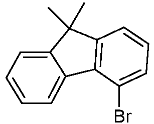 | 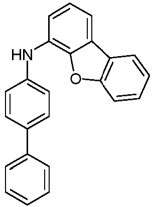 | 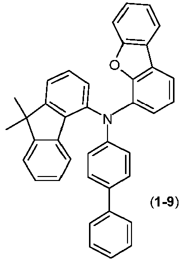 | 77% |
| 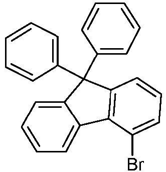 | 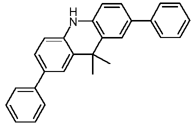 | 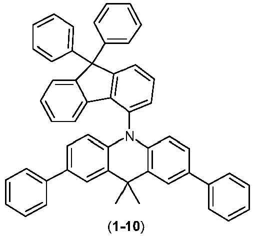 | 71% |
| 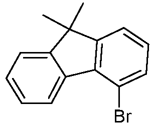 | 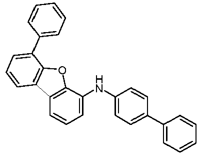 | 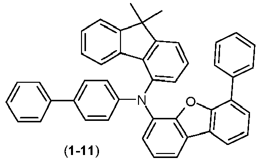 | 70% |
| 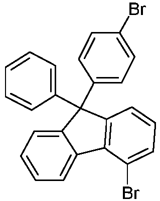 | 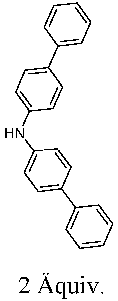 | 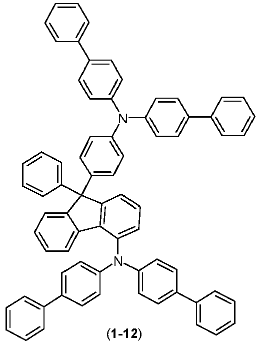 | 75% |
| 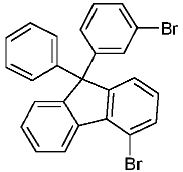 | 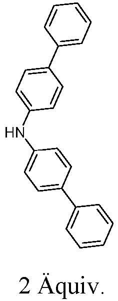 | 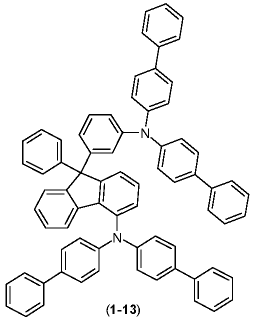 | 77% |
| 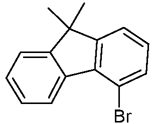 | 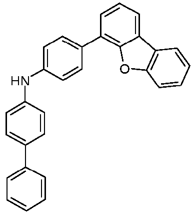 | 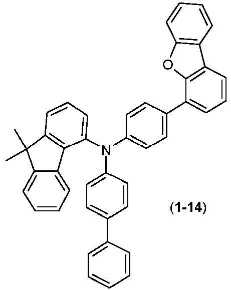 | 75% |
| 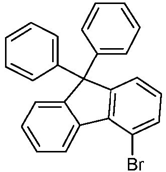 | 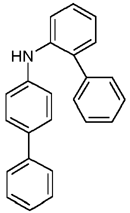 | 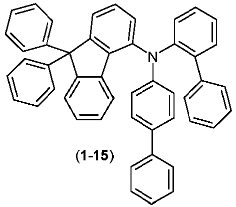 | 85% |
| 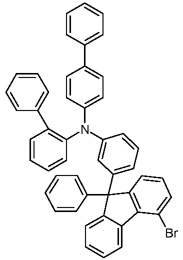 | 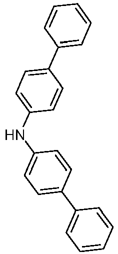 | 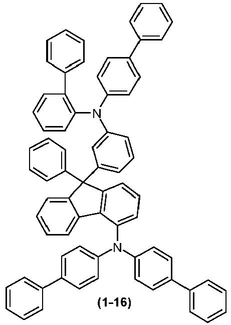 | 79% |
| 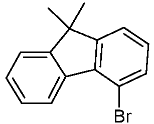 | 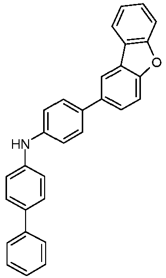 | 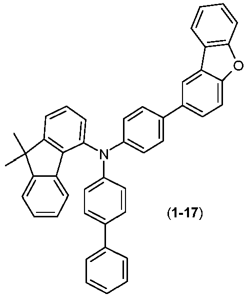 | 72% |

### Beispiel 2

### Synthese der Verbindung Biphenyl-3-yl-biphenyl-4-yl-(9,9-dimethyl-9H-fluoren-3-yl)-amin (2-1) sowie der Verbindungen (2-2) bis (2-10)

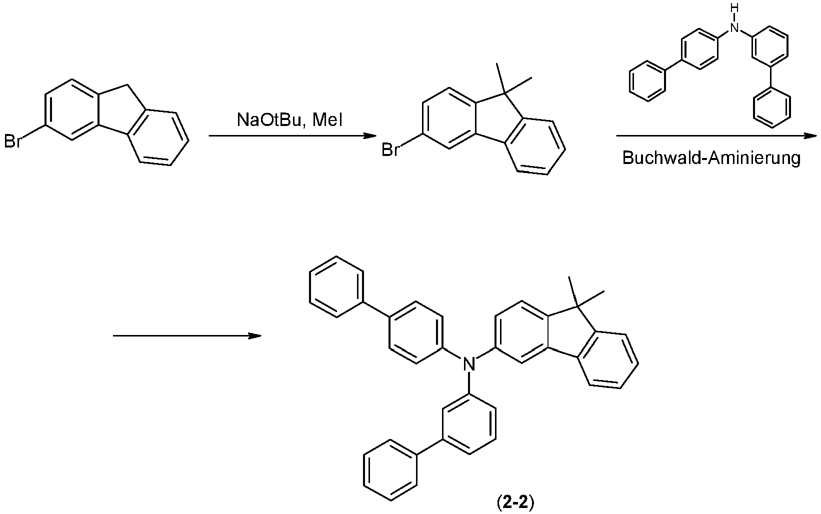

### 3-Bromo-9,9-dimethyl-9H-fluoren

29,5 g (120 mmol) 3-Bromo-9H-fluoren (Tetrahedron Letters, 51,37, 4894-4897; 2010) werden in einem ausgeheizten Kolben in 220 mL getrocknetem DMSO gelöst. Bei Raumtemperatur werden 34,7 g (361 mmol) NaO*^{t}*Bu zugegeben. Die Suspension wird auf eine Innentemperatur von 65 C gebracht. Bei dieser Temperatur wird eine Lösung von 22,5 mL (361 mmol) lodmethan in DMSO (50 mL) zugetropft, dass die Innentemperatur 65 °C nicht übersteigt (Dauer: ca. 30 min). Der Ansatz wird für weitere 30 min. bei 65°C Innentemperatur gehalten, und anschließend auf 400 mL einer eiskalten wässrigen NH₄OH-Lösung (1/1, v/v) gegossen und ca. 20 min. gerührt. Der ausgefallene Feststoff wird abgesaugt und nach einander mit ca. 200 mL H₂O und Methanol gewaschen. Ausbeute: 31 g (114 mmol) (95% der Theorie).

Analog hierzu werden die folgenden bromierten Verbindungen hergestellt:

| **Edukt 1** | **Edukt 2** | **Produkt** | **Ausbeute** |
|---|---|---|---|
| 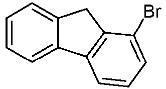 | H₃C-I | 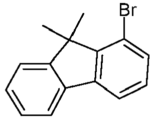 | 78 % |
| 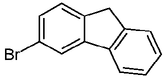 | 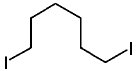 | 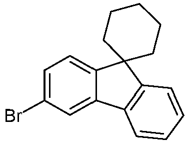 | 85% |

**Biphenyl-3-yl-biphenyl-4-yl-(9,9-dimethyl-9H-fluoren-3-yl)-amin (2-1)** 30 g Biphenyl-3-yl-biphenyl-4-yl-amin (93,4 mmol) und 25,5 g 3-Bromo-9,9-dimethyl-9H-fluoren (93,4 mol) werden in 600 mL Toluol gelöst: Die Lösung wird entgast und mit N₂ gesättigt. Danach wird sie mit 3,2 g (3,73 mmol) Tri-tert-Butylphosphin und 0,42 g (1,87 mmol) Palladium(II)acetat versetzt. Anschließend werden 13,9 g Natrium-tert-butylat (140 mmol) zugegeben. Die Reaktionsmischung wird 5 h unter Schutzatmosphäre zum Sieden erhitzt. Das Gemisch wird im Anschluss zwischen Toluol und Wasser verteilt, die organische Phase dreimal mit Wasser gewaschen, über Na₂SO₄ getrocknet und einrotiert. Nach Filtration des Rohproduktes über Kieselgel mit Toluol wird der verbleibende Rückstand aus Heptan/Toluol umkristallisiert und abschließend im Hochvakuum sublimiert, Reinheit beträgt 99.9%. Die Ausbeute beträgt 37,8 g (79% der Theorie).

Analog dazu werden die folgenden Verbindungen (**2-2**) bis (**2-10**) hergestellt:

| **Edukt 1** | **Edukt 2** | **Produkt** | **Ausbeute** |
|---|---|---|---|
| 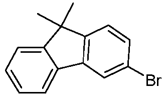 | 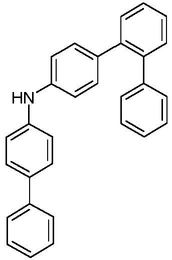 | 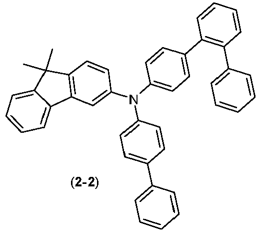 | 78 % |
| 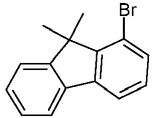 | 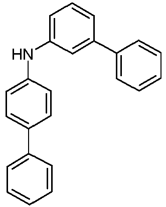 | 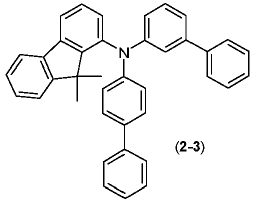 | 82% |
| 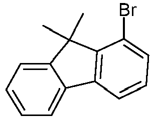 | 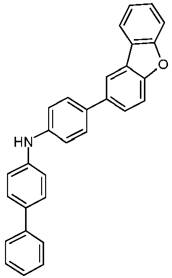 | 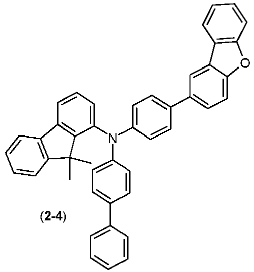 | 80% |
| 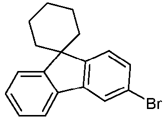 | 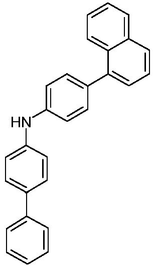 | 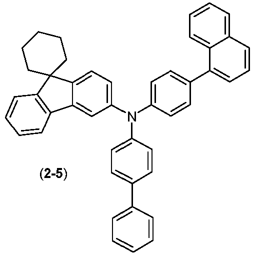 | 92% |
| 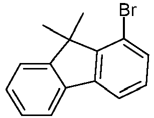 | 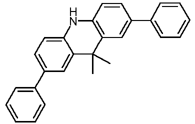 | 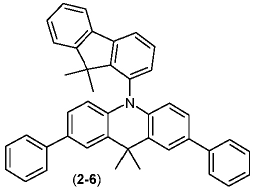 | 75% |
| 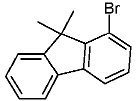 | 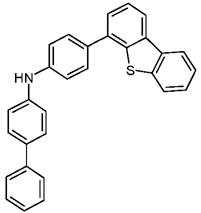 | 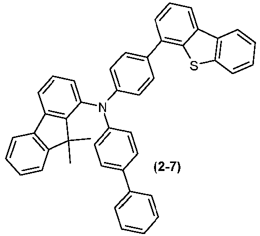 | 67% |
| 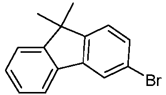 | 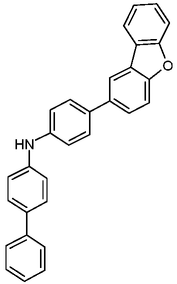 | 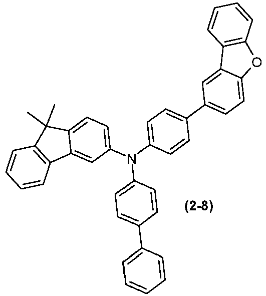 | 73% |
| 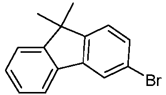 | 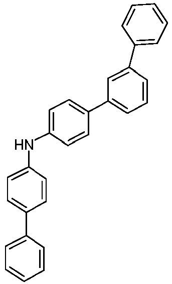 | 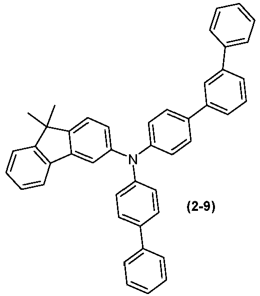 | 70% |
| 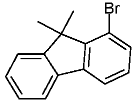 | 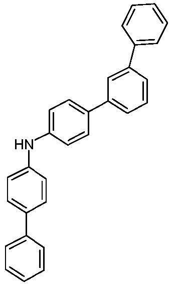 | 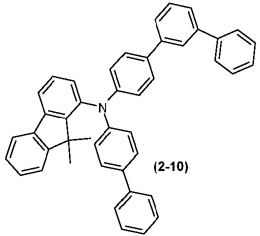 | 66% |

### Beispiel 3

### Synthese der Verbindung Biphenyl-2-yl-biphenyl-4-yl-(9,9-diphenyl-9H-fluoren-3-yl)-amin (3-1) sowie der Verbindungen (3-2) bis (3-5)

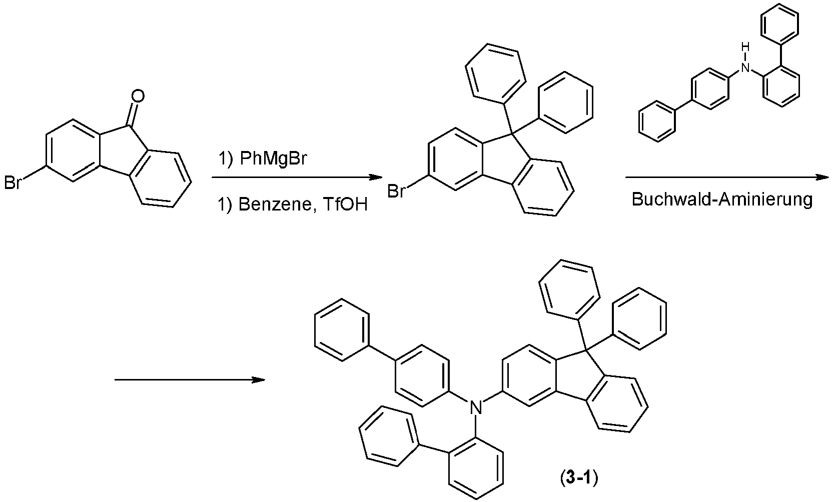

### 3-Bromo-9,9-diphenyl-9H-fluoren

50 g (193 mmol) 3-Bromo-9H-fluorenon (Tetrahedron, 51,7, 2039-54; 1995) werden in einem ausgeheizten Kolben in 500 mL getrocknetem THF gelöst. Die klare Lösung wird auf -10°C abgekühlt und dann werden 70,7 mL (212 mmol) einer 3 M-Phenylmagnesiumbromid Lösung zugegeben. Die Reaktionsmischung wird langsam auf Raumtemperatur erwärmt und dann mit NH₄Cl gequencht (500 mL). Das Gemisch wird im Anschluss zwischen Essigester und Wasser verteilt, die organische Phase dreimal mit Wasser gewaschen, über Na₂SO₄ getrocknet und einrotiert. Das Rohprodukt wird aus Heptan/Toluol umkristallisiert. Der Rückstand wird mit 400 mL Benzol versetzt. Der Ansatz wird auf 50°C erhitzt und anschließen werden 18,6 mL Trifluoromethansulfonsäure zugetropft. Nach 30 min. wird die Reaktionsmischung auf Raumtemperatur abgekühlt und auf 1 L Wasser gegossen. Das Gemisch wird zwischen Toluol und Wasser verteilt, die organische Phase dreimal mit Wasser gewaschen, über Na₂SO₄ getrocknet und einrotiert. Nach Filtration des Rohproduktes über Kieselgel mit (Heptane:Essigsester, 1:1) erhält man 55,6 g (135 mmol) (70% der Theorie).

Analog dazu werden folgenden bromierten Verbindungen hergestellt:

| **Edukt 1** | **Edukt 2** | **Edukt 3** | **Produkt** | **Ausbeute** |
|---|---|---|---|---|
| 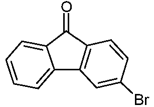 | 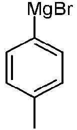 | 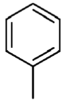 | 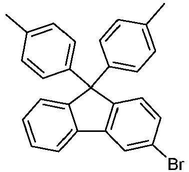 | 75% |
| 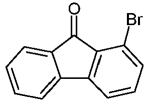 | 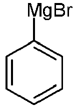 | 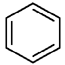 | 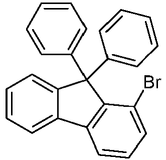 | 65% |

**Biphenyl-2-yl-biphenyl-4-yl-(9,9-diphenyl-9H-fluoren-3-yl)-amin (3-1)** 12 g Biphenyl-2-yl-biphenyl-4-yl-amine (37 mmol), 16,3 g 3-Bromo-9,9-diphenyl-9H-fluorene (41 mol) werden in 360 mL Toluol gelöst: Die Lösung wird entgast und mit N₂ gesättigt. Danach wird sie mit 3,7 mL (3,7 mmol) einer 1 M Lösung Tri-tert-Butylphosphin und 0,42 g (1,87 mmol) Palladium(II)acetat versetzt. Anschließend werden 9,0 g Natrium-tert-butylat (93,3 mmol) zugegeben. Die Reaktionsmischung wird 3 h unter Schutzatmosphäre zum Sieden erhitzt. Das Gemisch wird im Anschluss zwischen Toluol und Wasser verteilt, die organische Phase dreimal mit Wasser gewaschen, über Na₂SO₄ getrocknet und einrotiert. Nach Filtration des Rohproduktes über Kieselgel mit Toluol wird der verbleibende Rückstand aus Heptan/Toluol umkristallisiert und abschließend im Hochvakuum sublimiert, Reinheit beträgt 99.9%. Die Ausbeute beträgt 20 g (85% der Theorie).

Analog dazu werden die folgenden Verbindungen (**3-2**) bis (**3-5**) hergestellt:

| **Edukt 1** | **Edukt 2** | **Produkt** | **Ausbeute** |
|---|---|---|---|
| 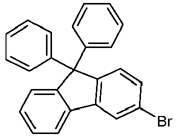 | 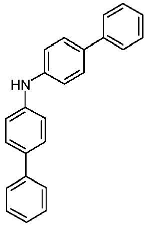 | 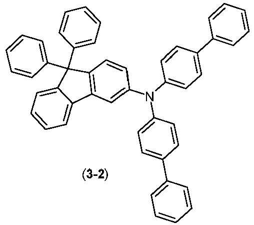 | 78% |
| 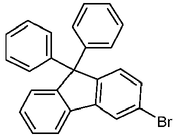 | 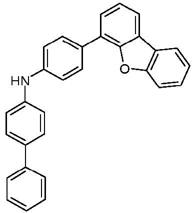 | 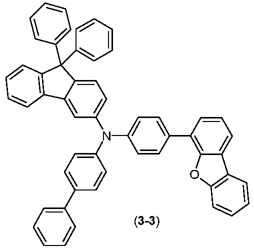 | 80% |
| 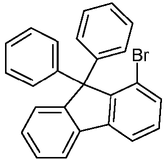 | 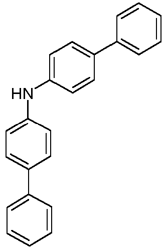 | 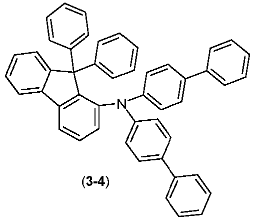 | 85% |
| 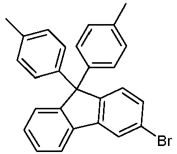 | 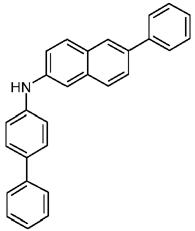 | 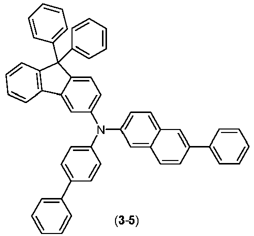 | 80% |

### Beispiel 4

### Synthese der Verbindung Bis-biphenyl-4-yl-[4-(9,9-diphenyl-9H-fluoren-4-yl)-phenyl]-amin (4-1) sowie der Verbindungen (4-2) bis (4-7)

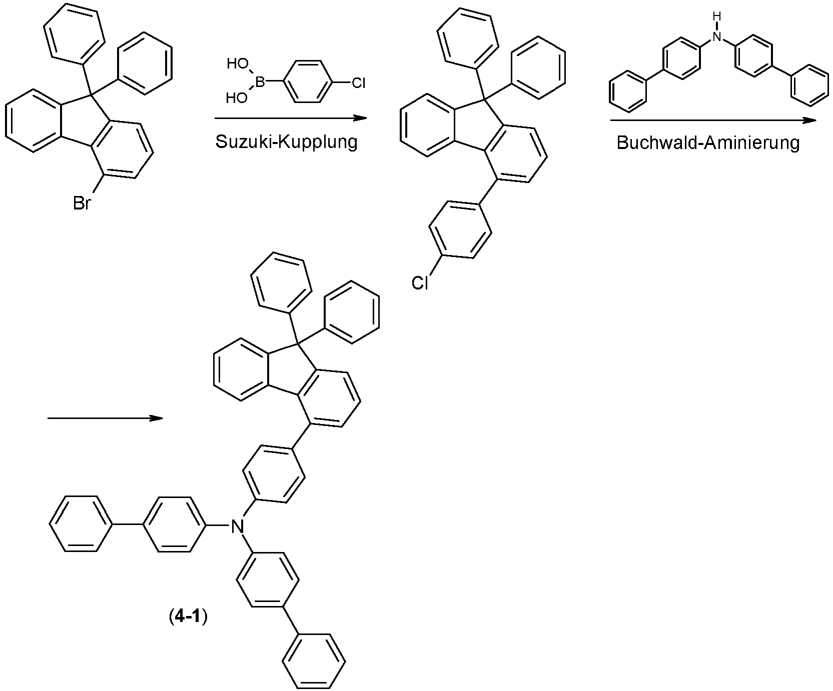

### 4-(4-Chloro-phenyl)-9,9-diphenyl-9H-fluoren

7,9 g (50 mmol) 4-Chlor-benzolboronsäure, 20 g (50 mmol) 4-Brom-9,9-diphenyl-9H-fluoren und 55 mL einer wässrigen 2 M NaHCO₃-Lösung (111 mmol) werden in 400 mL Dimethoxyethan suspendiert. Zu dieser Suspension werden 1,45 g (1,26 mmol) Tetrakis-(triphenyl)phosphin-palladium(0) gegeben. Die Reaktionsmischung wird 16 h unter Rückfluss erhitzt. Nach Erkalten wird die organische Phase abgetrennt, über Kieselgel filtriert, dreimal mit 300 mL Wasser gewaschen und anschließend zur Trockene eingeengt. Nach Filtration des Rohproduktes über Kieselgel mit Heptan/Essigsäureester (20:1) erhält man 18,4 g (85%) 4-(4-Chlorophenyl)-9,9-diphenyl-9H-fluoren.

Analog dazu werden folgende chlorierten Verbindungen hergestellt:

| **Edukt 1** | **Edukt 2** | **Produkt** | **Ausbeute** |
|---|---|---|---|
| 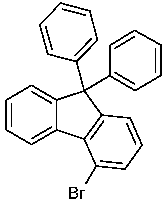 | 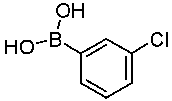 | 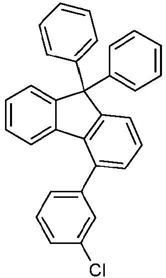 | 79% |
| 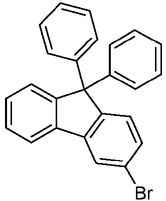 | 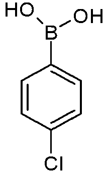 | 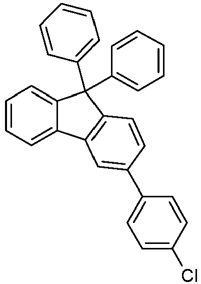 | 70% |
| 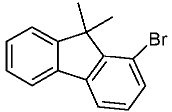 | 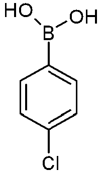 | 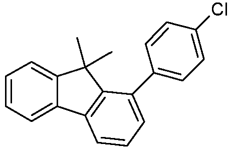 | 72% |

**Bis-biphenyl-4-yl-[4-(9,9-diphenyl-9H-fluoren-4-yl)-phenyl]-amin (4-1)** 13.60 g Bis-biphenyl-4-yl-amin (43 mmol) und 18.2 g 4-Chlor-9,9-diphenyl-9H-fluoren (43 mmol) werden in 400 mL Toluol gelöst: Die Lösung wird entgast und mit N₂ gesättigt. Danach wird sie mit 1,04g(2,55 mmol) SPhos und 1,94 g (2.1307 mmol) Palladium(II)dba versetzt. Anschließend werden 10 g Natrium-tert-butylat (106 mmol) zugegeben. Die Reaktionsmischung wird 3 h unter Schutzatmosphäre zum Sieden erhitzt. Das Gemisch wird im Anschluss zwischen Toluol und Wasser verteilt, die organische Phase dreimal mit Wasser gewaschen, über Na₂SO₄ getrocknet und einrotiert. Nach Filtration des Rohproduktes über Kieselgel mit Toluol wird der verbleibende Rückstand aus Heptan/Toluol umkristallisiert und abschließend im Hochvakuum sublimiert. Die Reinheit beträgt 99.9%. Die Ausbeute beträgt 23 g (77% der Theorie).

Analog dazu werden folgende Verbindungen hergestellt:

| **Edukt 1** | **Edukt 2** | **Produkt** | **Ausbeute** |
|---|---|---|---|
| 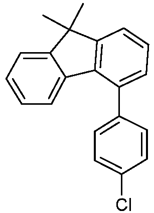 | 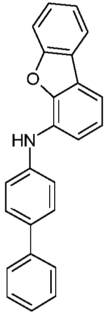 | 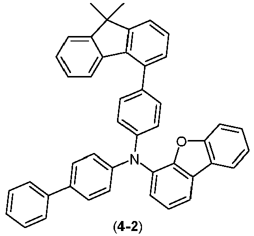 | 78% |
| 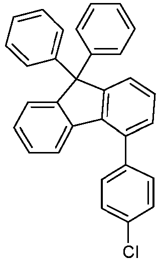 | 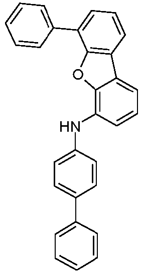 | 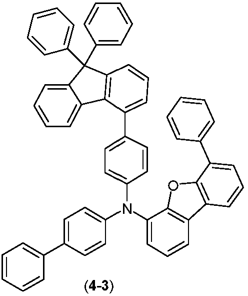 | 70% |
| 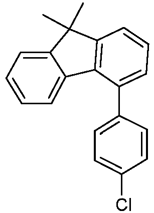 | 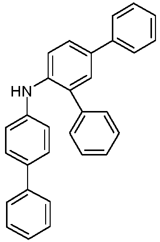 | 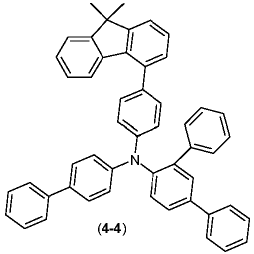 | 75% |
| 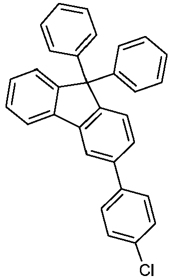 | 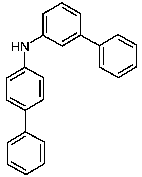 | 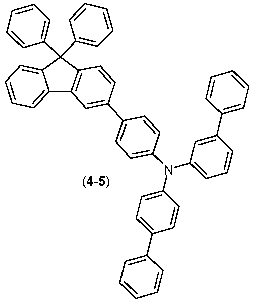 | 88% |
| 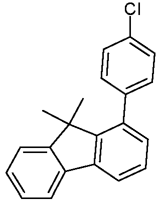 | 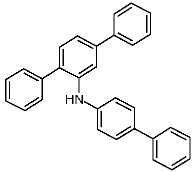 | 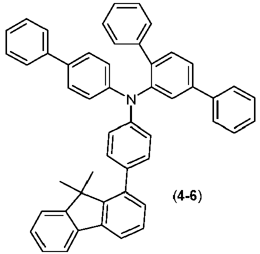 | 73% |
| 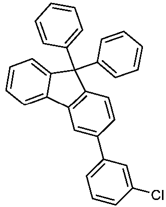 | 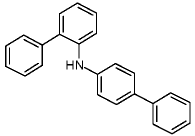 | 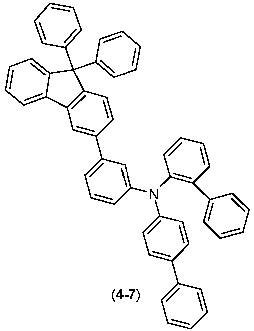 | 73% |

### Beispiel 5

### Synthese der Verbindung Bis-biphenyl-4-yl-[9,9-dimethyl-1-(9-phenyl-9H-carbazol-3-yl)-9H-fluoren-4-yl]-amine (5-1) sowie der Verbindungen (5-2) bis (5-5)

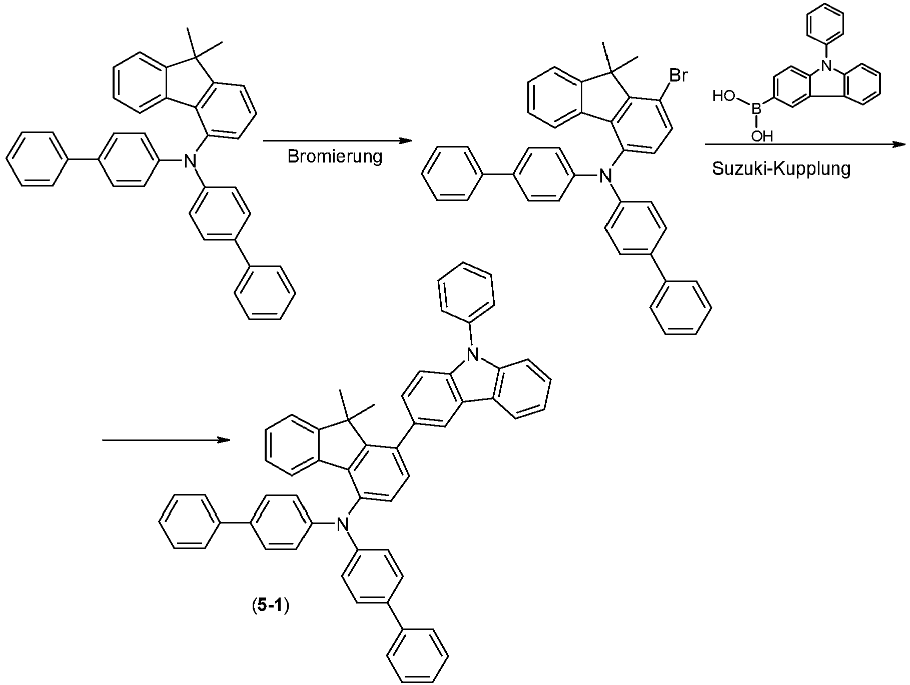

**Bis-biphenyl-4-yl-(1-bromo-9,9-dimethyl-9H-fluoren-4-yl)-amine** 15.0 g (29 mmol) Bis-biphenyl-4-yl-(9,9-dimethyl-9H-fluoren-4-yl)-amine werden in 150 mL Acetonitril gelöst und bei Raumtemperatur portionsweise mit 5.2 g (29 mmol) N-Bromsuccinimid portionsweise, versetzt. Nach vollständiger Umsetzung wird Wasser und Essigsäureethylester dazu gegeben, die organische Phase abgetrennt, getrocknet und eingeengt. Das Rohprodukt wird anschließend mit MeOH/Heptan (1:1) mehrfach heiß ausgerührt. Ausbeute: 13,5 g (80%) des Produktes.

Analog dazu werden die folgenden bromierten Verbindungen hergestellt:

| **Edukt 1** | **Produkt** | **Ausbeute** |
|---|---|---|
| 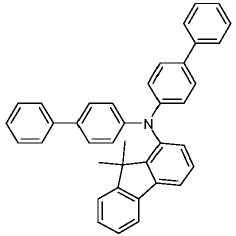 | 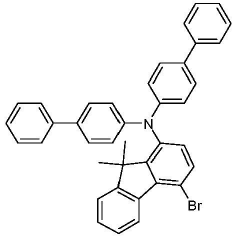 | 85% |
| 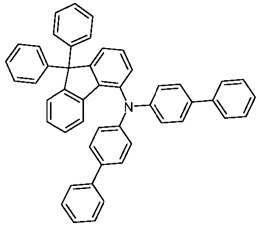 | 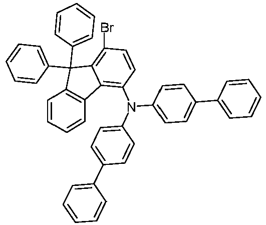 | 81% |

### Bis-biphenyl-4-yl-[9,9-dimethyl-1-(9-phenyl-9H-carbazol-3-yl)-9H-fluoren-4-yl]-amin (5-1)

6.3 g (22 mmol) N-Phenylcarbazol-3yl-boronsäure und 13 g (22 mmol) Bis-biphenyl-4-yl-(1-bromo-9,9-dimethyl-9H-fluoren-4-yl)-amine werden in 200 mL Dimethoxyethan und 30 mL 2 M Na₂CO₃ Lösung suspendiert. Zu dieser Suspension werden 0.6 g (2,0 mmol) Palladium tetrakis(triphenylphosphin) gegeben. Die Reaktionsmischung wird 16 h unter Rückfluss erhitzt. Nach dem Erkalten wird die Reaktionsmischung mit Essigester verdünnt, die organische Phase abgetrennt, dreimal mit 100 mL Wasser gewaschen und anschließend zur Trockene eingeengt. Nach Filtration des Rohproduktes über Kieselgel mit Heptan/Essigsäureester (20:1) erhält man 15 g (90%) Bis-biphenyl-4-yl-[9,9-dimethyl-1-(9-phenyl-9H-carbazol-3-yl)-9H-fluoren-4-yl]-amin (**5-1**).

Analog dazu werden die Verbindungen (**5-2**) bis (**5-5**) hergestellt:

| **Edukt 1** | **Edukt 2** | **Produkt** | **Ausbeute** |
|---|---|---|---|
| 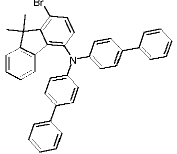 | 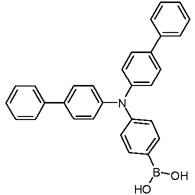 | 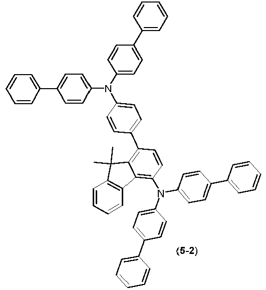 | 85% |
| 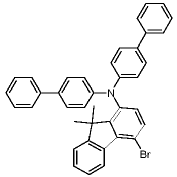 | 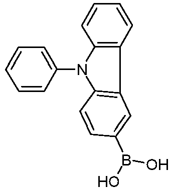 | 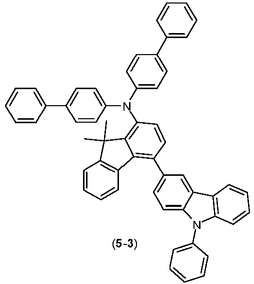 | 81% |
| 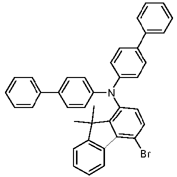 | 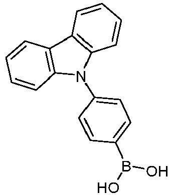 | 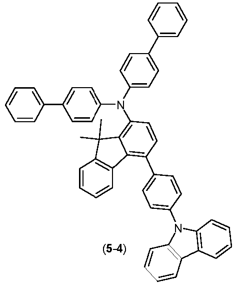 | 88% |
| 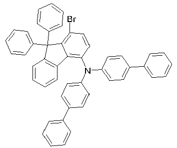 | 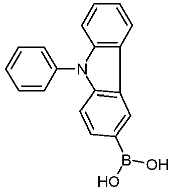 | 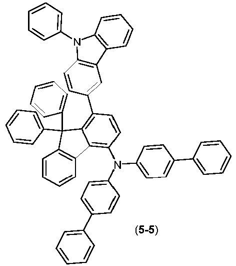 | 85% |

### Beispiel 6

### Synthese der Verbindung Biphenyl-4-yl-(4-dibenzofuran-4-yl-phenyl)-[4-(9,9-diphenyl-9H-fluoren-4-yl)-phenyl]-amine (7-1) sowie der Verbindungen (6-2) bis (6-5)

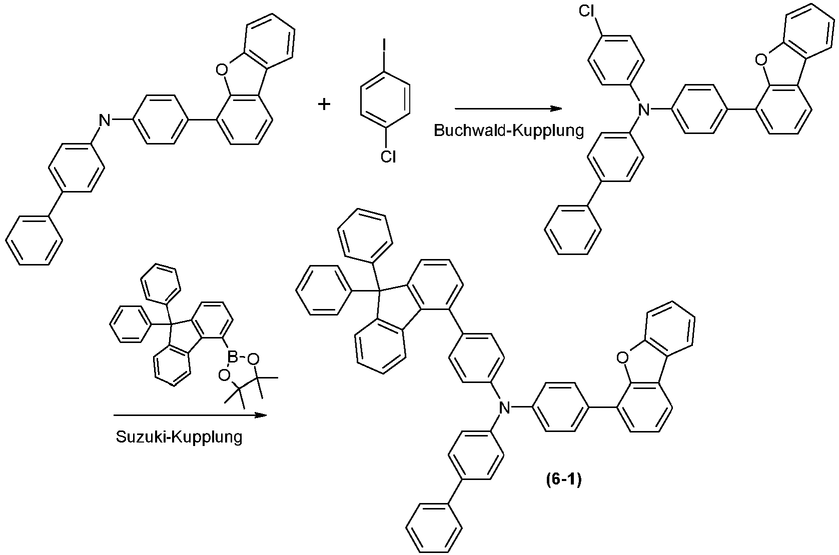

### Biphenyl-4-yl-(4-chloro-phenyl)-(4-dibenzofuran-4-yl-phenyl)-amine

30.0 g Biphenyl-4-yl-(4-dibenzofuran-4-yl-phenyl)-amin (CAS: 955959-89-4) (73 mmol) und 17.4 g 1-Chlor-2-Iodbenzol-(73 mmol) werden in 460 mL Toluol gelöst: Die Lösung wird entgast und mit N₂ gesättigt. Danach wird sie mit 2.9 mL (2,9 mmol) einer 1 M Tri-tert-Butylphosphin-Lösung und 0.33 g (1.46 mmol) Palladium(II)acetat versetzt. Anschließend werden 10,5 g Natrium-tert-butylat (109 mmol) zugegeben. Die Reaktionsmischung wird 3 h unter Schutzatmosphäre zum Sieden erhitzt. Das Gemisch wird im Anschluss zwischen Toluol und Wasser verteilt, die organische Phase dreimal mit Wasser gewaschen, über Na₂SO₄ getrocknet und einrotiert. Nach Filtration des Rohproduktes über Kieselgel mit Toluol wird der verbleibende Rückstand aus Heptan/Toluol umkristallisiert und abschließend im Hochvakuum sublimiert. Die Reinheit beträgt 99.9%. Die Ausbeute beträgt 30 g (80% der Theorie).

Analog dazu werden folgende Verbindungen hergestellt:

| **Edukt 1** | **Edukt 2** | **Produkt** | **Ausbeute** |
|---|---|---|---|
| 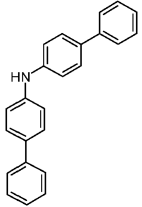 | 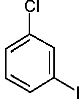 | 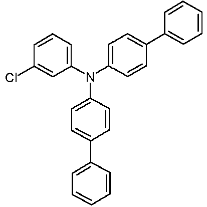 | 83% |
| 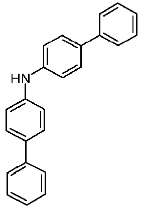 | 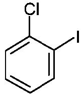 | 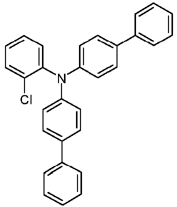 | 80% |
| 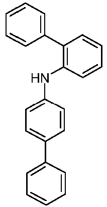 | 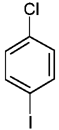 | 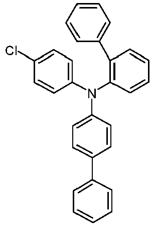 | 79% |

### Biphenyl-4-yl-(4-dibenzofuran-4-yl-phenyl)-[4-(9,9-diphenyl-9H-fluoren-4-yl)-phenyl]-amine (6-1)

20.0 g (45 mmol) (9,9-Diphenyl-9H-fluoren-4-yl)-pinacolboronester, 23.5 g (45 mmol) Biphenyl-4-yl-(4-chloro-phenyl)-(4-dibenzofuran-4-yl-phenyl)-amine werden in 400 mL Dioxan und 13.7 g Caesiumfluorid (90 mmol) suspendiert. Zu dieser Suspension werden 4.0 g (5.4 mmol) Palladium dichlorid-bis(tricyclohexylphosphin) gegeben, und die Reaktionsmischung wird 18 h unter Rückfluss erhitzt. Nach Erkalten wird die organische Phase abgetrennt, über Kieselgel filtriert, dreimal mit 80 mL Wasser gewaschen und anschließend zur Trockene eingeengt. Nach Filtration des Rohproduktes über Kieselgel mit Toluol wird der verbleibende Rückstand aus Heptan/Toluol umkristallisiert und abschließend im Hochvakuum sublimiert, Reinheit beträgt 99.9%. Die Ausbeute beträgt 25 g (80% d. Th).

Analog dazu werden die folgende Verbindungen (6-2) bis (6-5) hergestellt:

| **Edukt 1** | **Edukt 2** | **Produkt** | **Ausbeute** |
|---|---|---|---|
| 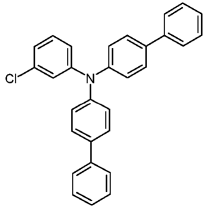 | 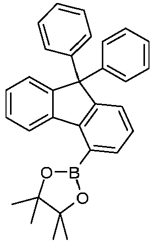 | 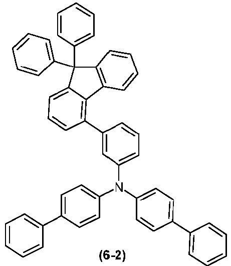 | 65% |
| 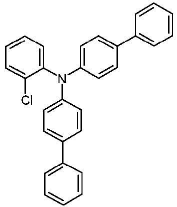 | 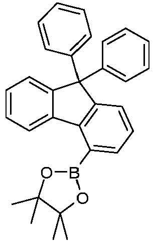 | 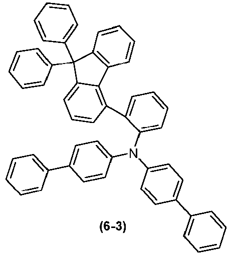 | 69% |
| 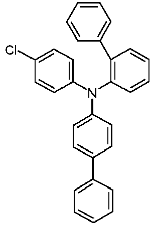 | 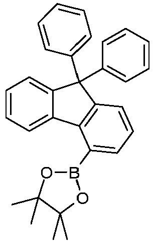 | 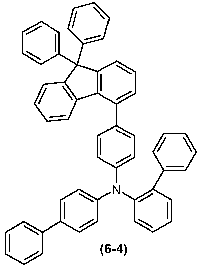 | 75% |
| 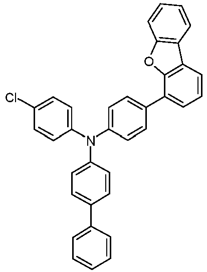 | 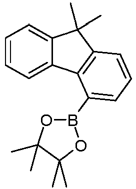 | 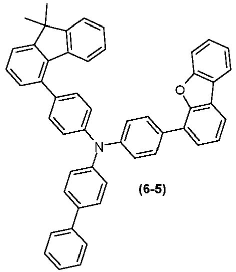 | 65% |

### Beispiel 7

### Synthese der Verbindung Bis-biphenyl-4-yl-(7-dibenzofuran-4-yl-9,9-diphenyl-9H-fluoren-4-yl)-amin (7-1) sowie der Verbindungen (7-2) bis (7-5)

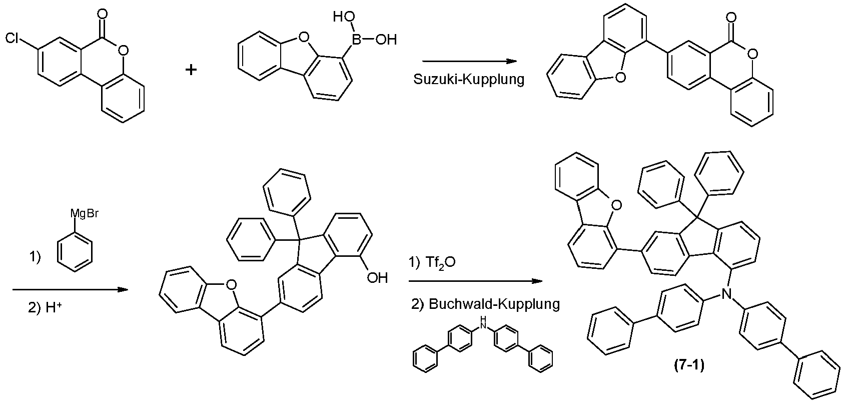

### 8-Dibenzofuran-4-yl-benzo[c]chromen-6-one

30.0 g (142 mmol) Dibenzofuran-4-Boronsäure, 32 g (142 mmol) 8-Chloro-benzo[c]chromen-6-on (CAS: 742058-81-7) und 43 g Caesiumfluorid (283 mmol) werden in 800 mL Dioxan suspendiert. Zu dieser Suspension werden 12.5 g (17 mmol) Palladium dichlorid-bis(tricyclohexylphosphin) gegeben, und die Reaktionsmischung wird 18 h unter Rückfluss erhitzt. Nach Erkalten wird die organische Phase abgetrennt, über Kieselgel filtriert, dreimal mit 100 mL Wasser gewaschen und anschließend zur Trockene eingeengt. Nach Filtration des Rohproduktes über Kieselgel mit Toluol wird der verbleibende Rückstand aus Heptan/Toluol umkristallisiert Die Ausbeute beträgt 45 g (88% d. Th).

Analog dazu werden folgende Verbindungen hergestellt:

| **Edukt 1** | **Edukt 2** | **Produkt** | **Ausbeute** |
|---|---|---|---|
| 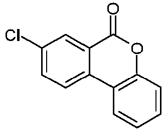 | 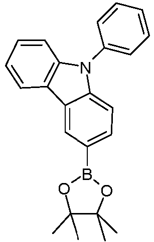 | 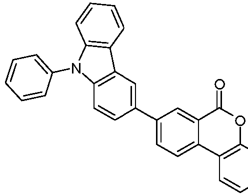 | 84% |
| 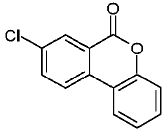 | 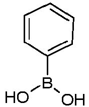 | 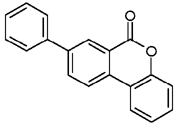 | 90% |
| 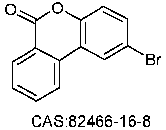 | 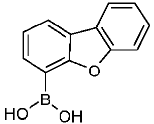 | 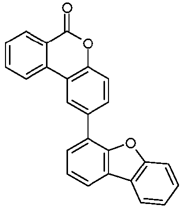 | 85% |
| 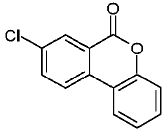 | 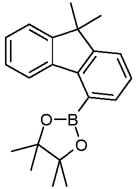 | 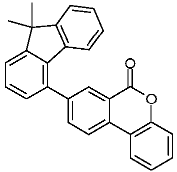 | 76% |

### 7-Dibenzofuran-4-yl-9,9-diphenyl-9H-fluoren-4-ol

25.4 g (70 mmol) 8-Dibenzofuran-4-yl-benzo[c]chromen-6-on werden in einem ausgeheizten Kolben in 340 mL getrocknetem THF gelöst. Die Lösung wird mit N₂ gesättigt. Die klare Lösung wird auf -10°C abgekühlt und dann werden 70 mL (210 mmol) einer 3M-Phenhylmagnesiumbromid Lösung zugegeben. Die Reaktionsmischung wird langsam auf Raumtemperatur erwärmt und dann mit Essigsäureanhidrid gequencht (70 mmol). Das Gemisch wird im Anschluss zwischen Essigester und Wasser verteilt, die organische Phase dreimal mit Wasser gewaschen, über Na₂SO₄ getrocknet und einrotiert. Die einrotierte Lösung wird vorsichtig mit 310 ml Essigsäure versetzt und anschließen werden 70 ml rauchende HCl zugegeben. Der Ansatz wird auf 75°C erhitzt und 4 h auf dieser Temperatur gehalten. Dabei fällt ein weißer Feststoff aus. Der Ansatz wird dann auf Raumtemperatur abgekühlt, der ausgefallene Feststoff wird abgesaugt und mit Methanol nachgewaschen. Der Rückstand wird im Vakuum bei 40°C getrocknet. Nach Filtration des Rohproduktes über Kieselgel mit (Heptane:Essigsester, 1:1) erhält man 26 g (75% der Theorie)

Analog dazu werden folgende bromierten Verbindungen hergestellt:

| **Edukt 1** | **Edukt 2** | **Produkt** | **Ausbeute** |
|---|---|---|---|
| 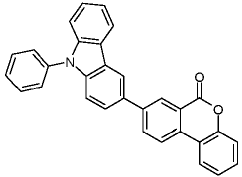 | 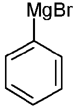 | 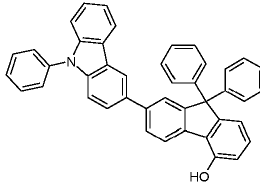 | 72% |
| 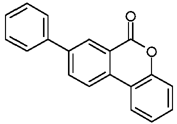 | 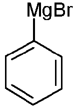 | 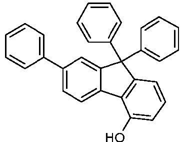 | 80% |
| 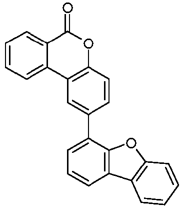 | 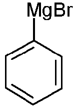 | 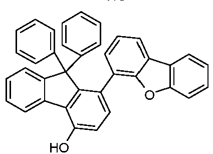 | 77% |
| 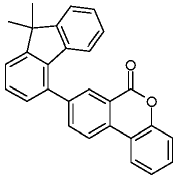 | CH₃MgBr | 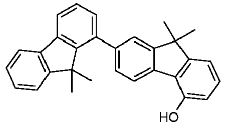 | 72% |

### Bis-biphenyl-4-yl-(7-dibenzofuran-4-yl-9,9-diphenyl-9H-fluoren-4-yl)-amine (7-1)

25 g (50 mmol) 7-Dibenzofuran-4-yl-9,9-diphenyl-9H-fluoren-4-ol werden in einem ausgeheizten Kolben in 300 mL getrocknetem THF gelöst. Die Lösung wird mit N₂ gesättigt. Die klare Lösung wird auf 5°C abgekühlt und dann werden 20 mL (150 mmol) Triethylamin, 122 mg 4-dimethylaminopyridine und 8.65 mL trifluoromethansulfonsäure anhydride zugegeben. Die Reaktionsmischung wird langsam auf Raumtemperatur erwärmt. Das Reaktiongemisch wird im Anschluss mit Heptan verdünnt, einrotiert und Wasser verteilt, die organische Phase dreimal mit Wasser gewaschen, über Na₂SO₄ getrocknet und einrotiert. Nach Filtration des Rohproduktes über Kieselgel mit Heptane:Essigsester, 1:1) erhält man 30 g (98% der Theorie)

18.9 g des Triflates (30 mmol) und 8,16 g Bis-4-Biphenylamin (25 mmol) werden in 240 mL Toluol gelöst: Die Lösung wird entgast und mit N₂ gesättigt. Danach wird sie mit 0.74 g (1.79 mmol) SPhos und 1.36 g Palladium-dba (1.49 mmol) versetzt. Anschließend werden 5.7 g Natrium-tert-butylat (59.7 mmol) zugegeben. Die Reaktionsmischung wird 3 h unter Schutzatmosphäre auf 85°C erhitzt. Das Gemisch wird im Anschluss zwischen Toluol und Wasser verteilt, die organische Phase dreimal mit Wasser gewaschen, über Na₂SO₄ getrocknet und einrotiert. Nach Filtration des Rohproduktes über Kieselgel mit Toluol wird der verbleibende Rückstand aus Heptan/Toluol umkristallisiert. Die Reinheit beträgt 99.9%. Die Ausbeute beträgt 15 g (65% der Theorie).

Analog dazu werden folgende Verbindungen hergestellt:

| **Edukt 1** | **Edukt 2** | **Produkt** | **Ausbeute** |
|---|---|---|---|
| 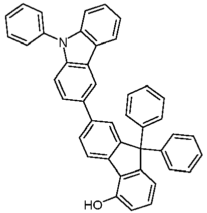 | 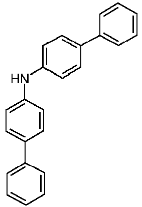 | 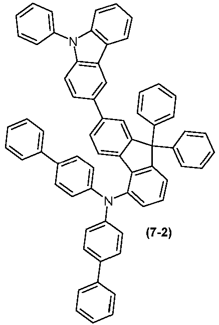 | 73% |
| 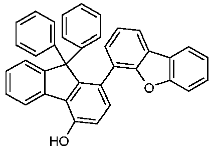 | 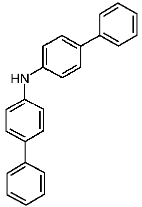 | 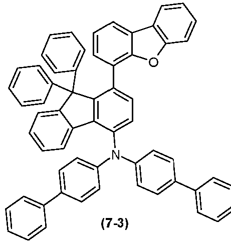 | 80% |
| 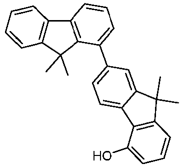 | 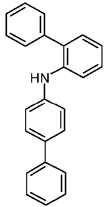 | 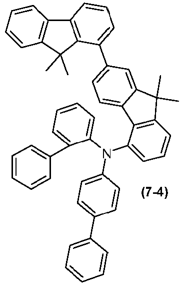 | 69% |
| 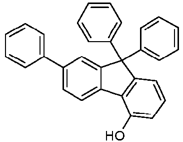 | 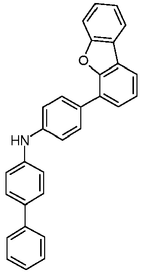 | 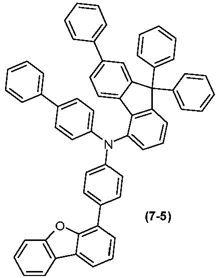 | 75% |

### Beispiel 8

### Synthese der Verbindung N*2*,N*5*,N*5*-Tris-biphenyl-4-yl-N*2*-biphenyl-2-yl-9,9-diphenyl-9H-fluorene-2,5-diamin (8-1)

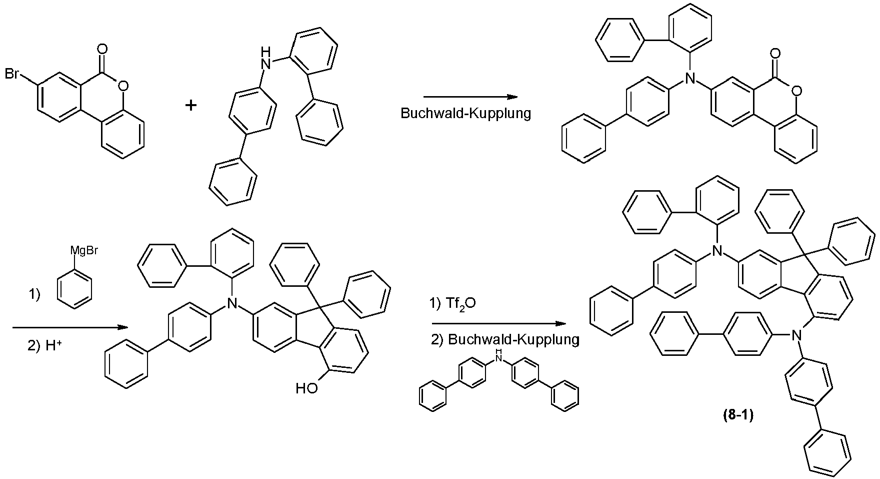

### 8-(Biphenyl-4-yl-biphenyl-2-yl-amino)-benzo[c]chromen-6-one

19.0 g Biphenyl-2-yl-biphenyl-4-yl-amin (59 mmol) und 16.3 g 8-Bromo-benzo[c]chromen-6-one-(59 mmol) werden in 400 mL Toluol gelöst: Die Lösung wird entgast und mit N₂ gesättigt. Danach wird sie mit 2.36 mL (2.36 mmol) einer 1 M Tri-tert-Butylphosphin-Lösung und 0.27 g (1.18 mmol) Palladium(II)acetat versetzt. Anschließend werden 11.6 g Natrium-tert-butylat (109 mmol) zugegeben. Die Reaktionsmischung wird 3 h unter Schutzatmosphäre zum Sieden erhitzt. Das Gemisch wird im Anschluss zwischen Toluol und Wasser verteilt, die organische Phase dreimal mit Wasser gewaschen, über Na₂SO₄ getrocknet und einrotiert. Nach Filtration des Rohproduktes über Kieselgel mit Toluol wird der verbleibende Rückstand aus Heptan/Toluol. Die Ausbeute beträgt 27 g (90% der Theorie).

Analog dazu werden folgende Verbindungen hergestellt:

| **Edukt 1** | **Edukt 2** | **Produkt** | **Ausbeute** |
|---|---|---|---|
| 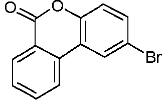 | 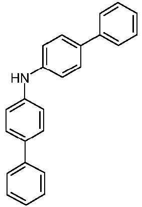 | 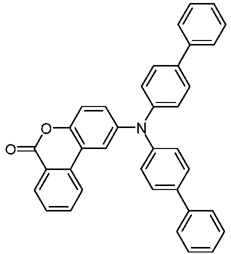 | 85% |
| 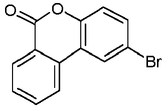 | 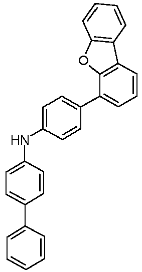 | 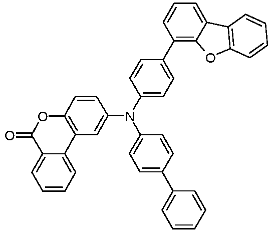 | 75% |

### 7-(Biphenyl-4-yl-biphenyl-2-yl-amino)-9,9-diphenyl-9H-fluoren-4-ol

Analog zu 7-Dibenzofuran-4-yl-9,9-diphenyl-9H-fluoren-4-ol werden folgende Verbindungen hergestellt:

| **Edukt 1** | **Edukt 2** | **Produkt** | **Ausbeute** |
|---|---|---|---|
| 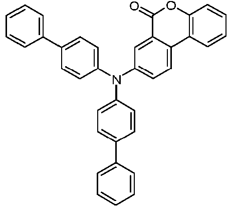 | 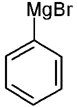 | 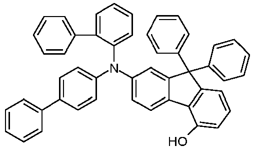 | 88% |
| 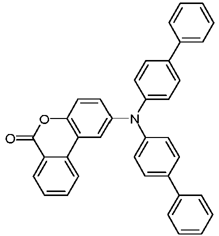 | 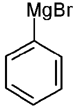 | 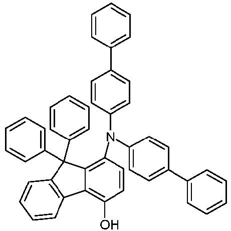 | 73% |
| 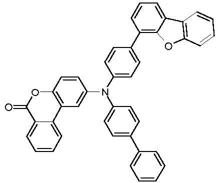 | CH₃MgBr | 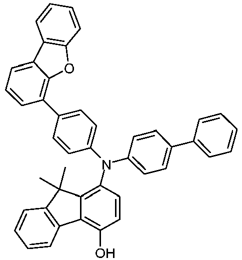 | 76% |

### N*2*,N*5*,N*5*-Tris-biphenyl-4-yl-N*2*-biphenyl-2-yl-9,9-diphenyl-9H-fluorene-2,5-diamin (8-1)

Analog zu Bis-biphenyl-4-yl-(7-dibenzofuran-4-yl-9,9-diphenyl-9H-fluoren-4-yl)-amine Verbindung (7-1) wird Verbindung (8-1) hergestellt:

### Beispiel 9

### Synthese der Verbindung Bis-biphenyl-4-yl-(4-dibenzofuran-4-yl-9,9-diphenyl-9H-fluoren-1-yl)-amin (9-1) sowie der Verbindungen (9-2) und (9-3)

| **Edukt 1** | **Edukt 2** | **Produkt** | **Ausbeute** |
|---|---|---|---|
| 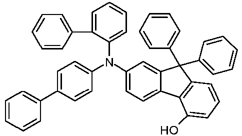 | 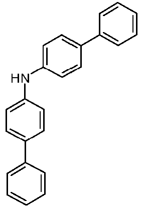 | 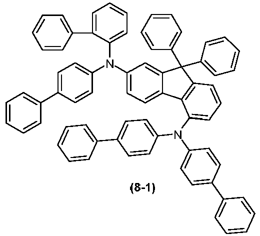 | 74% |

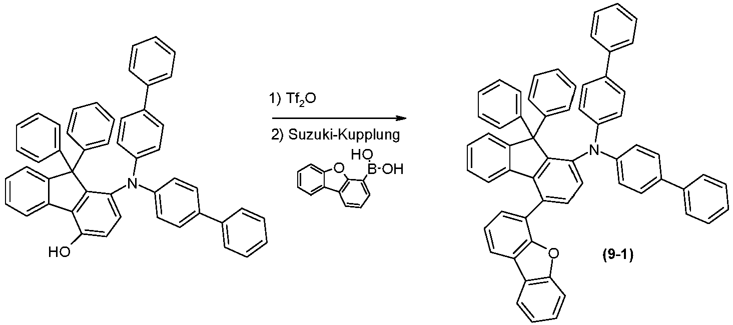

24.4 g (37 mmol) 1-(Bis-biphenyl-4-yl-amino)-9,9-diphenyl-9H-fluoren-4-ol werden in einem ausgeheizten Kolben in 210 mL getrocknetem THF gelöst. Die Lösung wird mit N₂ gesättigt. Die klare Lösung wird auf 5°C abgekühlt und dann werden 15.5 mL (112 mmol) Triethylamin, 100 mg 4-dimethylaminopyridine und 6.45 mL Trifluoromethansulfonsäureanhydrid zugegeben. Die Reaktionsmischung wird langsam auf Raumtemperatur erwärmt. Das Reaktiongemisch wird im Anschluss mit Heptan verdünnt, einrotiert und Wasser verteilt, die organische Phase dreimal mit Wasser gewaschen, über Na₂SO₄ getrocknet und einrotiert. Nach Filtration des Rohproduktes über Kieselgel mit Heptane:Essigsester, 1:1) erhält man 26,7 g (91% der Theorie)

17.2 g (22 mmol) 1-(Bis-biphenyl-4-yl-amino)-9,9-diphenyl-9H-fluoren-4-ol, 6.9 g (33 mmol) 4-Bibenzofuranboronsäure, 9.0 g natriummetaborat-Oktahydrat (32.9 mmol) und 0.03 mL hydraziniumhydroxid (0.657 mmol) werden in 200 mL THF suspendiert. Zu dieser Suspension werden 0.3 g (0.44 mmol) Palladium dichlorid-bis(triphenylphosphin) gegeben, und die Reaktionsmischung wird 18 h auf 70°C erhitzt. Nach dem Erkalten wird das Gemisch zwischen Essigester und Wasser verteilt, die organische Phase dreimal mit Wasser gewaschen, über Na₂SO₄ g getrocknet und anschließend zur Trockene eingeengt. Nach Filtration des Rohproduktes über Kieselgel mit Toluol wird der verbleibende Rückstand aus Heptan/Toluol umkristallisiert und anschliessen sublimiert. Die Ausbeute beträgt 12 g (70% d. Th).

Analog dazu werden folgende Verbindungen hergestellt:

| **Edukt 1** | **Edukt 2** | **Produkt** | **Ausbeute** |
|---|---|---|---|
| 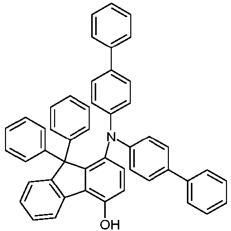 | 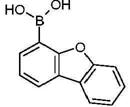 | 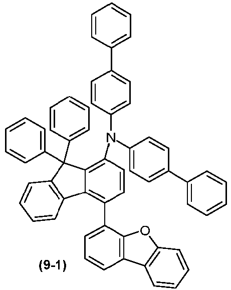 | 74% |
| 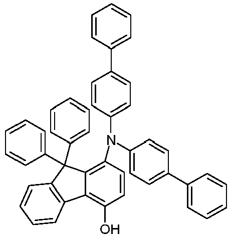 | 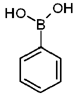 | 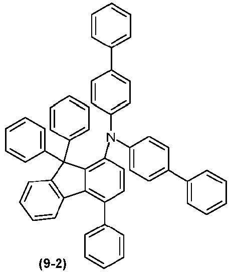 | 73% |
| 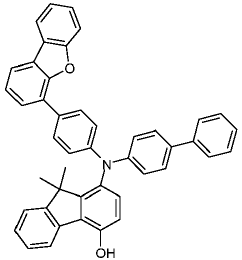 | 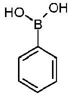 | 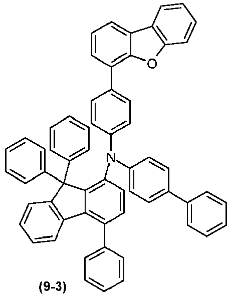 | 76% |

### Beispiel 10

### Charakterisierung der Verbindungen

Die Herstellung von erfindungsgemäßen OLEDs sowie OLEDs nach dem Stand der Technik erfolgt nach einem allgemeinen Verfahren gemäß WO 04/058911, das auf die hier beschriebenen Gegebenheiten (Schichtdickenvariation, Materialien) angepasst wird.

In den folgenden Beispielen V1 bis V13 und E1 bis E43 (siehe Tabellen 1, 3 und 2, 4) werden die Daten verschiedener OLEDs dargestellt. Als Substrate werden Glasplättchen verwendet, die mit strukturiertem ITO (Indium Zinn Oxid) der Dicke 50 nm beschichtet sind. Die OLEDs haben prinzipiell folgenden Schichtaufbau: Substrat / Optionale Lochinjektionsschicht (HIL1) / Lochtransportschicht (HTL) / Lochinjektionsschicht (HIL2) / Elektronenblockerschicht (EBL) / Emissionsschicht (EML) / Elektronentransportschicht (ETL) / Optionale Elektroneninjektionsschicht (EIL) und abschließend eine Kathode. Die Kathode wird durch eine 100 nm dicke Aluminiumschicht gebildet. Der genaue Aufbau der OLEDs ist den Tabellen 1 und 3 zu entnehmen. Die zur Herstellung der OLEDs benötigten Materialien sind oben offenbart.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie H1:SEB1 (95%:5%) bedeutet hierbei, dass das Material H1 in einem Volumenanteil von 95% und SEB1 in einem Anteil von 5% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in Im/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 cd/m² bestimmt und daraus die CIE 1931 x und y Farbkoordinaten berechnet. Die Angabe EQE @ 1000 cd/m² bezeichnet die externe Quanteneffizienz bei einer Betriebsleuchtdichte von 1000 cd/m². LD80 @ 6000 cd/m² ist die Lebensdauer bis das OLED bei einer Helligkeit von 6000 cd/m² auf 80% der Anfangsintensität, also auf 4800 cd/m² abgefallen ist. Die Daten der verschiedenen OLEDs sind in den Tabellen 2 und 4 zusammengefasst.

### Verwendung von erfindungsgemäßen Verbindungen als Lochtransportmaterialien in OLEDs

Insbesondere eignen sich erfindungsgemäße Verbindungen als HIL, HTL oder EBL in OLEDs. Sie eignen sich als Einzelschicht, aber auch als Mixed Komponente als HIL, HTL, EBL oder innerhalb der EML.

Verglichen mit NPB Referenz Bauteilen (V1, V8) zeigen die Proben mit den erfindungsgemäßen Verbindungen sowohl höhere Effizienzen, als auch deutlich verbesserte Lebensdauern sowohl in Singulett blau als auch in Triplett grün.

Im Vergleich zum Referenzmaterial HTMV1 (V2, V9) hat die erfindungsgemäße Verbindung (**1-1**) (E1, E7) deutlich bessere Lebensdauern in blau und grün.

Im Vergleich zum Referenzmaterial HTMV2 - HTMV6 (V3 - V7 und V9 - V13) zeigen die erfindungsgemäßen Materialien **(9-2), (2-7)** und **(2-10)** bessere Lebensdauern in blau und/oder grün.

### Verwendung von erfindungsgemäßen Verbindungen als Lochtransportmaterialien in fluorszierenden und phosporezierenden OLEDs

Insbesondere eignen sich erfindungsgemäße Verbindungen als HIL, HTL oder EBL in OLEDs. Sie eignen sich als Einzelschicht, aber auch als Mixed Komponente als HIL, HTL, EBL oder innerhalb der EML.

Verglichen mit NPB Referenz Bauteilen (V1) zeigen alle Proben mit den erfindungsgemäßen Verbindungen sowohl höhere Effizienzen, als auch deutlich verbesserte Lebensdauern in Singulett blau und Triplett grün.

| **Tabelle 1: Aufbau der OLEDs** (Schichtaufbau:Substrat/HIL1/HTL/HIL2/EBL/EML/ETL/EIL(1 nm LiQ)/Kathode) | | | | | | |
|---|---|---|---|---|---|---|
| ***Bsp.*** | ***HIL1*** | ***HTL*** | ***HIL2*** | ***EBL*** | ***EML*** | ***ETL*** |
| | *Dicke* / *nm* | *Dicke* / *nm* | *Dicke* / *nm* | *Dicke* / *nm* | *Dicke* / *nm* | *Dicke* / *nm* |
| *V1* | *HIL1* | *HIL2* | *HIL1* | *NPB* | *H1(95%):SEB1(5%)* | *ETM1(50%):LiQ(50%)* |
| | *5 nm* | *140 nm* | *5 nm* | *20 nm* | *20 nm* | *30 nm* |
| *V2* | *HIL1* | *HIL2* | *HIL1* | *HTMV1* | *H1(95%):SEB1(5%)* | *ETM1(50%):LiQ(50%)* |
| | *5 nm* | *140 nm* | *5 nm* | *20 nm* | *20 nm* | *30 nm* |
| *V3* | *HIL1* | *HIL2* | *HIL1* | *HTMV2* | *H1(95%):SEB1(5%)* | *ETM1(50%):LiQ(50%)* |
| | *5 nm* | *140 nm* | *5 nm* | *20 nm* | *20 nm* | *30 nm* |
| *V4* | *HIL1* | *HIL2* | *HIL1* | *HTMV3* | *H1(95%):SEB1(5%)* | *ETM1(50%):LiQ(50%)* |
| | *5 nm* | *140 nm* | *5 nm* | *20 nm* | *20 nm* | *30 nm* |
| *V5* | *HIL1* | *HIL2* | *HIL1* | *HTMV4* | *H1(95%):SEB1(5%)* | *ETM1(50%):LiQ(50%)* |
| | *5 nm* | *140 nm* | *5 nm* | *20 nm* | *20 nm* | *30 nm* |
| *V6* | *HIL1* | *HIL2* | *HIL1* | *HTMV5* | *H1(95%):SEB1(5%)* | *ETM1(50%):LiQ(50%)* |
| | *5 nm* | *140 nm* | *5 nm* | *20 nm* | *20 nm* | *30 nm* |
| *V7* | *HIL1* | *HIL2* | *HIL1* | *HTMV6* | *H1(95%):SEB1(5%)* | *ETM1(50%):LiQ(50%)* |
| | *5 nm* | *140 nm* | *5 nm* | *20 nm* | *20 nm* | *30 nm* |
| *E1* | *HIL1* | *HIL2* | *HIL1* | *(**1-1**)* | *H1(95%):SEB1(5%)* | *ETM1(50%):LiQ(50%)* |
| | *5 nm* | *140 nm* | *5 nm* | *20 nm* | *20 nm* | *30 nm* |
| *E2* | *HIL1* | *HIL2* | *HIL1* | *(**1-4**)* | *H1(95%):SEB1(5%)* | *ETM1(50%):LiQ(50%)* |
| | *5 nm* | *140 nm* | *5 nm* | *20 nm* | *20 nm* | *30 nm* |
| *E3* | *HIL1* | *HIL2* | *HIL1* | *(**1-7**)* | *H1(95%):SEB1(5%)* | *ETM1(50%):LiQ(50%)* |
| | *5 nm* | *140 nm* | *5 nm* | *20 nm* | *20 nm* | *30 nm* |
| *E4* | *HIL1* | *HIL2* | *HIL1* | *(**5-1**)* | *H1(95%):SEB1(5%)* | *ETM1(50%):LiQ(50%)* |
| | *5 nm* | *140 nm* | *5 nm* | *20 nm* | *20 nm* | *30 nm* |
| *E5* | *HIL1* | *HIL2* | *HIL1* | *(**4-1**)* | *H1(95%):SEB1(5%)* | *ETM1(50%):LiQ(50%)* |
| | *5 nm* | *140 nm* | *5 nm* | *20 nm* | *20 nm* | *30 nm* |
| *E6* | *HIL1* | *HIL2* | *HIL1* | *(**1-12**)* | *H1(95%):SEB1(5%)* | *ETM1(50%):LiQ(50%)* |
| | *5 nm* | *140 nm* | *5 nm* | *20 nm* | *20 nm* | *30 nm* |
| *E7* | *HIL1* | *HIL2* | *HIL1* | *(**1-13**)* | *H1(95%):SEB1(5%)* | *ETM1(50%):LiQ(50%)* |
| | *5 nm* | *140 nm* | *5 nm* | *20 nm* | *20 nm* | *30 nm* |
| *E8* | *HIL1* | *HIL2* | *HIL1* | *(**1-14**)* | *H1(95%):SEB1(5%)* | *ETM1(50%):LiQ(50%)* |
| | *5 nm* | *140 nm* | *5 nm* | *20 nm* | *20 nm* | *30 nm* |
| *E9* | *HIL1* | *HIL2* | *HIL1* | *(**1-15**)* | *H1(95%):SEB1(5%)* | *ETM1(50%):LiQ(50%)* |
| | *5 nm* | *140 nm* | *5 nm* | *20 nm* | *20 nm* | *30 nm* |
| *E10* | *HIL1* | *HIL2* | *HIL1* | *(**6-3**)* | *H1(95%):SEB1(5%)* | *ETM1(50%):LiQ(50%)* |
| | *5 nm* | *140 nm* | *5 nm* | *20 nm* | *20 nm* | *30 nm* |
| *E11* | *HIL1* | *HIL2* | *HIL1* | *(**6-2**)* | *H1(95%):SEB1(5%)* | *ETM1(50%):LiQ(50%)* |
| | *5 nm* | *140 nm* | *5 nm* | *20 nm* | *20 nm* | *30 nm* |
| *E12* | *HIL1* | *HIL2* | *HIL1* | *(**6-1**)* | *H1(95%):SEB1(5%)* | *ETM1(50%):LiQ(50%)* |
| | *5 nm* | *140 nm* | *5 nm* | *20 nm* | *20 nm* | *30 nm* |
| *E13* | *HIL1* | *HIL2* | *HIL1* | *(**6-4**)* | *H1(95%):SEB1(5%)* | *ETM1(50%):LiQ(50%)* |
| | *5 nm* | *140 nm* | *5 nm* | *20 nm* | *20 nm* | *30 nm* |
| *E14* | *HIL1* | *HIL2* | *HIL1* | *(**6-5**)* | *H1(95%):SEB1(5%)* | *ETM1(50%):LiQ(50%)* |
| | *5 nm* | *140 nm* | *5 nm* | *20 nm* | *20 nm* | *30 nm* |
| *E15* | *HIL1* | *HIL2* | *HIL1* | *(**8-1**)* | *H1(95%):SEB1(5%)* | *ETM1(50%):LiQ(50%)* |
| | *5 nm* | *140 nm* | *5 nm* | *20 nm* | *20 nm* | *30 nm* |
| *E16* | *HIL1* | *HIL2* | *HIL1* | *(**7-1**)* | *H1(95%):SEB1(5%)* | *ETM1(50%):LiQ(50%)* |
| | *5 nm* | *140 nm* | *5 nm* | *20 nm* | *20 nm* | *30 nm* |
| *E17* | *HIL1* | *HIL2* | *HIL1* | *(**7-2**)* | *H1(95%):SEB1(5%)* | *ETM1(50%):LiQ(50%)* |
| | *5 nm* | *140 nm* | *5 nm* | *20 nm* | *20 nm* | *30 nm* |
| *E18* | *HIL1* | *HIL2* | *HIL1* | *(**9-2**)* | *H1(95%):SEB1(5%)* | *ETM1(50%):LiQ(50%)* |
| | *5 nm* | *140 nm* | *5 nm* | *20 nm* | *20 nm* | *30 nm* |
| *E19* | *HIL1* | *HIL2* | *HIL1* | *(**2-7**)* | *H1(95%):SEB1(5%)* | *ETM1(50%):LiQ(50%)* |
| | *5 nm* | *140 nm* | *5 nm* | *20 nm* | *20 nm* | *30 nm* |
| *E20* | *HIL1* | *HIL2* | *HIL1* | *(**2-8**)* | *H1(95%):SEB1(5%)* | *ETM1(50%):LiQ(50%)* |
| | *5 nm* | *140 nm* | *5 nm* | *20 nm* | *20 nm* | *30 nm* |
| *E21* | *HIL1* | *HIL2* | *HIL1* | *(**2-10**)* | *H1(95%):SEB1(5%)* | *ETM1(50%):LiQ(50%)* |
| | *5 nm* | *140 nm* | *5 nm* | *20 nm* | *20 nm* | *30 nm* |
| *E22* | *HIL1* | *HIL2* | *HIL1* | *(**2-9**)* | *H1(95%):SEB1(5%)* | *ETM1(50%):LiQ(50%)* |
| | *5 nm* | *140 nm* | *5 nm* | *20 nm* | *20 nm* | *30 nm* |
| *E23* | *HIL1* | *HIL2* | *HIL1* | *(**1-17**)* | *H1(95%):SEB1(5%)* | *ETM1(50%):LiQ(50%)* |
| | *5 nm* | *140 nm* | *5 nm* | *20 nm* | *20 nm* | *30 nm* |

| **Tabelle 2: Daten der OLEDs** | | | | |
|---|---|---|---|---|
| ***Bsp.*** | ***EQE @ 1000 cd*/*m2*** | ***LD80 @ 6000 cd*/*m²*** | ***CIE*** | |
| | *%* | *[h]* | *X* | y |
| *V1* | *4.8* | *70* | 0.14 | 0.17 |
| *V2* | *6.8* | *160* | 0.14 | 0.14 |
| *V3* | *6.9* | *115* | 0.14 | 0.14 |
| *V4* | *6.8* | *115* | 0.14 | 0.14 |
| *V5* | *6.5* | *130* | 0.14 | 0.15 |
| *V6* | *6.6* | *100* | 0.14 | 0.14 |
| *V7* | *6.9* | *135* | 0.13 | 0.14 |
| *E1* | *7.0* | *180* | 0.14 | 0.15 |
| *E2* | *6.9* | *175* | 0.13 | 0.15 |
| *E3* | *7.0* | *165* | 0.13 | 0.15 |
| *E4* | *6.7* | *150* | 0.14 | 0.15 |
| *E5* | *6.9* | *170* | 0.14 | 0.13 |
| *E6* | *7.0* | *145* | 0.14 | 0.14 |
| *E7* | *7.0* | *155* | 0.14 | 0.14 |
| *E8* | *7.8* | *120* | 0.14 | 0.14 |
| *E9* | *6.9* | *135* | 0.13 | 0.14 |
| *E10* | *6.9* | *150* | 0.14 | 0.14 |
| *E11* | *7.0* | *135* | 0.14 | 0.13 |
| *E12* | *7.0* | *180* | 0.14 | 0.15 |
| *E13* | *7.0* | *150* | 0.14 | 0.14 |
| *E14* | *7.2* | *170* | 0.14 | 0.14 |
| *E15* | *7.0* | *150* | 0.14 | 0.14 |
| *E16* | *6.9* | *160* | 0.14 | 0.14 |
| *E17* | *6.9* | *155* | 0.14 | 0.15 |
| *E18* | *6.9* | *170* | 0.14 | 0.14 |
| *E19* | *6.9* | *135* | 0.14 | 0.14 |
| *E20* | *7.0* | *115* | 0.14 | 0.14 |
| *E21* | *7.0* | *150* | 0.14 | 0.14 |
| *E22* | *7.0* | *135* | 0.14 | 0.14 |
| *E23* | *7.0* | *140* | 0.14 | 0.14 |

| **Tabelle 3: Aufbau der OLEDs** (Schichtaufbau: Substrat/HTL/HIL2/EBL/EML/ETL/Kathode) | | | | | |
|---|---|---|---|---|---|
| ***Bsp.*** | ***HTL*** | ***HIL2*** | ***EBL*** | ***EML*** | ***ETL*** |
| | *Dicke* / *nm* | *Dicke* / *nm* | *Dicke* / *nm* | *Dicke* / *nm* | *Dicke* / *nm* |
| *V8* | *HIL2* | *HIL1* | *NPB* | *H2(88%): Irpy(12%)* | *ETM1(50%):LiQ(50%)* |
| | *70 nm* | *5 nm* | *20 nm* | *30 nm* | *40 nm* |
| *V9* | *HIL2* | *HIL1* | *HTMV1* | *H2(88%): Irpy(12%)* | *ETM1(50%):LiQ(50%)* |
| | *70 nm* | *5 nm* | *20 nm* | *30 nm* | *40 nm* |
| *V10* | *HIL2* | *HIL1* | *HTMV2* | *H2(88%): Irpy(12%)* | *ETM1(50%):LiQ(50%)* |
| | *70 nm* | *5 nm* | *20 nm* | *30 nm* | *40 nm* |
| *V11* | *HIL2* | *HIL1* | *HTMV3* | *H2(88%): Irpy(12%)* | *ETM1(50%):LiQ(50%)* |
| | *70 nm* | *5 nm* | *20 nm* | *30 nm* | *40 nm* |
| *V12* | *HIL2* | *HIL1* | *HTMV5* | *H2(88%): Irpy(12%)* | *ETM1(50%):LiQ(50%)* |
| | *70 nm* | *5 nm* | *20 nm* | *30 nm* | *40 nm* |
| *V13* | *HIL2* | *HIL1* | *HTMV6* | *H2(88%): Irpy(12%)* | *ETM1(50%):LiQ(50%)* |
| | *70 nm* | *5 nm* | *20 nm* | *30 nm* | *40 nm* |
| *E24* | *HIL2* | *HIL1* | *(**1-1**)* | *H2(88%): Irpy(12%)* | *ETM1(50%):LiQ(50%)* |
| | *70 nm* | *5 nm* | *20 nm* | *30 nm* | *40 nm* |
| *E25* | *HIL2* | *HIL1* | *(**1-4**)* | *H2(88%): Irpy(12%)* | *ETM1(50%):LiQ(50%)* |
| | *70 nm* | *5 nm* | *20 nm* | *30 nm* | *40 nm* |
| *E26* | *HIL2* | *HIL1* | *(**1-7**)* | *H2(88%): Irpy(12%)* | *ETM1(50%):LiQ(50%)* |
| | *70 nm* | *5 nm* | *20 nm* | *30 nm* | *40 nm* |
| *E27* | *HIL2* | *HIL1* | *(**5-1**)* | *H2(88%): Irpy(12%)* | *ETM1(50%):LiQ(50%)* |
| | *70 nm* | *5 nm* | *20 nm* | *30 nm* | *40 nm* |
| *E28* | *HIL2* | *HIL1* | *(**4-1**)* | *H2(88%): Irpy(12%)* | *ETM1(50%):LiQ(50%)* |
| | *70 nm* | *5 nm* | *20 nm* | *30 nm* | *40 nm* |
| *E29* | *HIL2* | *HIL1* | *(**1-12**)* | *H2(88%): Irpy(12%)* | *ETM1(50%):LiQ(50%)* |
| | *70 nm* | *5 nm* | *20 nm* | *30 nm* | *40 nm* |
| *E30* | *HIL2* | *HIL1* | *(**1-13**)* | *H2(88%): Irpy(12%)* | *ETM1(50%):LiQ(50%)* |
| | *70 nm* | *5 nm* | *20 nm* | *30 nm* | *40 nm* |
| *E31* | *HIL2* | *HIL1* | *(**1-14**)* | *H2(88%): Irpy(12%)* | *ETM1(50%):LiQ(50%)* |
| | *70 nm* | *5 nm* | *20 nm* | *30 nm* | *40 nm* |
| *E32* | *HIL2* | *HIL1* | *(**1-3**)* | *H2(88%): Irpy(12%)* | *ETM1(50%):LiQ(50%)* |
| | *70 nm* | *5 nm* | *20 nm* | *30 nm* | *40 nm* |
| *E33* | *HIL2* | *HIL1* | *(**1-15**)* | *H2(88%): Irpy(12%)* | *ETM1(50%):LiQ(50%)* |
| | *70 nm* | *5 nm* | *20 nm* | *30 nm* | *40 nm* |
| *E34* | *HIL2* | *HIL1* | *(**6-2**)* | *H2(88%): Irpy(12%)* | *ETM1(50%):LiQ(50%)* |
| | *70 nm* | *5 nm* | *20 nm* | *30 nm* | *40 nm* |
| *E35* | *HIL2* | *HIL1* | *(**6-1**)* | *H2(88%): Irpy(12%)* | *ETM1(50%):LiQ(50%)* |
| | *70 nm* | *5 nm* | *20 nm* | *30 nm* | *40 nm* |
| *E36* | *HIL2* | *HIL1* | *(**6-4**)* | *H2(88%): Irpy(12%)* | *ETM1(50%):LiQ(50%)* |
| | *70 nm* | *5 nm* | *20 nm* | *30 nm* | *40 nm* |
| *E37* | *HIL2* | *HIL1* | *(**7-2**)* | *H2(88%): Irpy(12%)* | *ETM1(50%):LiQ(50%)* |
| | *70 nm* | *5 nm* | *20 nm* | *30 nm* | *40 nm* |
| *E38* | *HIL2* | *HIL1* | *(**9-2**)* | *H2(88%): Irpy(12%)* | *ETM1(50%):LiQ(50%)* |
| | *70 nm* | *5 nm* | *20 nm* | *30 nm* | *40 nm* |
| *E39* | *HIL2* | *HIL1* | *(**2-7**)* | *H2(88%): Irpy(12%)* | *ETM1(50%):LiQ(50%)* |
| | *70 nm* | *5 nm* | *20 nm* | *30 nm* | *40 nm* |
| *E40* | *HIL2* | *HIL1* | *(**2-8**)* | *H2(88%): Irpy(12%)* | *ETM1(50%):LiQ(50%)* |
| | *70 nm* | *5 nm* | *20 nm* | *30 nm* | *40 nm* |
| *E41* | *HIL2* | *HIL1* | *(**2-10**)* | *H2(88%): Irpy(12%)* | *ETM1(50%):LiQ(50%)* |
| | *70 nm* | *5 nm* | *20 nm* | *30 nm* | *40 nm* |
| *E42* | *HIL2* | *HIL1* | *(**2-9**)* | *H2(88%): Irpy(12%)* | *ETM1(50%):LiQ(50%)* |
| | *70 nm* | *5 nm* | *20 nm* | *30 nm* | *40 nm* |
| *E43* | *HIL2* | *HIL1* | *(**1-17**)* | *H2(88%): Irpy(12%)* | *ETM1(50%):LiQ(50%)* |
| | *70 nm* | *5 nm* | *20 nm* | *30 nm* | *40 nm* |

| **Tabelle 4: Daten der OLEDs** | | | | |
|---|---|---|---|---|
| ***Bsp.*** | ***Effizienz* @ *1000 cd*/*m2*** | ***LD80 @ 8000 cd*/*m²*** | ***CIE*** | |
| | *%* | *[h]* | *x* | Y |
| *V8* | *13.4* | 85 | 0.36 | 0.61 |
| *V9* | *16.3* | *140* | 0.35 | 0.62 |
| *V10* | *16.0* | *130* | 0.36 | 0.61 |
| *V11* | *16.7* | *155* | 0.36 | 0.61 |
| *V12* | *16.4* | *150* | 0.37 | 0.60 |
| *V13* | *17.0* | *170* | 0.35 | 0.62 |
| *E24* | *17.2* | *210* | 0.35 | 0.61 |
| *E25* | *17.2* | *200* | 0.36 | 0.61 |
| *E26* | *17.5* | *190* | 0.36 | 0.61 |
| *E27* | *16.7* | *190* | 0.37 | 0.60 |
| *E28* | *17.4* | *200* | 0.35 | 0.61 |
| *E29* | *17.0* | *180* | 0.37 | 0.61 |
| *E30* | *17.0* | *180* | 0.37 | 0.61 |
| *E31* | *17.5* | *220* | 0.37 | 0.61 |
| *E32* | *17.3* | *170* | 0.37 | 0.61 |
| *E33* | *17.2* | *200* | 0.37 | 0.61 |
| *E34* | *17.3* | *210* | 0.37 | 0.61 |
| *E35* | *17.2* | *220* | 0.37 | 0.61 |
| *E36* | *17.2* | *190* | 0.37 | 0.61 |
| *E37* | *17.2* | *200* | 0.37 | 0.61 |
| *E38* | *16.9* | *220* | 0.37 | 0.61 |
| *E39* | *16.9* | *160* | 0.37 | 0.61 |
| *E40* | *170* | *170* | 0.37 | 0.61 |
| *E41* | *17.0* | *195* | 0.37 | 0.61 |
| *E42* | *17.0* | *180* | 0.37 | 0.61 |
| *E43* | *17.1* | *190* | 0.37 | 0.61 |

## Patentansprüche

1. Verbindung der allgemeinen Formel (1) 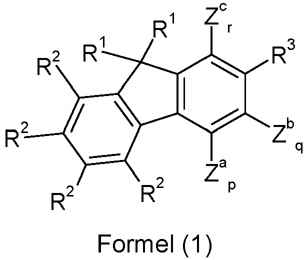 wobei für die verwendeten Symbole und Indices gilt:
R¹ ist, bei jedem Auftreten gleich oder verschieden, H, D, F, Cl, Br, I, C(=O)R⁴, CN, Si(R⁴)₃, NO₂, P(=O)(R⁴)₂, S(=O)R⁴, S(=O)₂R⁴, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁴ substituiert sein können und wobei eine oder mehrere CH₂-Gruppen in den oben genannten Gruppen durch -R⁴C=CR⁴-, -C=C-, Si(R⁴)₂, C=O, C=S, C=NR⁴, -C(=O)O-, -C(=O)NR⁴-, P(=O)(R⁴), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches oder heteroaromatisches Ringsystem mit 6 bis 30 aromatischen Ringatomen, das jeweils durch einen oder mehrere Reste R⁴ substituiert sein kann, oder eine Aryloxygruppe mit 5 bis 60 aromatischen Ringatomen, die mit einem oder mehreren Resten R⁴ substituiert sein kann, oder einer Aralkylgruppe mit 5 bis 60 aromatischen Ringatomen, die jeweils mit einem oder mehreren Resten R⁴ substituiert sein kann, wobei die beiden Reste R¹ miteinander verknüpft sein können und einen Ring bilden können, so dass eine Spiroverbindung in Position 9 des Fluorens entsteht, wobei Spirobifluorene ausgeschlossen sind;
R², R³ sind bei jedem Auftreten gleich oder verschieden, bevorzugt gleich, H, D, F, Cl, Br, I, C(=O)R⁴, CN, Si(R⁴)₃, NO₂, P(=O)(R⁴)₂, S(=O)R⁴, S(=O)₂R⁴, N(R⁴)₂, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁴ substituiert sein können und wobei eine oder mehrere CH₂-Gruppen in den oben genannten Gruppen durch -R⁴C=CR⁴-, -C=C-, Si(R⁴)₂, C=O, C=S, C=NR⁴, -C(=O)O-, -C(=O)NR⁴-, P(=O)(R⁴), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches Ringsystem mit 6 bis 30 aromatischen Ringatomen, das jeweils durch einen oder mehrere Reste R⁴ substituiert sein kann;
R⁴ ist bei jedem Auftreten gleich oder verschieden H, D, F, Cl, Br, I, C(=O)R⁵, CN, Si(R⁵)₃, NO₂, P(=O)(R⁵)₂, S(=O)R⁵, S(=O)₂R⁵, , N(R⁵)₂, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁵ substituiert sein können und wobei eine oder mehrere CH₂-Gruppen in den oben genannten Gruppen durch - R⁵C=CR⁵-, -C≡C-, Si(R⁵)₂, C=O, C=S, C=NR⁵, -C(=O)O-, -C(=O)NR⁵-, P(=O)(R⁵), -O-, -S-, SO oder SO₂ ersetzt sein können und wobei ein oder mehrere H-Atome in den oben genannten Gruppen durch D, F, Cl, Br, I, CN oder NO₂ ersetzt sein können, oder ein aromatisches Ringsystem mit 5 bis 30 aromatischen Ringatomen, das jeweils durch einen oder mehrere Reste R⁵ substituiert sein kann;
R⁵ ist ausgewählt aus der Gruppe bestehend aus H, D, F, einem aliphatischem Kohlenwasserstoffrest mit 1 bis 20 C-Atomen, in dem ein oder mehrere H-Atome durch D oder F ersetzt sein können, wobei zwei oder mehr benachbarte Substituenten R⁵ miteinander ein mono- oder polycyclisches, aliphatisches, Ringsystem bilden können;
r ist 1;
p, q sind 0;
Z^{a}₀, Z^{b}₀ sind gleich oder verschieden bei jedem Auftreten gleich R³
Z^{c}₁ ist gleich 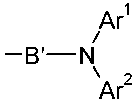
B' ist eine Einfachbindung, eine Arylgruppe mit 6 bis 30 Ringatomen oder eine mono- oder bicyclische Heteroarylgruppe mit 5 bis 30 Ringatomen, die jeweils mit einem oder mehreren Resten R⁴ substituiert sein können, wobei, wenn B' eine Einfachbindung ist, das Stickstoffatom direkt an das Fluoren gebunden ist;
Ar¹, Ar² sind bei jedem Auftreten gleich oder verschieden, ausgewählt aus den folgenden Gruppen der Formeln (37) bis (66), (69) bis (72) und (76) bis (96), die mit einem oder mehreren Resten R⁶ substituiert sein können 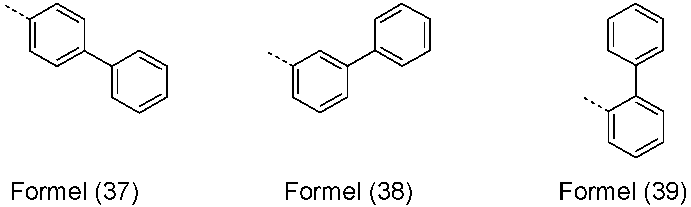 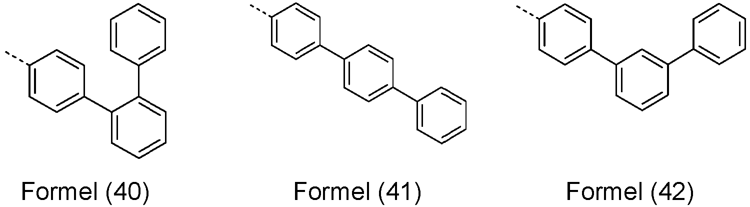 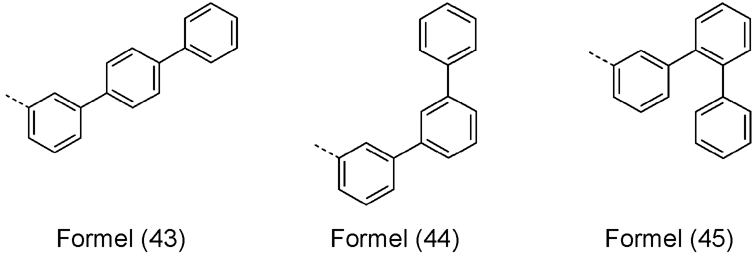 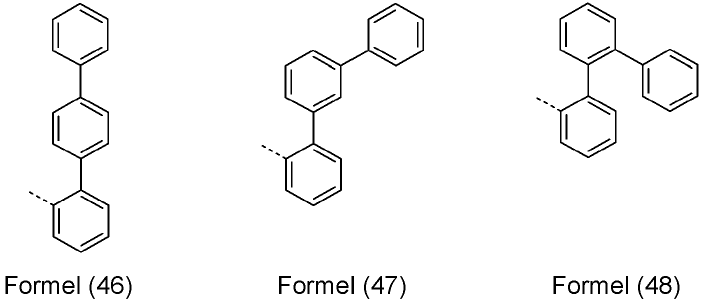 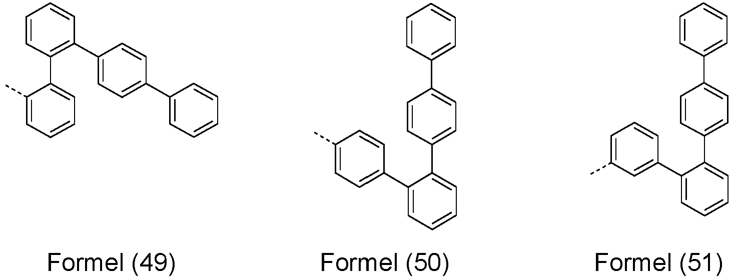 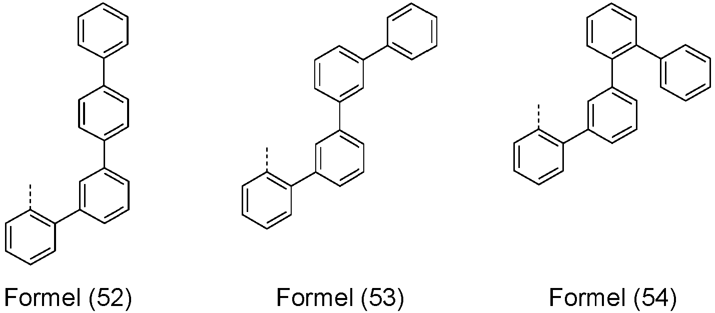 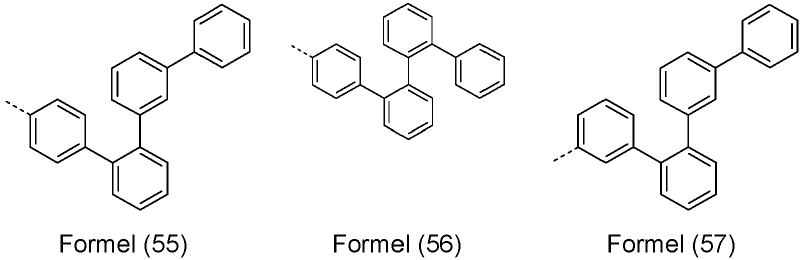 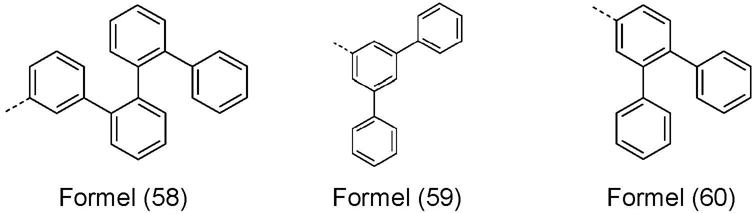 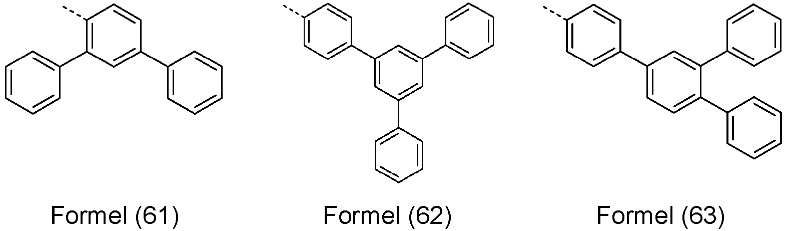 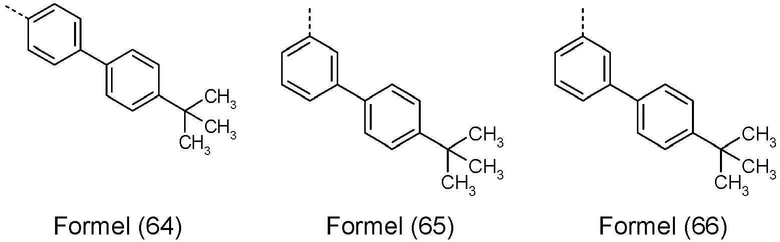 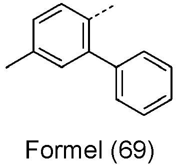 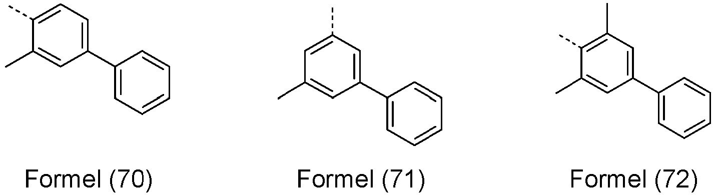 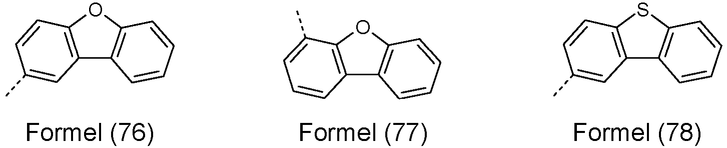 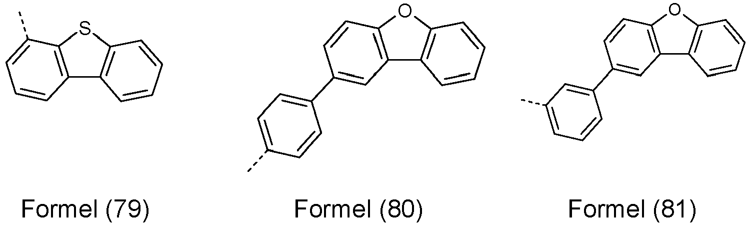 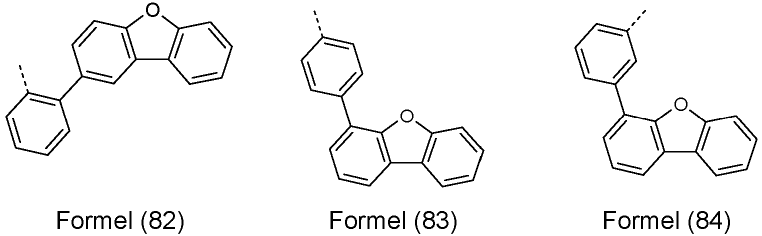 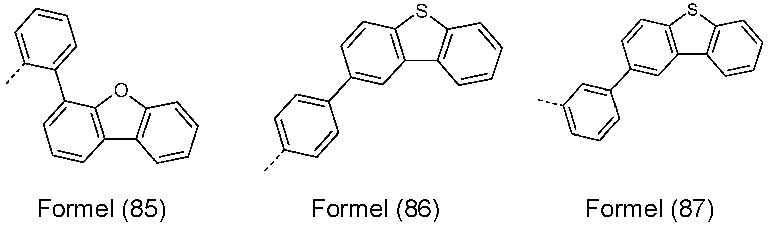 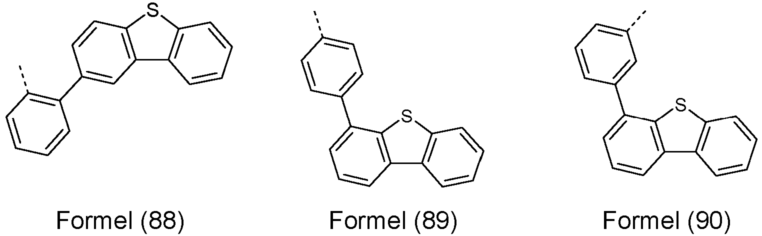 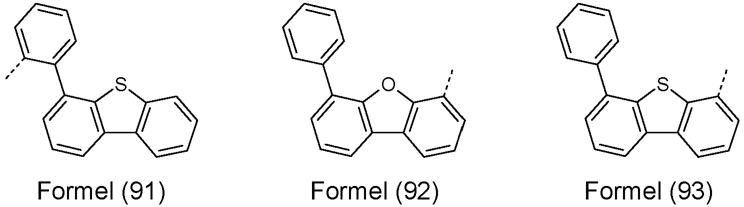 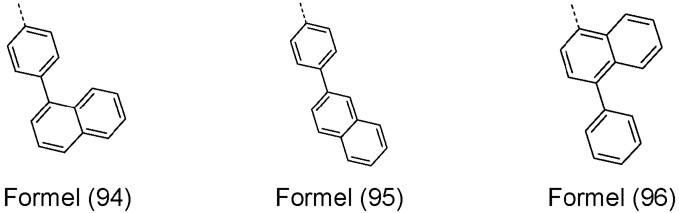
R⁶ ist bei jedem Auftreten gleich oder verschieden H, D, F, Cl, Br, I, C(=O)R⁵, CN, Si(R⁵)₃, NO₂, P(=O)(R⁵)₂, S(=O)R⁵, S(=O)₂R⁵, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen oder eine Alkenyl- oder Alkinylgruppe mit 2 bis 20 C-Atomen, wobei die oben genannten Gruppen jeweils mit einem oder mehreren Resten R⁵ substituiert sein können und wobei eine oder mehrere CH₂-Gruppen in den oben genannten Gruppen durch -R⁵C=CR⁵-, -C≡C-, Si(R⁵)₂, C=O, C=S, C=NR⁵, -C(=O)O-, -C(=O)NR⁵-, P(=O)(R⁵), -O-, -S-, SO oder SO₂ ersetzt sein können und wobei ein oder mehrere H-Atome in den oben genannten Gruppen durch D, F, Cl, Br, I, CN oder NO₂ ersetzt sein können.

2. Verbindung gemäß Anspruch 1, **dadurch gekennzeichnet, dass** sie die allgemeine Formel (5) hat 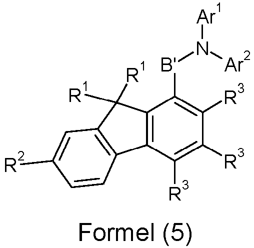 wobei für die verwendeten Symbole die Definitionen aus Anspruch 1 gelten.

3. Verbindung gemäß Anspruch 1 oder 2, **dadurch gekennzeichnet, dass** sie die allgemeine Formel (8) hat 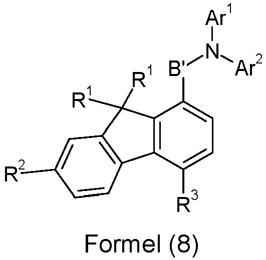 wobei für die verwendeten Symbole die Definitionen aus Anspruch 1 gelten.

4. Verbindung gemäß einem oder mehreren der Ansprüche 1 bis 3, **dadurch gekennzeichnet, dass** beide R¹ identisch sind.

5. Verbindung gemäß einem oder mehreren der Ansprüche 1 bis 4, **dadurch gekennzeichnet, dass** B' eine Einfachbindung oder eine Phenylen-, Biphenylen-, Terphenylen-, Naphthylen-, Pyridinylen-, Pyrimidinylen-, Pyrazinylen-, Pyridazinylen-, Triazinylen-, Dibenzofuranylen- oder Dibenzothiophenylengruppe ist, die mit einem oder mehreren Resten R⁴ substituiert sein kann.

6. Verbindung gemäß einem oder mehreren der Ansprüche 1 bis 5, **dadurch gekennzeichnet, dass** B' eine Einfachbindung oder eine Phenylen-, Biphenylen-, Terphenylen-, Naphthylen-, Dibenzofuranylen- oder Dibenzothiophenylengruppe ist, die mit einem oder mehreren Resten R⁴ substituiert sein kann.

7. Verbindung gemäß einem oder mehreren der Ansprüche 1 bis 6, **dadurch gekennzeichnet, dass** die Verbindung ausgewählt ist aus einer der folgenden Verbindungen

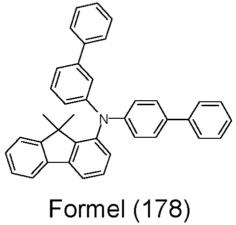

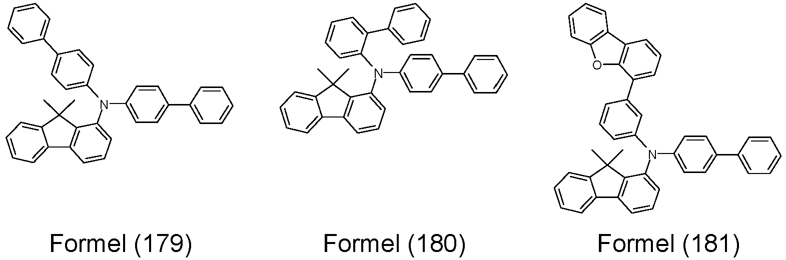

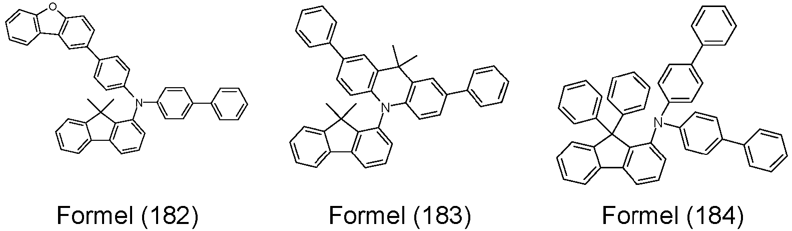

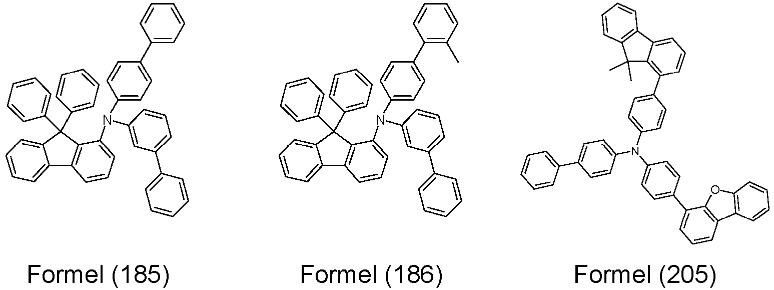

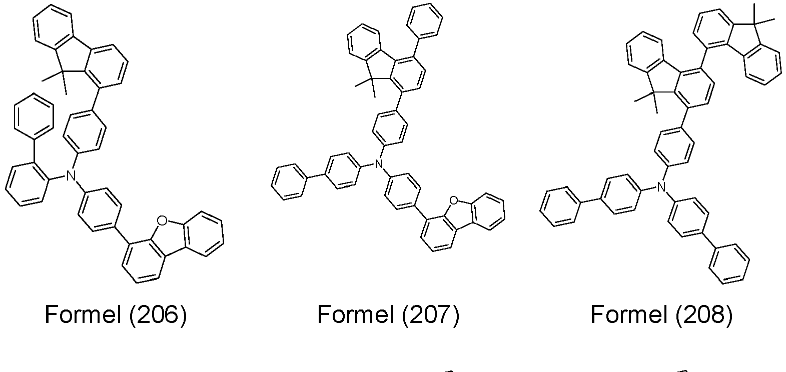

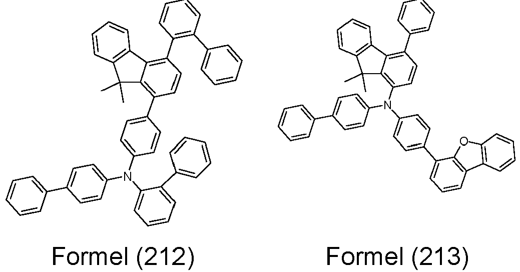

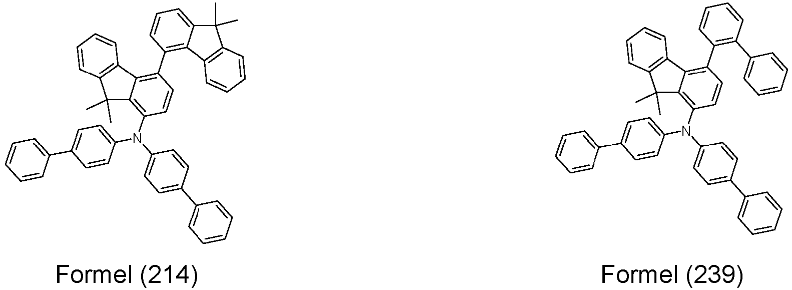

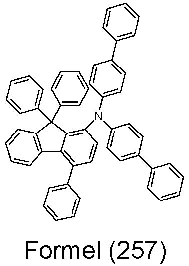

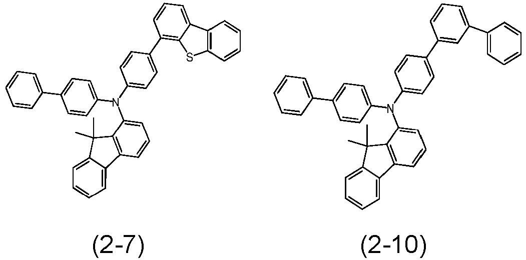

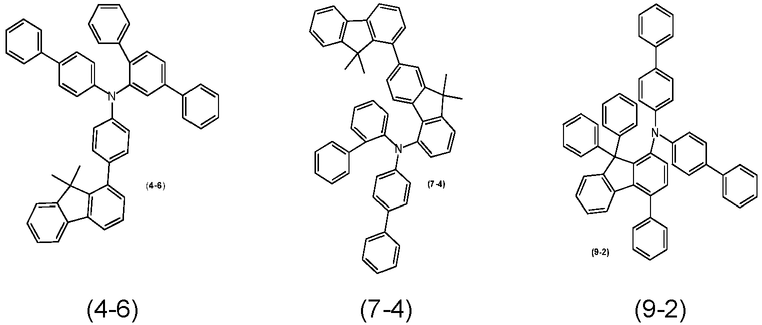

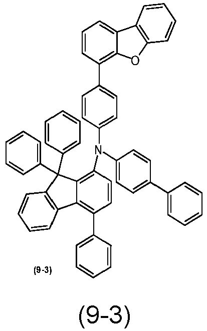

8. Verbindung gemäß einem oder mehreren der Ansprüche 1 bis 7, **dadurch gekennzeichnet, dass** B' eine Einfachbindung ist.

9. Verbindung gemäß einem oder mehreren der Ansprüche 1 bis 8, **dadurch gekennzeichnet, dass** es sich um eine Monoaminverbindung handelt.

10. Verfahren zur Herstellung einer Verbindung nach einem oder mehreren der Ansprüche 1 bis 9 mittels einstufiger Buchwald Kupplung durch Umsetzung eines Fluorenderivats, das eine Abgangsgruppe enthält, mit Ar²-NH-Ar¹.

11. Verfahren zur Herstellung einer Verbindung gemäß einem oder mehreren der Ansprüche 1 bis 9 mittels zweistufiger Buchwald Kupplung durch stufenweise Umsetzung eines Fluorenderivats, das eine Abgangsgruppe enthält, mit (1) Ar²-NH₂ und (2) NH₂-Ar¹.

12. Verfahren zur Herstellung einer Verbindung gemäß einem oder mehrere der Ansprüche 1 bis 9, **dadurch gekennzeichnet, dass** die Verbindung aus einem Benzochromen-6-on hergestellt wird.

13. Verfahren gemäß Anspruch 12 umfassend die folgenden Schritte:
a) Addition einer organometallischen Verbindung an ein Benzochromen-6-on und anschließende
b) säurekatalysierte Cyclisierung zur einem 4-Hydroxy-Fluorenderivat und anschließende
c) Umwandlung der Hydroxygruppe in Position 4 des Fluorens in eine Abgangsgruppe und anschließende
d) Umsetzung des Fluorens in das gewünschte Produkt.

14. Oligomer, Polymer oder Dendrimer enthaltend eine oder mehrere Verbindungen gemäß einem oder mehreren der Ansprüche 1 bis 9, wobei die Bindung(en) zum Polymer, Oligomer oder Dendrimer an beliebigen, in Formel (1) mit R¹ bis R⁶ substituierten Positionen lokalisiert sein können.

15. Zusammensetzung enthaltend eine oder mehrere Verbindung gemäß einem oder mehreren der Ansprüche 1 bis 9 oder ein oder mehrere Polymerre, Oligomere oder Dendrimere gemäß Anspruch 14 sowie wenigstens ein weiteres organisch funktionelles Material ausgewählt aus der Gruppe bestehend aus fluoreszierenden Emitter, phosphoreszierenden Emitter, Host Materialien, Matrix Materialien, Elektronentransportmaterialien, Elektroneninjektionsmaterialien, Lochleitermaterialien, Lochinjektionsmaterialien, Elektronenblockiermaterialien und Lochblockiermaterialien.

16. Formulierung enthaltend mindestens eine Verbindung gemäß einem oder mehreren der Ansprüche 1 bis 9 oder mindestens ein Polymer, Oligomer oder Dendrimer gemäß Anspruch 14 oder mindestens eine Zusammensetzung nach Anspruch 15 sowie mindestens ein Lösungsmittel.

17. Elektronische Vorrichtung, enthaltend mindestens eine Verbindung gemäß einem oder mehreren der Ansprüche 1 bis 9 oder mindestens ein Polymer, Oligomer oder Dendrimer gemäß Anspruch 14 oder mindestens eine Zusammensetzung nach Anspruch 15.

18. Elektronische Vorrichtung gemäß Anspruch 17, **dadurch gekennzeichnet, dass** sie ausgewählt ist aus organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und organischen Elektrolumineszenzvorrichtungen (OLEDs).

19. Elektronische Vorrichtung gemäß Anspruch 17 oder 18, welche ausgewählt ist aus der Gruppe der organischen Elektrolumineszenzvorrichtungen, insbesondere eine organische lichtemittierende Diode (OLED), **dadurch gekennzeichnet, dass** die Verbindung gemäß einem oder mehreren der Ansprüche 1 bis 9 oder das Polymer, Oligomer oder Dendrimer gemäß Anspruch 14 oder eine Zusammensetzung nach Anspruch 15 in einer oder mehreren der folgenden Funktionen eingesetzt wird:
- als Lochtransportmaterial in einer Lochtransport- oder Lochinjektionsschicht,
- als Matrixmaterial in einer emittierenden Schicht,
- als Elektronenblockiermaterial,
- als Excitonenblockiermaterial
wobei die Verwendung als als Lochtransportmaterial in einer Lochtransport- oder Lochinjektionsschicht bevorzugt ist.

## Claims

1. Compound of the general formula (1) 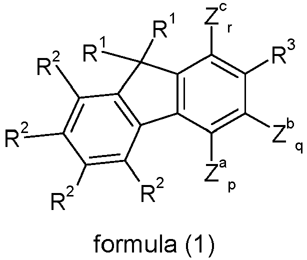 where the following applies to the symbols and indices used:
R¹ is on each occurrence, identically or differently, H, D, F, Cl, Br, I, C(=O)R⁴, CN, Si(R⁴)₃, NO₂, P(=O)(R⁴)₂, S(=O)R⁴, S(=O)₂R⁴, a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the above-mentioned groups may in each case be substituted by one or more radicals R⁴ and where one or more CH₂ groups in the above-mentioned groups may be replaced by -R⁴C=CR⁴-, -C≡C-, Si(R⁴)₂, C=O, C=S, C=NR⁴, -C(=O)O-, -C(=O)NR⁴-, P(=O)(R⁴), -O-, -S-, SO or SO₂ and where one or more H atoms in the above-mentioned groups may be replaced by D, F, Cl, Br, I, CN or NO₂, or an aromatic or heteroaromatic ring system having 6 to 30 aromatic ring atoms, which may in each case be substituted by one or more radicals R⁴, or an aryloxy group having 5 to 60 aromatic ring atoms, which may be substituted by one or more radicals R⁴, or an aralkyl group having 5 to 60 aromatic ring atoms, which may in each case be substituted by one or more radicals R⁴, where the two radicals R¹ may be linked to one another and may form a ring, so that a spiro compound forms in position 9 of the fluorene, where spirobifluorenes are excluded;
R², R³ are on each occurrence, identically or differently, preferably identically, H, D, F, Cl, Br, I, C(=O)R⁴, CN, Si(R⁴)₃, NO₂, P(=O)(R⁴)₂, S(=O)R⁴, S(=O)₂R⁴, N(R⁴)₂, a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the above-mentioned groups may in each case be substituted by one or more radicals R⁴ and where one or more CH₂ groups in the above-mentioned groups may be replaced by -R⁴C=CR⁴-, -C≡C-, Si(R⁴)₂, C=O, C=S, C=NR⁴, -C(=O)O-, -C(=O)NR⁴-, P(=O)(R⁴), -O-, -S-, SO or SO₂ and where one or more H atoms in the above-mentioned groups may be replaced by D, F, Cl, Br, I, CN or NO₂, or an aromatic ring system having 6 to 30 aromatic ring atoms, which may in each case be substituted by one or more radicals R⁴;
R⁴ is on each occurrence, identically or differently, H, D, F, Cl, Br, I, C(=O)R⁵, CN, Si(R⁵)₃, NO₂, P(=O)(R⁵)₂, S(=O)R⁵, S(=O)₂R⁵, N(R⁵)₂, a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the above-mentioned groups may in each case be substituted by one or more radicals R⁵ and where one or more CH₂ groups in the above-mentioned groups may be replaced by -R⁵C=CR⁵-, -C≡C-, Si(R⁵)₂, C=O, C=S, C=NR⁵, -C(=O)O-, -C(=O)NR⁵-, P(=O)(R⁵), -O-, -S-, SO or SO₂ and where one or more H atoms in the above-mentioned groups may be replaced by D, F, Cl, Br, I, CN or NO₂, or an aromatic ring system having 5 to 30 aromatic ring atoms, which may in each case be substituted by one or more radicals R⁵;
R⁵ is selected from the group consisting of H, D, F, an aliphatic hydrocarbon radical having 1 to 20 C atoms, in which one or more H atoms may be replaced by D or F, where two or more adjacent substituents R⁵ may form a mono- or polycyclic, aliphatic ring system with one another;
r is 1;
p, q are 0;
Z^{a}₀, Z^{b}₀ are, identically or differently on each occurrence, equal to R³;
Z^{c}₁ is equal to 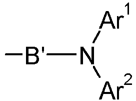
B' is a single bond, an aryl group having 6 to 30 ring atoms or a mono- or bicyclic heteroaryl group having 5 to 30 ring atoms, which may in each case be substituted by one or more radicals R⁴, where, if B' is a single bond, the nitrogen atom is bonded directly to the fluorene;
Ar¹, Ar² are selected on each occurrence, identically or differently, from the following groups of the formulae (37) to (66), (69) to (72) and (76) to (96), which may be substituted by one or more radicals R⁶, 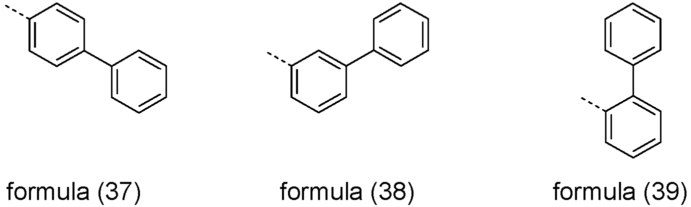 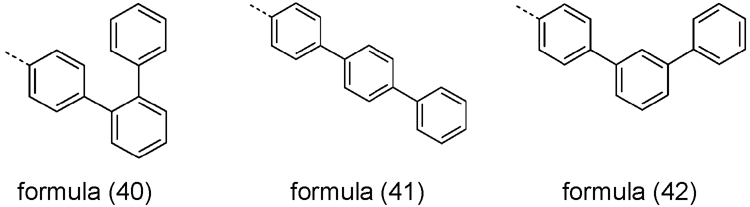 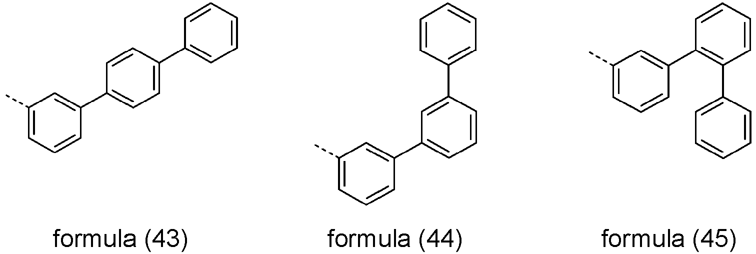 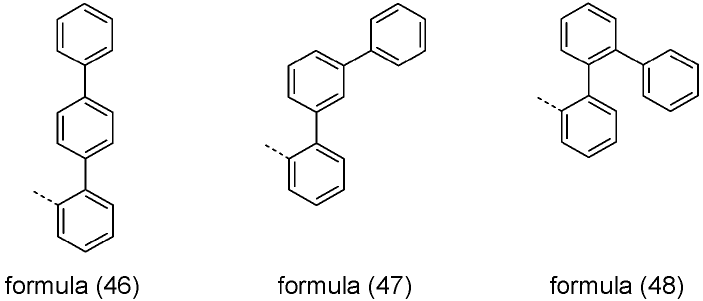 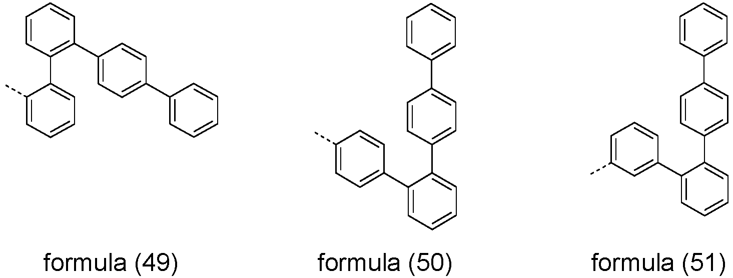 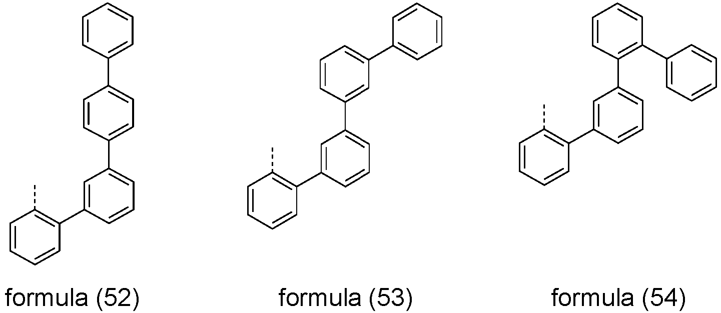 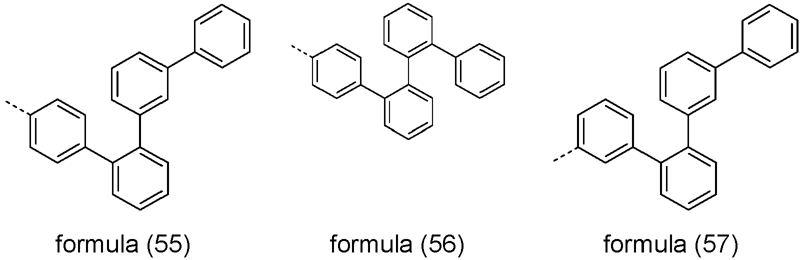 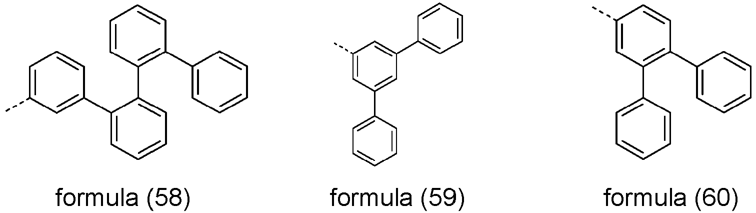 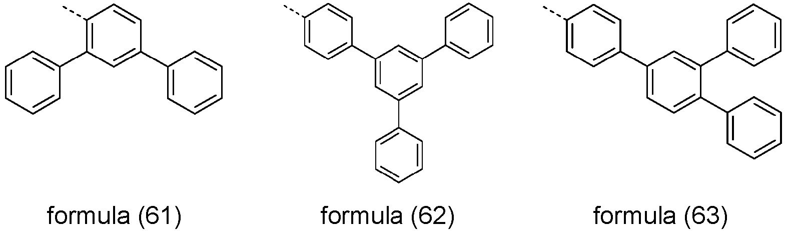 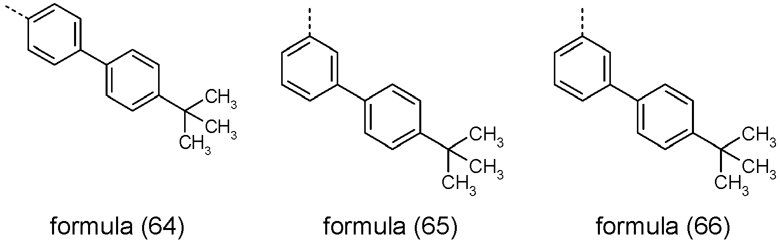 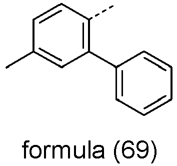 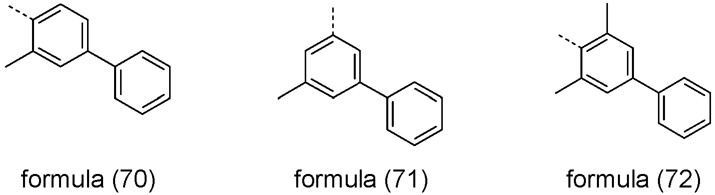 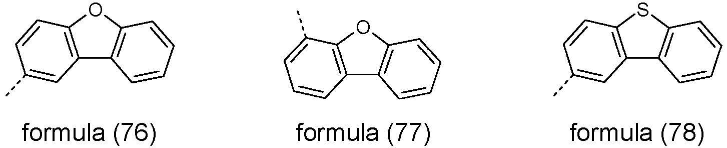 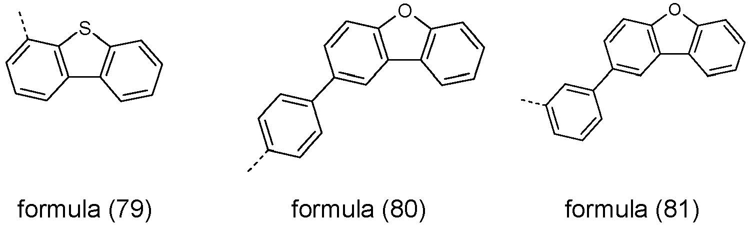 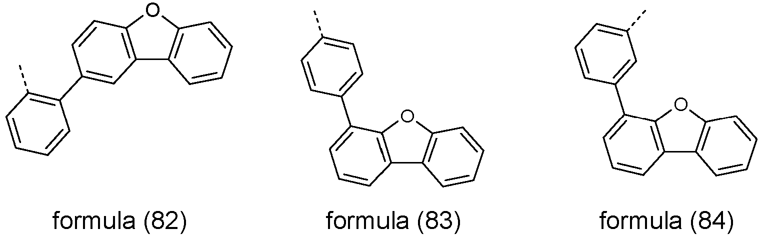 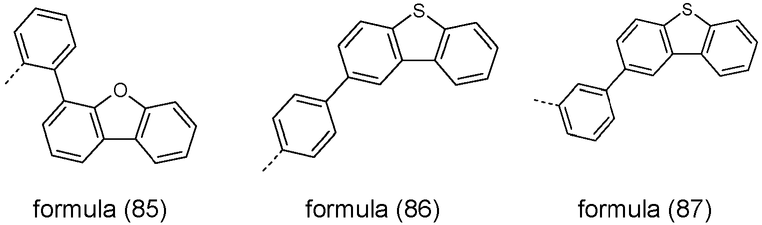 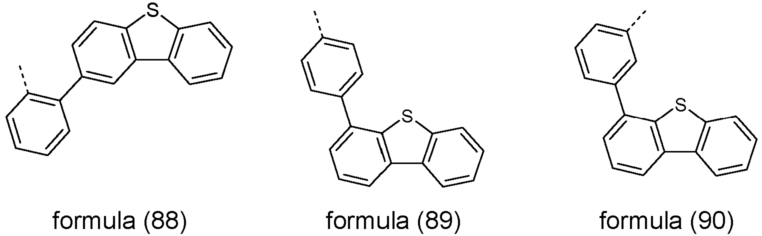 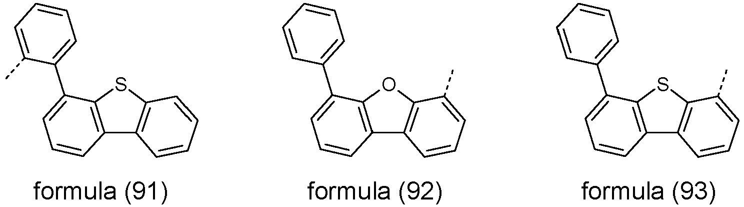 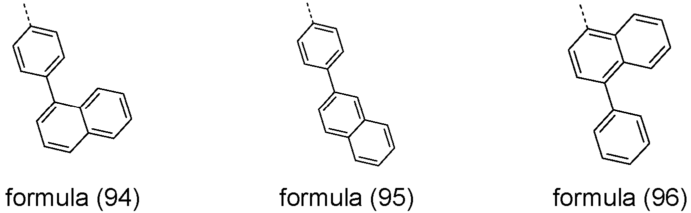
R⁶ is on each occurrence, identically or differently, H, D, F, Cl, Br, I, C(=O)R⁵, CN, Si(R⁵)₃, NO₂, P(=O)(R⁵)₂, S(=O)R⁵, S(=O)₂R⁵, a straight-chain alkyl, alkoxy or thioalkyl group having 1 to 20 C atoms or a branched or cyclic alkyl, alkoxy or thioalkyl group having 3 to 20 C atoms or an alkenyl or alkynyl group having 2 to 20 C atoms, where the above-mentioned groups may in each case be substituted by one or more radicals R⁵ and where one or more CH₂ groups in the above-mentioned groups may be replaced by -R⁵C=CR⁵-, -C≡C-, Si(R⁵)₂, C=O, C=S, C=NR⁵, -C(=O)O-, -C(=O)NR⁵-, P(=O)(R⁵), -O-, -S-, SO or SO₂ and where one or more H atoms in the above-mentioned groups may be replaced by D, F, Cl, Br, I, CN or NO₂.

2. Compound according to Claim 1, **characterised in that** it has the general formula (5) 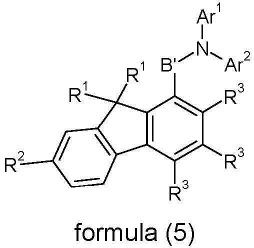 where the definitions from Claim 1 apply to the symbols used.

3. Compound according to Claim 1 or 2, **characterised in that** it has the general formula (8) 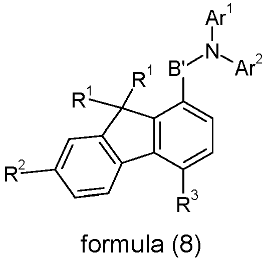 where the definitions from Claim 1 apply to the symbols used.

4. Compound according to one or more of Claims 1 to 3, **characterised in that** both R¹ are identical.

5. Compound according to one or more of Claims 1 to 4, **characterised in that** B' is a single bond or a phenylene, biphenylene, terphenylene, naphthylene, pyridinylene, pyrimidinylene, pyrazinylene, pyridazinylene, triazinylene, dibenzofuranylene or dibenzothiophenylene group, which may be substituted by one or more radicals R⁴.

6. Compound according to one or more of Claims 1 to 5, **characterised in that** B' is a single bond or a phenylene, biphenylene, terphenylene, naphthylene, dibenzofuranylene or dibenzothiophenylene group, which may be substituted by one or more radicals R⁴.

7. Compound according to one or more of Claims 1 to 6, **characterised in that** the compound is selected from one of the following compounds

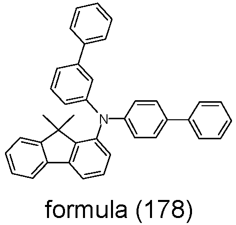

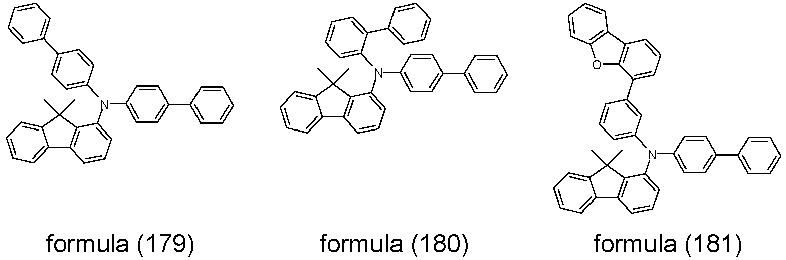

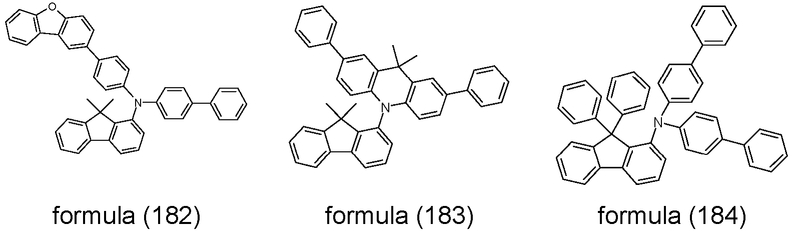

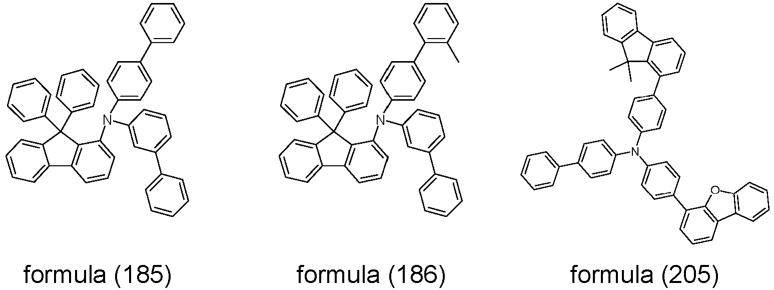

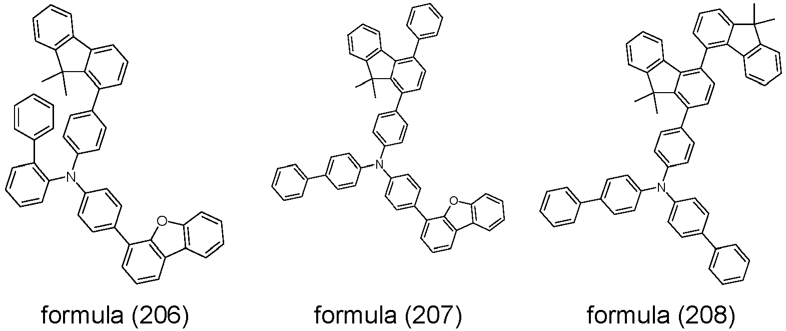

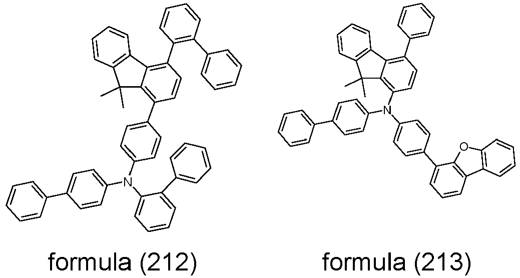

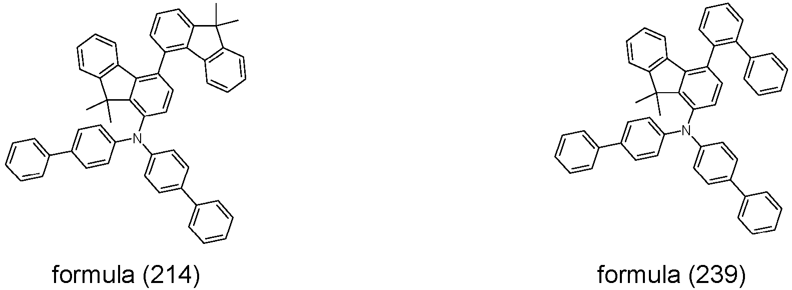

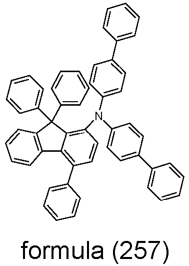

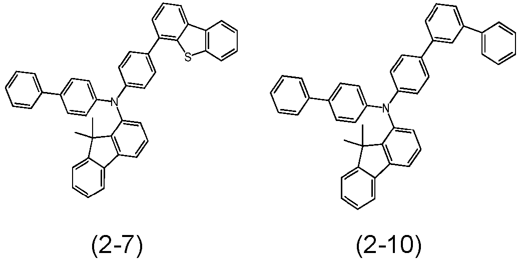

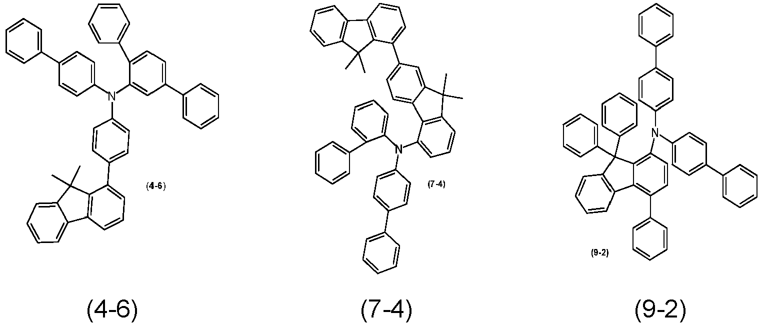

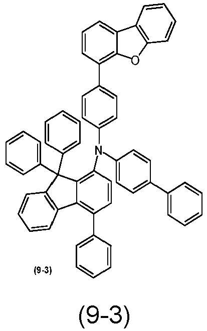

8. Compound according to one or more of Claims 1 to 7, **characterised in that** B' is a single bond.

9. Compound according to one or more of Claims 1 to 8, **characterised in that** it is a monoamine compound.

10. Process for the preparation of a compound according to one or more of Claims 1 to 9 by means of one-step Buchwald coupling by reaction of a fluorene derivative containing a leaving group with Ar²-NH-Ar¹.

11. Process for the preparation of a compound according to one or more of Claims 1 to 9 by means of two-step Buchwald coupling by stepwise reaction of a fluorene derivative containing a leaving group with (1) Ar²-NH₂ and (2) NH₂-Ar¹.

12. Process for the preparation of a compound according to one or more of Claims 1 to 9, **characterised in that** the compound is prepared from a benzochromen-6-one.

13. Process according to Claim 12, comprising the following steps:
a) addition of an organometallic compound onto a benzochromen-6-one and subsequent
b) acid-catalysed cyclisation to give a 4-hydroxyfluorene derivative and subsequent
c) conversion of the hydroxyl group in position 4 of the fluorene into a leaving group and subsequent
d) conversion of the fluorene into the desired product.

14. Oligomer, polymer or dendrimer containing one or more compounds according to one or more of Claims 1 to 9, where the bond(s) to the polymer, oligomer or dendrimer may be localised at any desired positions in formula (1) that are substituted by R¹ to R⁶.

15. Composition comprising one or more compounds according to one or more of Claims 1 to 9 or one or more polymers, oligomers or dendrimers according to Claim 14 and at least one further organofunctional material selected from the group consisting of fluorescent emitters, phosphorescent emitters, host materials, matrix materials, electrontransport materials, electron-injection materials, hole-conductor materials, hole-injection materials, electron-blocking materials and holeblocking materials.

16. Formulation comprising at least one compound according to one or more of Claims 1 to 9 or at least one polymer, oligomer or dendrimer according to Claim 14 or at least one composition according to Claim 15 and at least one solvent.

17. Electronic device containing at least one compound according to one or more of Claims 1 to 9 or at least one polymer, oligomer or dendrimer according to Claim 14 or at least one composition according to Claim 15.

18. Electronic device according to Claim 17, **characterised in that** it is selected from organic integrated circuits (O-ICs), organic field-effect transistors (O-FETs), organic thin-film transistors (O-TFTs), organic light-emitting transistors (O-LETs), organic solar cells (O-SCs), organic optical detectors, organic photoreceptors, organic field-quench devices (O-FQDs), light-emitting electrochemical cells (LECs), organic laser diodes (O-lasers) and organic electroluminescent devices (OLEDs).

19. Electronic device according to Claim 17 or 18 which is selected from the group of organic electroluminescent devices, in particular an organic light-emitting diode (OLED), **characterised in that** the compound according to one or more of Claims 1 to 9 or the polymer, oligomer or dendrimer according to Claim 14 or a composition according to Claim 15 is employed in one or more of the following functions:
- as hole-transport material in a hole-transport or hole-injection layer,
- as matrix material in an emitting layer,
- as electron-blocking material,
- as exciton-blocking material,
where the use as hole-transport material in a hole-transport or hole-injection layer is preferred.

## Revendications

1. Composé de formule générale (1) 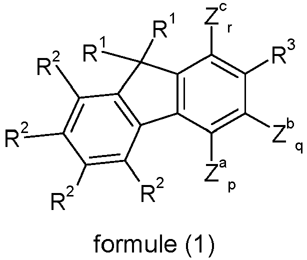 dans laquelle ce qui suit s'applique aux symboles et indices utilisés :
R¹ est à chaque occurrence, de manière identique ou différente, H, D, F, Cl, Br, I, C(=O)R⁴, CN, Si(R⁴)₃, NO₂, P(=O)(R⁴)₂, S(=O)R⁴, S(=O)_{2R}⁴, un groupement alkyle, alcoxy ou thioalkyle à chaîne linéaire ayant de 1 à 20 atomes de C ou un groupement alkyle, alcoxy ou thioalkyle ramifié ou cyclique ayant de 3 à 20 atomes de C ou un groupement alcényle ou alcynyle ayant de 2 à 20 atomes de C, où les groupements susmentionnés peuvent dans chaque cas être substitués par un ou plusieurs radicaux R⁴ et où un ou plusieurs groupements CH₂ dans les groupements susmentionnés peuvent être remplacés par -R⁴C=CR⁴-, -C=C-, Si(R⁴)₂, C=O, C=S, C=NR⁴, -C(=O)O-, -C(=O)NR⁴-, P(=O)(R⁴), -O-, -S-, SO ou SO₂ et où un ou plusieurs atomes de H dans les groupements susmentionnés peuvent être remplacés par D, F, Cl, Br, I, CN ou NO₂, ou un noyau aromatique ou hétéroaromatique ayant de 6 à 30 atomes de cycle aromatique, qui peut dans chaque cas être substitué par un ou plusieurs radicaux R⁴, ou un groupement aryloxy ayant de 5 à 60 atomes de cycle aromatique, qui peut être substitué par un ou plusieurs radicaux R⁴, ou un groupement aralkyle ayant de 5 à 60 atomes de cycle aromatique, qui peut dans chaque cas être substitué par un ou plusieurs radicaux R⁴, où les deux radicaux R¹ **peuvent** être liés l'un avec l'autre et peuvent former un cycle, de sorte qu'un composé spiro se forme dans la position 9 du fluorène, où les spirobifluorènes sont exclus ;
R², R³ sont à chaque occurrence, de manière identique ou différente, préférablement de manière identique, H, D, F, Cl, Br, I, C(=O)R⁴, CN, Si(R⁴)₃, NO₂, P(=O)(R⁴)₂, S(=O)R⁴, S(=O)_{2R}⁴, N(R⁴)₂, un groupement alkyle, alcoxy ou thioalkyle à chaîne linéaire ayant de 1 à 20 atomes de C ou un groupement alkyle, alcoxy ou thioalkyle ramifié ou cyclique ayant de 3 à 20 atomes de C ou un groupement alcényle ou alcynyle ayant de 2 à 20 atomes de C, où les groupements susmentionnés peuvent dans chaque cas être substitués par un ou plusieurs radicaux R⁴ et où un ou plusieurs groupements CH₂ dans les groupements susmentionnés peuvent être remplacés par -R⁴C=CR⁴-, -C=C-, Si(R⁴)₂, C=O, C=S, C=NR⁴, -C(=O)O-, -C(=O)NR⁴-, P(=O)(R⁴), -O-, -S-, SO ou SO₂ et où un ou plusieurs atomes de H dans les groupements susmentionnés peuvent être remplacés par D, F, Cl, Br, I, CN ou NO₂, ou un noyau aromatique ayant de 6 à 30 atomes de cycle aromatique, qui peut dans chaque cas être substitué par un ou plusieurs radicaux R⁴ ;
R⁴ est à chaque occurrence, de manière identique ou différente, H, D, F, Cl, Br, I, C(=O)R⁵, CN, Si(R⁵)₃, NO₂, P(=O)(R⁵)₂, S(=O)R⁵, S(=O)₂R⁵, N(R⁵)₂, un groupement alkyle, alcoxy ou thioalkyle à chaîne linéaire ayant de 1 à 20 atomes de C ou un groupement alkyle, alcoxy ou thioalkyle ramifié ou cyclique ayant de 3 à 20 atomes de C ou un groupement alcényle ou alcynyle ayant de 2 à 20 atomes de C, où les groupements susmentionnés peuvent dans chaque cas être substitués par un ou plusieurs radicaux R⁵ et où un ou plusieurs groupements CH₂ dans les groupements susmentionnés peuvent être remplacés par -R⁵C=CR⁵-, -C=C-, Si(R⁵)₂, C=O, C=S, C=NR⁵, -C(=O)O-, -C(=O)NR⁵-, P(=O)(R⁵), -O-, -S-, SO ou SO₂ et où un ou plusieurs atomes de H dans les groupements susmentionnés peuvent être remplacés par D, F, Cl, Br, I, CN ou NO₂, ou un noyau aromatique ayant de 5 à 30 atomes de cycle aromatique, qui peut dans chaque cas être substitué par un ou plusieurs radicaux R⁵ ;
R⁵ est choisi dans le groupe constitué par H, D, F, un radical hydrocarboné aliphatique ayant de 1 à 20 atomes de C, où un ou plusieurs atomes de H peuvent être remplacés par D ou F, où deux, ou plus, substituants R⁵ adjacents peuvent former un noyau aliphatique mono- ou polycyclique les uns avec les autres ;
r vaut 1 ;
p, q valent 0 ;
Z^{a}₀, Z^{b}₀ sont, de manière identique ou différente à chaque occurrence, égaux à R³ ;
Z^{c}₁ est égal à 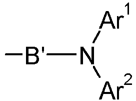
B' est une liaison simple, un groupement aryle ayant de 6 à 30 atomes de cycle ou un groupement hétéroaryle mono- ou bicyclique ayant de 5 à 30 atomes de cycle, qui peut dans chaque cas être substitué par un ou plusieurs radicaux R⁴, où, si B' est une liaison simple, l'atome d'azote est lié directement au fluorène ;
Ar¹, Ar² sont choisis à chaque occurrence, de manière identique ou différente, parmi les groupements suivants de formules (37) à (66), (69) à (72) et (76) à (96), qui peuvent être substitués par un ou plusieurs radicaux R⁶, 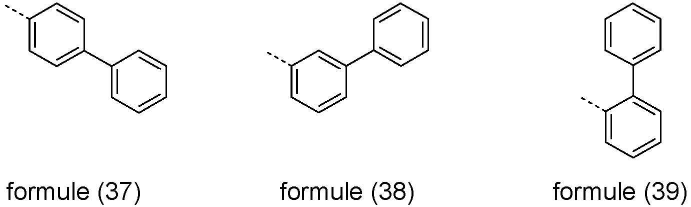 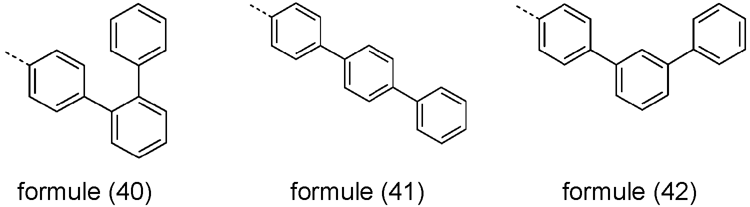 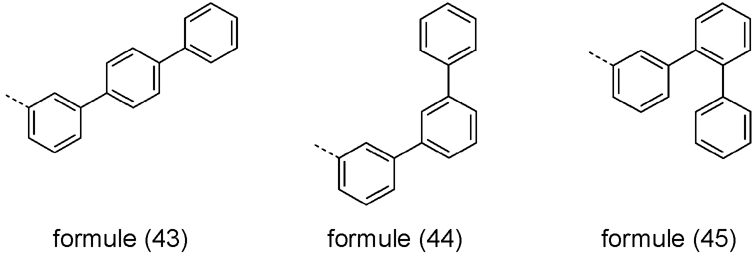 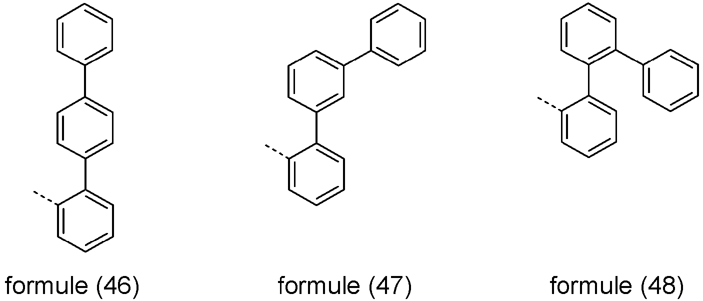 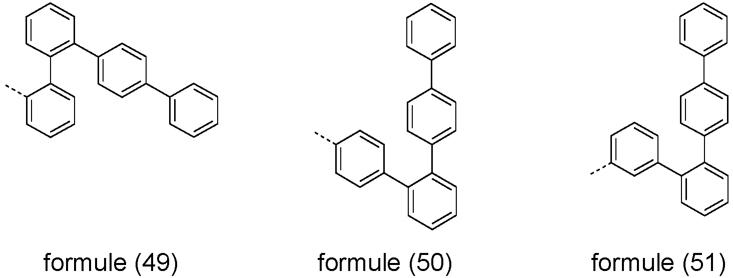 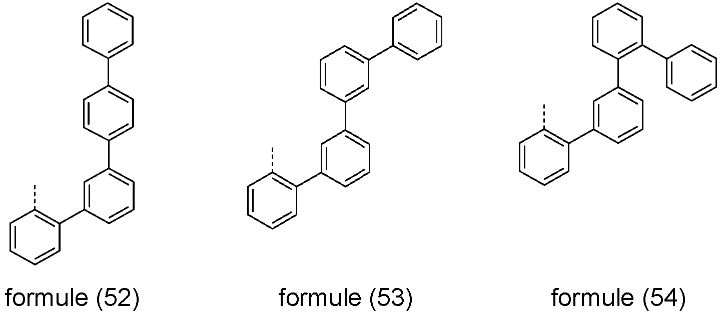 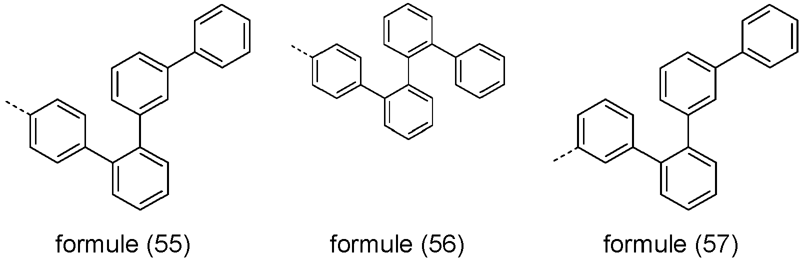 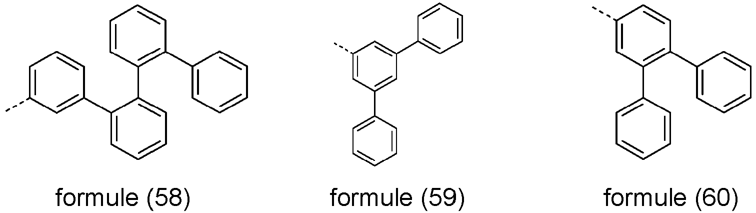 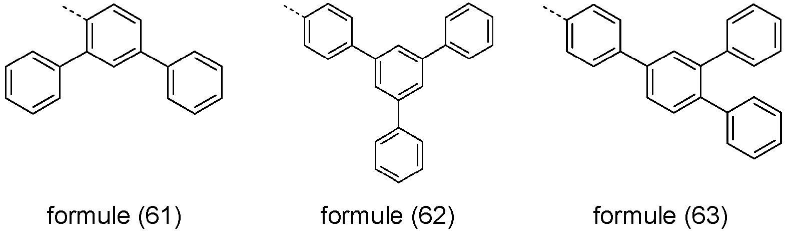 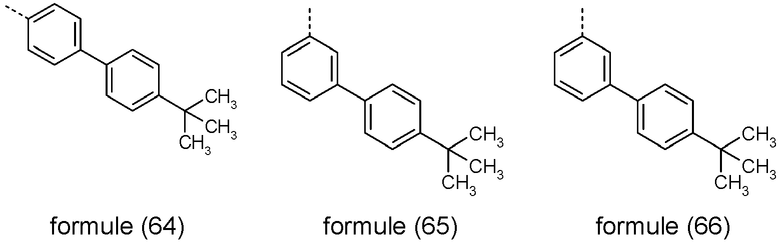 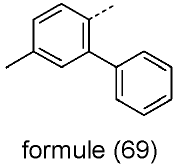 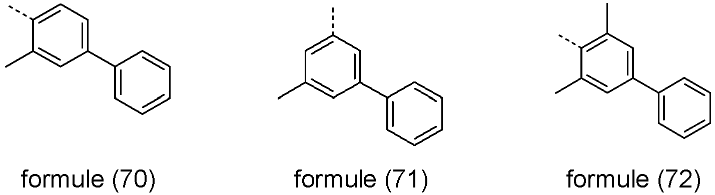 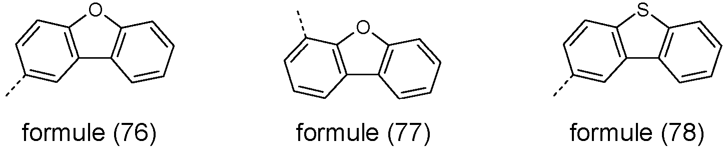 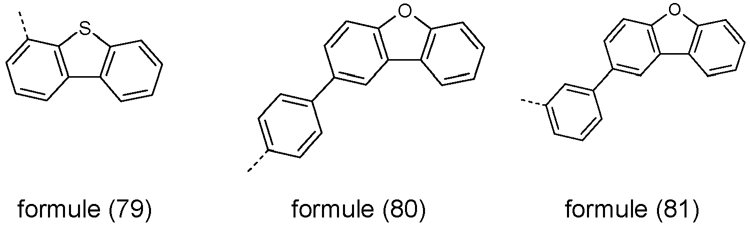 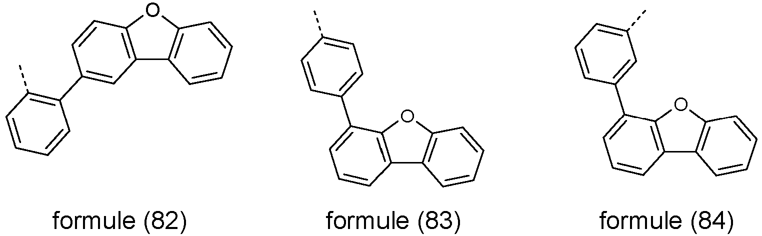 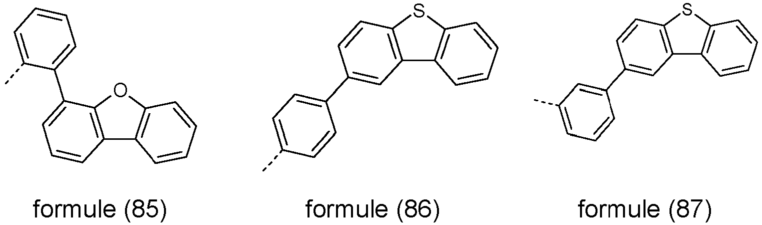 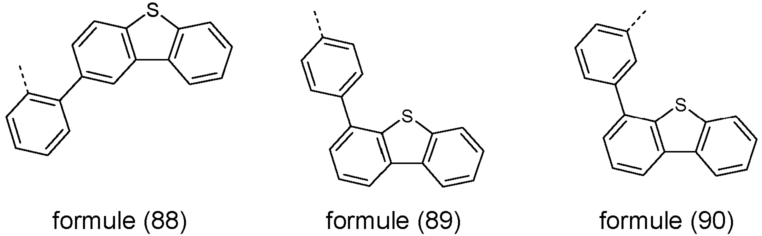 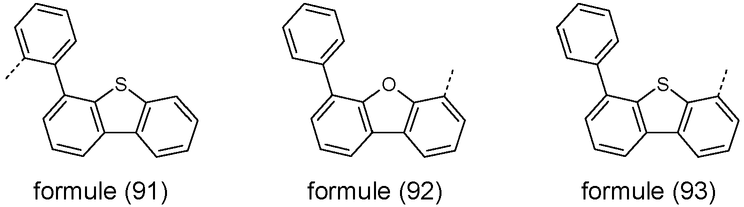 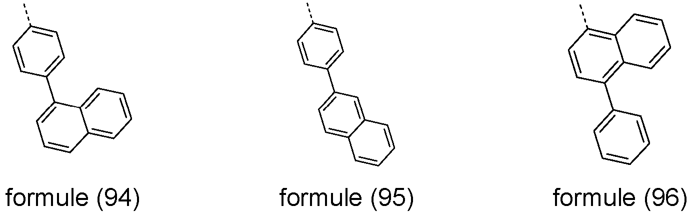
R⁶ est à chaque occurrence, de manière identique ou différente, H, D, F, Cl, Br, I, C(=O)R⁵, CN, Si(R⁵)₃, NO₂, P(=O)(R⁵)₂, S(=O)R⁵, S(=O)₂R⁵, un groupement alkyle, alcoxy ou thioalkyle à chaîne linéaire ayant de 1 à 20 atomes de C ou un groupement alkyle, alcoxy ou thioalkyle ramifié ou cyclique ayant de 3 à 20 atomes de C ou un groupement alcényle ou alcynyle ayant de 2 à 20 atomes de C, où les groupements susmentionnés peuvent dans chaque cas être substitués par un ou plusieurs radicaux R⁵ et où un ou plusieurs groupements CH₂ dans les groupements susmentionnés peuvent être remplacés par -R⁵C=CR⁵-, -C=C-, Si(R⁵)₂, C=O, C=S, C=NR⁵, -C(=O)O-, -C(=O)NR⁵-, P(=O)(R⁵), -O-, -S-, SO ou SO₂ et où un ou plusieurs atomes de H dans les groupements susmentionnés peuvent être remplacés par D, F, Cl, Br, I, CN ou NO₂.

2. Composé selon la revendication 1, **caractérisé en ce qu'**il répond à la formule générale (5) 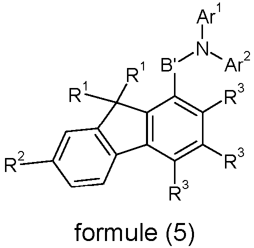 dans laquelle les définitions selon la revendication 1 s'appliquent aux symboles utilisés.

3. Composé selon la revendication 1 ou 2, **caractérisé en ce qu'**il répond à la formule générale (8) 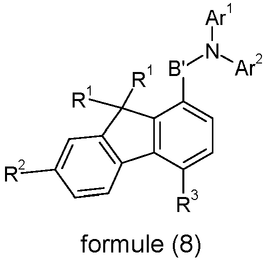 dans laquelle les définitions selon la revendication 1 s'appliquent aux symboles utilisés.

4. Composé selon l'une ou plusieurs parmi les revendications 1 à 3, **caractérisé en ce que** les deux R¹ sont identiques.

5. Composé selon l'une ou plusieurs parmi les revendications 1 à 4, **caractérisé en ce que** B' est une liaison simple ou un groupement phénylène, biphénylène, terphénylène, naphtylène, pyridinylène, pyrimidinylène, pyrazinylène, pyridazinylène, triazinylène, dibenzofuranylène ou dibenzothiophénylène, qui peut être substitué par un ou plusieurs radicaux R⁴.

6. Composé selon l'une ou plusieurs parmi les revendications 1 à 5, **caractérisé en ce que** B' est une liaison simple ou un groupement phénylène, biphénylène, terphénylène, naphtylène, dibenzofuranylène ou dibenzothiophénylène, qui peut être substitué par un ou plusieurs radicaux R⁴.

7. Composé selon l'une ou plusieurs parmi les revendications 1 à 6, **caractérisé en ce que** le composé est choisi parmi l'un des composés suivants

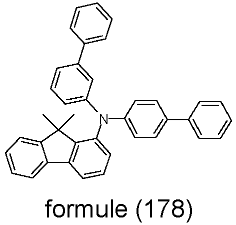

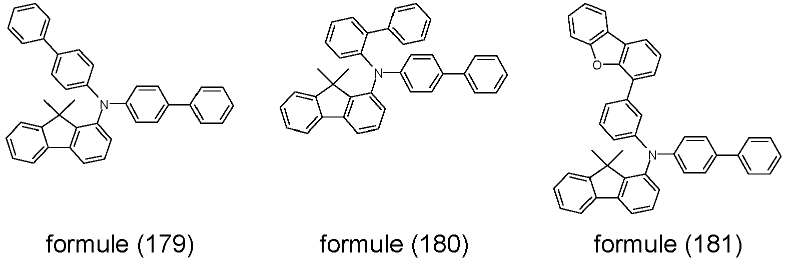

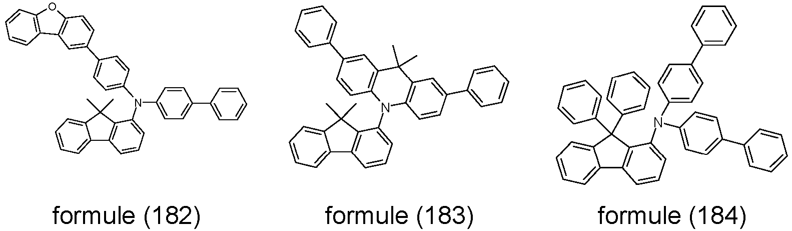

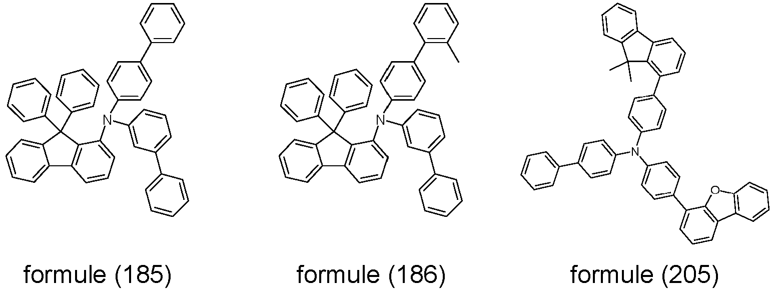

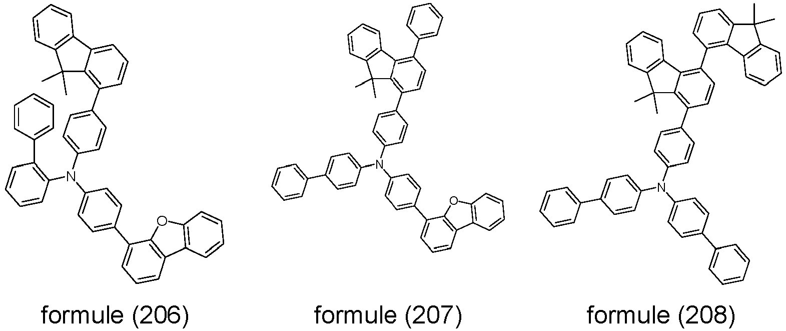

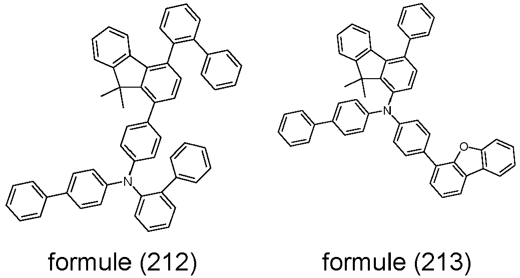

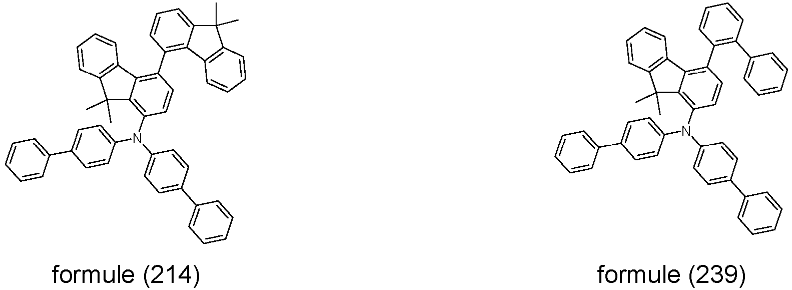

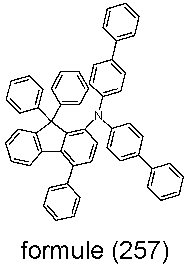

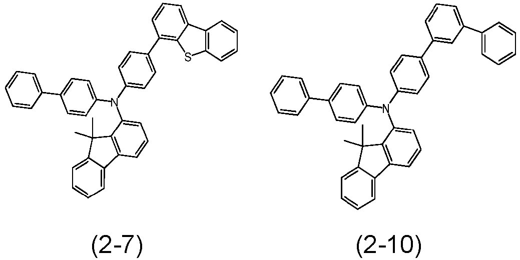

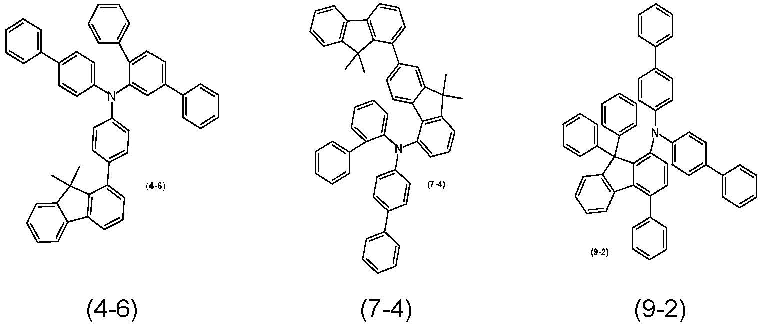

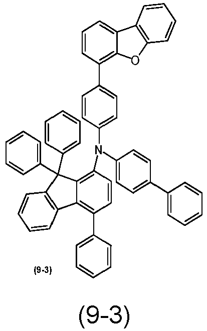

8. Composé selon l'une ou plusieurs parmi les revendications 1 à 7, **caractérisé en ce que** B' est une liaison simple.

9. Composé selon l'une ou plusieurs parmi les revendications 1 à 8, **caractérisé en ce qu'**il s'agit d'un composé de monoamine.

10. Procédé de préparation d'un composé selon l'une ou plusieurs parmi les revendications 1 à 9, au moyen d'un couplage de Buchwald en une étape, par réaction d'un dérivé de fluorène contenant un groupement partant avec Ar²-NH-Ar¹.

11. Procédé de préparation d'un composé selon l'une ou plusieurs parmi les revendications 1 à 9, au moyen d'un couplage de Buchwald en deux étapes, par réaction par étapes d'un dérivé de fluorène contenant un groupement partant avec (1) Ar²-NH₂ et (2) NH₂-Ar¹.

12. Procédé de préparation d'un composé selon l'une ou plusieurs parmi les revendications 1 à 9, **caractérisé en ce que** le composé est préparé à partir d'une benzochromén-6-one.

13. Procédé selon la revendication 12, comprenant les étapes suivantes :
a) l'addition d'un composé organométallique sur une benzochromén-6-one, et ensuite
b) une cyclisation catalysée par un acide pour donner un dérivé de 4-hydroxyfluorène, et ensuite
c) la conversion du groupement hydroxyle en position 4 du fluorène en un groupement partant, et ensuite
d) la conversion du fluorène en produit désiré.

14. Oligomère, polymère ou dendrimère contenant un ou plusieurs composés selon l'une ou plusieurs parmi les revendications 1 à 9, la ou les liaisons au polymère, à l'oligomère ou au dendrimère pouvant être situées au niveau de positions désirées quelconques dans la formule (1) qui sont substituées par R¹ à R⁶.

15. Composition comprenant un ou plusieurs composés selon l'une ou plusieurs parmi les revendications 1 à 9, ou un ou plusieurs polymères, oligomères ou dendrimères selon la revendication 14, et au moins un autre matériau organique fonctionnel choisi dans le groupe constitué par les émetteurs fluorescents, les émetteurs phosphorescents, les matériaux hôtes, les matériaux de matrice, les matériaux de transport d'électrons, les matériaux d'injection d'électrons, les matériaux conducteurs de trous, les matériaux d'injection de trous, les matériaux de blocage d'électrons et les matériaux de blocage de trous.

16. Formulation comprenant au moins un composé selon l'une ou plusieurs parmi les revendications 1 à 9, ou au moins un polymère, oligomère ou dendrimère selon la revendication 14, ou au moins une composition selon la revendication 15, et au moins un solvant.

17. Dispositif électronique contenant au moins un composé selon l'une ou plusieurs parmi les revendications 1 à 9, ou au moins un polymère, oligomère ou dendrimère selon la revendication 14, ou au moins une composition selon la revendication 15.

18. Dispositif électronique selon la revendication 17, **caractérisé en ce qu'**il est choisi parmi les circuits intégrés organiques (O-IC), les transistors organiques à effet de champ (O-FET), les transistors organiques à couche mince (O-TFT), les transistors organiques émetteurs de lumière (O-LET), les cellules solaires organiques (O-SC), les détecteurs optiques organiques, les photorécepteurs organiques, les dispositifs organiques à extinction de champ (O-FQD), les cellules électrochimiques émettrices de lumière (LEC), les diodes laser organiques (O-lasers) et les dispositifs électroluminescents organiques (OLED).

19. Dispositif électronique selon la revendication 17 ou 18, qui est choisi dans le groupe constitué par les dispositifs électroluminescents organiques, en particulier une diode électroluminescente organique (OLED), **caractérisé en ce que** le composé selon l'une ou plusieurs parmi les revendications 1 à 9, ou le polymère, l'oligomère ou le dendrimère selon la revendication 14, ou une composition selon la revendication 15, est employé(e) dans une ou plusieurs parmi les fonctions suivantes :
- comme matériau de transport de trous dans une couche de transport de trous ou d'injection de trous,
- comme matériau de patrice dans une couche émettrice,
- comme matériau de blocage d'électrons,
- comme matériau de blocage d'excitons,
où l'utilisation comme matériau de transport de trous dans une couche de transport de trous ou d'injection de trous est préférée.
